# EUROPEAN PATENT APPLICATION

(11) **EP 4 289 857 A1**
(43) Date of publication of application: **13.12.2023**
(21) Application number: 22749058.8
(22) Date of filing: 28.01.2022
(51) Int. Cl.: C07J 71/00, C07K 16/24, A61K 47/68, A61K 31/58, A61P 37/00

(54) **DRUG CONJUGATE OF GLUCOCORTICOID RECEPTOR AGONIST, AND APPLICATION THEREOF IN MEDICINE**

(30) Priority: 04.02.2021 CN 202110166634
(71) Applicant: Shanghai Senhui Medicine Co., Ltd., Shanghai 201203 (CN); Shanghai Shengdi Pharmaceutical Co., Ltd, Shanghai 201210 (CN); Jiangsu Hengrui Pharmaceuticals Co., Ltd., Lianyungang, Jiangsu 222047 (CN)
(72) Inventor: ZHU, Lingjian, Shanghai 201203 (CN); HONG, Min, Shanghai 201203 (CN); TANG, Manping, Shanghai 201203 (CN); SU, Lu, Shanghai 201210 (CN); DENG, Mengdie, Shanghai 201210 (CN); ZHANG, Jingyang, Shanghai 201210 (CN); REN, Wenming, Shanghai 201210 (CN); LIN, Kan, Shanghai 201210 (CN); HUANG, Jian, Shanghai 201203 (CN); LIAO, Cheng, Shanghai 201210 (CN); ZHANG, Lianshan, Lianyungang, Jiangsu 222047 (CN)
(74) Representative: Pistolesi, Roberto
(86) International application number: PCT/CN2022/074455
(87) International publication number: WO 2022/166779

(57) **Abstract**

A drug conjugate of a glucocorticoid receptor agonist, and an application thereof in medicine. Specifically, the present invention relates to an antibody-drug conjugate as represented by formula (I): Ab-(L-D)k (I), wherein Ab is an antibody or an antigen-binding fragment thereof, L is a linker covalently linking Ab to D, k is 1 to 20, and D is as represented by formula (II-A) or (II-B). The groups in the formulas are as defined in the description. The antibody-drug conjugate can effectively treat autoimmune diseases.

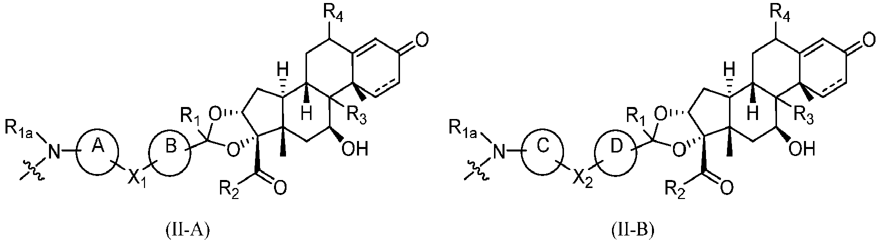

## Description

### TECHNICAL FIELD

The present disclosure belongs to the field of pharmaceutics, and particularly relates to a drug conjugate of a glucocorticoid receptor agonist and use thereof in pharmaceutics.

### BACKGROUND

Rheumatoid arthritis (RA) is a common arthritis. It is an autoimmune disease with an incidence of 0.3-1% in the population. Without timely treatment, it may cause damage to bones and injuries to joints. The pathogenesis of RA involves a variety of pro-inflammatory cytokines, such as tumor necrosis factor-α (TNFα) and interleukins such as IL-1, IL-6 and IL-8. Therefore, inhibiting the generation of pro-inflammatory cytokines or blocking their physiological actions is now a popular topic in RA research. In recent years, many newly developed biological agents, such as TNFα inhibitors and anti-IL-6R antibodies, have been able to control the progression of the disease by blocking or down-regulating the activity of pro-inflammatory cytokines. TNFα is now believed to be one of the most important pro-inflammatory cytokines among many cytokines of inflammatory responses to RA; it plays an important role in the progression of RA, local inflammatory responses and tissue injuries. TNFα inhibitors that have been approved by the U.S. FDA include: the soluble receptor antagonist etanercept, the human murine chimeric antibody infliximab, the fully human-derived monoclonal antibody adalimumab (Humira^{®}), the fully human-derived monoclonal antibody golimumab, and the polyethylene glycol humanized Fab' fragment certolizumab pegol. Despite their clinical success, TNFα inhibitors are still limited by their maximal efficacy in patients; there is a need to identify and develop more potent and effective therapeutic agents. Patients treated with TNFα inhibitors may also produce immunogenic responses to the therapeutic agents such that their effectiveness is limited.

Glucocorticoid receptor agonists are also relatively effective drugs for the treatment of rheumatoid arthritis. As typical glucocorticoid receptor agonists, glucocorticoid receptor agonists prepared *in vivo* such as cortisol and corticosterone and synthetic glucocorticoid receptor agonists such as dexamethasone, prednisone and prednisolone are known. These glucocorticoid receptor agonists are collectively called steroids as they have a steroid structure. They are used in the treatment of various diseases. However, these steroids may sometimes cause side effects such as steroid peptic ulcer, steroid purpura, steroid pancreatitis, steroid diabetes, steroid cataract and steroid glaucoma due to their use.

An antibody-drug conjugate (ADC) is a molecule in which a monoclonal antibody or an antibody fragment is linked to a biologically active drug by a stable chemical linker compound. Most ADCs in preclinical and clinical development are used for oncological indications. The cytotoxic payloads target cancer cells expressing antigens. However, the regulation of pathogenic cellular activity through ADC-mediated delivery of biologically active small molecules is also appealing to non-oncological indications, thereby leading to the widespread use of this technology.

Some drug conjugates of glucocorticoid receptor agonists have been disclosed in the prior art, e.g., WO2017210471 and WO2019106609.

### SUMMARY

In one aspect, the present disclosure provides an antibody-drug conjugate (ADC) of formula (I),

Ab-(L-D)ₖ (I)

wherein Ab is an antibody or an antigen-binding fragment thereof;
L is a linker covalently linking Ab to D, and k is 1 to 20 (including 1, 2, 3, 4, 5, 6, 7, 8, 9, 10, 11, 12, 13, 14, 15, 16, 17, 18, 19, 20, or any value between any two values);
D is represented by formula (II-A) or (II-B): 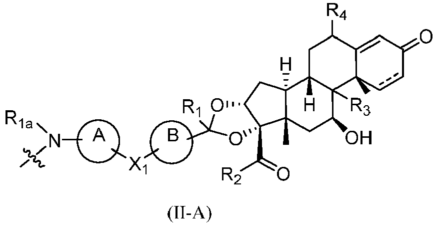 or 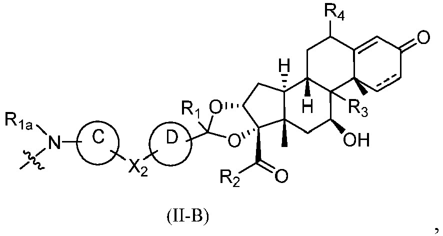
wherein,
-̅ -̅ -̅ represents a single bond or a double bond;
each R₁ₐ is independently selected from the group consisting of hydrogen, alkyl and alkoxy, and the alkyl and alkoxy are each independently optionally substituted with one or more substituents selected from the group consisting of alkyl, alkoxy, halogen, deuterium, amino, cyano, nitro, hydroxy and hydroxyalkyl;
ring A is aryl or heteroaryl optionally substituted with one or more Qi substituents;
ring B is aryl or heteroaryl optionally substituted with one or more Qi substituents;
X₁ is -(CR₅ₐR_{5b})m- or is aryl or heteroaryl optionally substituted with one or more Qi substituents;
R₅ₐ and R_{5b} are each independently selected from the group consisting of hydrogen, halogen, hydroxy, sulfhydryl, deuterium, nitro, cyano, and the following groups optionally substituted with one or more Qi substituents: alkyl, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, alkoxy, alkylthio, alkenyl and alkynyl, or together R₅ₐ and R_{5b} form oxo or thio;
ring C and ring D are each independently selected from the group consisting of aryl and heteroaryl optionally substituted with one or more Qi substituents, and at least one of ring C and ring D is selected from the group consisting of fused cycloaryl and fused heteroaryl optionally substituted with one or more Qi substituents;
X₂ is selected from the group consisting of -(CR₆ₐR_{6b})n-, aryl or heteroaryl optionally substituted with one or more Qi substituents, -O-, -S-, -S(O)-, -S(O)(O)-, -NR_{6c}-, -CH₂S-, -CH₂O-, -NHCR_{6d}R₆ₑ-, -CR_{6f}=CR_{6g}- and -C=C-, or X₂ is absent;
R₆ₐ and R_{6b} are each independently selected from the group consisting of hydrogen, halogen, hydroxy, sulfhydryl, deuterium, nitro, cyano, and the following groups optionally substituted with one or more Qi substituents: alkyl, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, alkoxy, alkylthio, alkenyl and alkynyl, or R₆ₐ and R_{6b}, together with the carbon atom to which they are attached, form 3- to 10-membered cycloalkyl, or together R₆ₐ and R_{6b} form oxo or thio;
R_{6c}, R_{6d}, R₆ₑ, R_{6f} and R_{6g} are each independently selected from the group consisting of hydrogen, C₁-C₆ alkyl, C₁-C₆ haloalkyl and C₁-C₆ alkoxy;
each R₁ is independently selected from the group consisting of hydrogen, alkyl and alkoxy, wherein the alkyl and alkoxy are each independently optionally substituted with one or more substituents selected from the group consisting of alkyl, alkoxy, halogen, deuterium, amino, cyano, nitro, hydroxy and hydroxyalkyl;
each R₂ is independently selected from the group consisting of -CH₂OH, -CH₂SH, -CH₂Cl, -SCH₂Cl, -SCH₂F, -SCH₂CF₃, -OH, -OCH₂CN, -OCH₂Cl, -OCH₂F, -OCH₃, -OCH₂CH₃, -SCH₂CN, 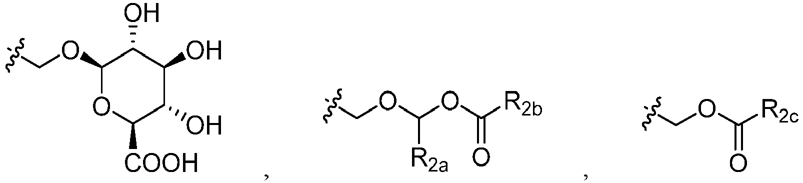 and 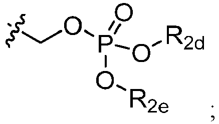
each R₂ₐ is independently hydrogen or C₁-C₆ alkyl;
each R_{2b} is independently C₁-C₆ alkyl or C₁-C₆ alkoxy;
each R_{2c} is independently selected from the group consisting of hydrogen, C₁-C₆ alkyl, -CH₂OH and C₁-C₆ alkoxy;
R_{2d} and R₂ₑ are each independently hydrogen or C₁-C₆ alkyl;
each R₃ is independently hydrogen or a halogen;
each R₄ is independently selected from the group consisting of hydrogen, halogen and hydroxy;
m and n are each independently an integer of 1 to 6;
the Qi substituents are each independently selected from the group consisting of C₁-C₆ alkyl, halogen, deuterium, hydroxy, sulfhydryl, -NRᵢRⱼ, oxo, thio, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthio, C₂-C₆ alkenyl, C₂-C₆ alkynyl, 3- to 10-membered cycloalkyl, 3- to 10-membered heterocyclyl, 6- to 10-membered aryl, 5- to 10-membered heteroaryl, 8-to 12-membered fused cycloaryl, and 5- to 12-membered fused heteroaryl;
Rᵢ and Rⱼ are each independently selected from the group consisting of hydrogen, hydroxy, C₁-C₆ alkyl and C₁-C₆ alkoxy;
Rₖ is independently selected from the group consisting of hydrogen, C₁-C₆ alkyl, C₁-C₆ haloalkyl, C₁-C₆ alkoxy, hydroxy and -NRᵢRⱼ, wherein the alkyl, alkoxy and haloalkyl are each independently optionally substituted with one or more substituents selected from the group consisting of C₁-C₆ alkyl, halogen, hydroxy, sulfhydryl, -NRᵢRⱼ, oxo, thio, carboxyl, nitro, cyano, C₁-C₆ alkoxy, C₁-C₆ alkylthio, C₂-C₆ alkenyl, C₂-C₆ alkynyl, 3- to 10-membered cycloalkyl, 3- to 10-membered heterocyclyl, 6- to 10-membered aryl, and 5- to 10-membered heteroaryl;
provided that when R₅ₐ is hydrogen or alkyl, R_{5b} is not hydrogen or alkyl.

In certain embodiments, each R₁ₐ is independently selected from the group consisting of hydrogen, C₁-C₆ alkyl and C₁-C₆ alkoxy, and the alkyl and alkoxy are each independently optionally substituted with one or more substituents selected from the group consisting of C₁-C₆ alkyl, C₁-C₆ alkoxy, halogen, deuterium, amino, cyano and hydroxy.

In certain embodiments, each R₁ₐ is independently selected from the group consisting of hydrogen, C₁-C₆ alkyl and C₁-C₆ alkoxy.

In certain embodiments, ring A is 6- to 10-membered aryl or 5- to 10-membered heteroaryl optionally substituted with one or more Qi substituents, and the heteroaryl comprises at least one nitrogen atom.

In certain embodiments, ring A is 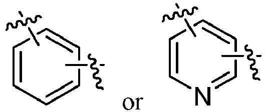 optionally substituted with one or more Qi substituents.

In certain embodiments, ring B is 6- to 10-membered aryl or 5- to 10-membered heteroaryl optionally substituted with one or more Qi substituents, and the heteroaryl comprises at least one nitrogen atom.

In certain embodiments, ring B is 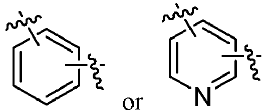 optionally substituted with one or more Qi substituents.

In certain embodiments, X₁ is -(CR₅ₐR_{5b})m- or is 6- to 10-membered aryl or 5- to 10-membered heteroaryl optionally substituted with one or more Qi substituents, and the heteroaryl comprises at least one nitrogen atom.

In certain embodiments, R₅ₐ and R_{5b} are each independently selected from the group consisting of hydrogen, halogen, hydroxy, sulfhydryl, deuterium, nitro, cyano, and the following groups optionally substituted with one or more Qi substituents: C₁-C₆ alkyl, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, C₁-C₆ alkoxy, C₁-C₆ alkylthio, C₂-C₆ alkenyl and C₂-C₆ alkynyl, or together R₅ₐ and R_{5b} form oxo or thio.

In certain embodiments, R₅ₐ and R_{5b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ, -S(O)Rₖ, -S(O)ORₖ, -S(O)(O)Rₖ, -S(O)(O)ORₖ, C₁-C₆ alkoxy, C₁-C₆ alkylthio, C₂-C₆ alkenyl and C₂-C₆ alkynyl, or together R₅ₐ and R_{5b} form oxo or thio.

In certain embodiments, R₅ₐ and R_{5b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ, -S(O)Rₖ, -S(O)(O)Rₖ, C₁-C₆ alkoxy, C₂-C₆ alkenyl and C₂-C₆ alkynyl, or together R₅ₐ and R_{5b} form oxo or thio.

In certain embodiments, R₅ₐ and R_{5b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy, or together R₅ₐ and R_{5b} form oxo or thio. In certain embodiments, ring C and ring D are each independently selected from the group consisting of 6- to 10-membered aryl, 5- to 10-membered heteroaryl, 8- to 12-membered fused cycloaryl and 5- to 12-membered fused heteroaryl optionally substituted with one or more Qi substituents, and the heteroaryl or fused heteroaryl comprises at least one nitrogen atom.

In certain embodiments, ring C and ring D are each independently selected from the group consisting of the following groups optionally substituted with one or more Qi substituents:

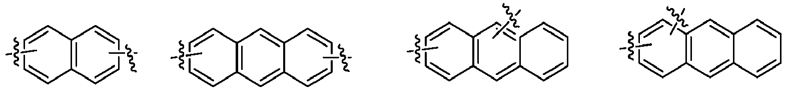

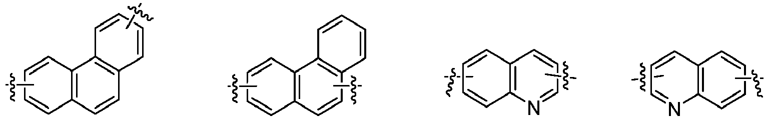

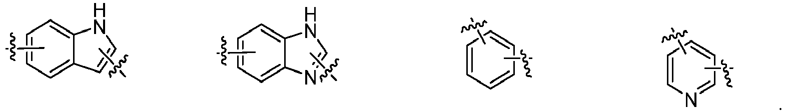

In certain embodiments, ring C is selected from the group consisting of: 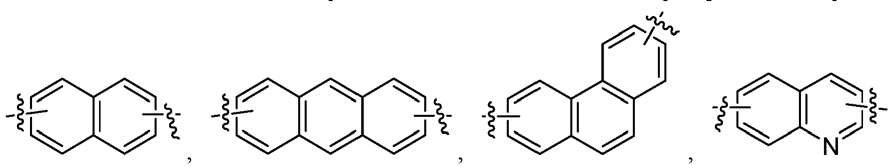 and 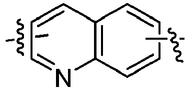 optionally substituted with one or more Qi substituents.

In certain embodiments, ring D is 6- to 10-membered aryl or 5- to 10-membered heteroaryl optionally substituted with one or more Qi substituents, and the heteroaryl comprises at least one nitrogen atom.

In certain embodiments, ring D is 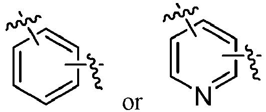 optionally substituted with one or more Qi substituents.

In certain embodiments, X₂ is selected from the group consisting of -(CR₆ₐR_{6b})n-, -O-, -S-, -NR_{6c}-, -CH₂S-, -CH₂O-, -NHCR_{6d}R₆ₑ-, and 6- to 10-membered aryl or 5- to 10-membered heteroaryl optionally substituted with one or more Qi substituents, and the heteroaryl comprises at least one nitrogen atom.

In certain embodiments, R₆ₐ and R_{6b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ, -S(O)Rₖ, -S(O)ORₖ, -S(O)(O)Rₖ, -S(O)(O)ORₖ, C₁-C₆ alkoxy, C₁-C₆ alkylthio, C₂-C₆ alkenyl and C₂-C₆ alkynyl, or R₆ₐ and R_{6b}, together with the carbon atom to which they are attached, form 3- to 10-membered cycloalkyl, or together R₆ₐ and R_{6b} form oxo or thio.

In certain embodiments, R₆ₐ and R_{6b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ, -S(O)Rₖ, -S(O)(O)Rₖ, C₁-C₆ alkoxy, C₂-C₆ alkenyl and C₂-C₆ alkynyl, or R₆ₐ and R_{6b}, together with the carbon atom to which they are attached, form 3- to 10-membered cycloalkyl, or together R₆ₐ and R_{6b} form oxo or thio.

In certain embodiments, R₆ₐ and R_{6b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy, or together R₆ₐ and R_{6b} form oxo or thio. In certain embodiments, each R₁ is independently selected from the group consisting of hydrogen, C₁-C₆ alkyl and C₁-C₆ alkoxy, preferably hydrogen.

In certain embodiments, each R₄ is independently hydrogen.

In certain embodiments, the Q₁ substituents are each independently selected from the group consisting of halogen, hydroxy, sulfhydryl, deuterium, oxo, thio, cyano, amino, carboxyl, C₁-C₆ alkyl and C₁-C₆ alkoxy.

In certain embodiments, Rₖ is independently selected from the group consisting of hydrogen, C₁-C₆ alkyl, C₁-C₆ haloalkyl, C₁-C₆ alkoxy, hydroxy and -NRᵢRⱼ.

In certain embodiments, D is represented by formula (II-A') or (II-B'): 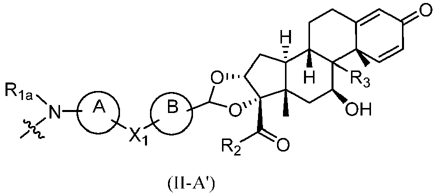 or 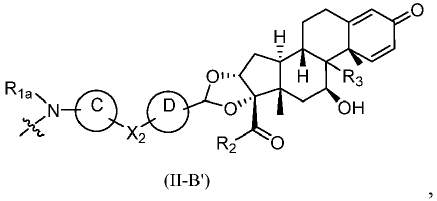 wherein,
each R₁ₐ is independently selected from the group consisting of hydrogen, C₁-C₆ alkyl and C₁-C₆ alkoxy;
ring A is 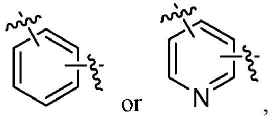 and the ring A is optionally substituted with one or more Qi substituents;
ring B is 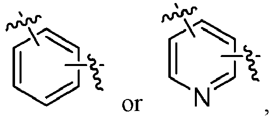 and the ring B is optionally substituted with one or more Qi substituents; X₁ is -(CR₅ₐR_{5b})m- or is 6- to 10-membered aryl or 5- to 10-membered heteroaryl optionally substituted with one or more Qi substituents, and the heteroaryl comprises at least one nitrogen atom;
R₅ₐ and R_{5b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy, or together R₅ₐ and R_{5b} form oxo or thio;
ring C is selected from the group consisting of 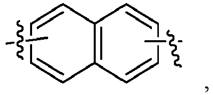 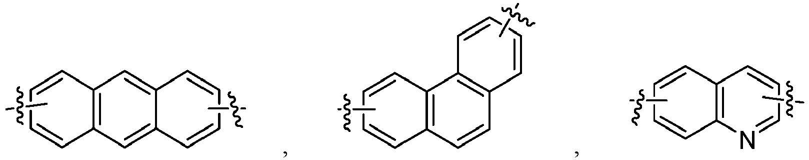 and 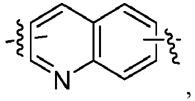 and the ring C is optionally substituted with one or more Qi substituents;
ring D is 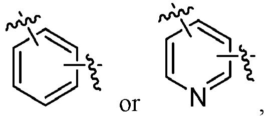 and the ring D is optionally substituted with one or more Qi substituents;
X₂ is selected from the group consisting of -(CR₆ₐR_{6b})n-, -O-, -S-, -NR_{6c}-, -CH₂S-, -CH₂O-, -NHCR_{6d}R₆ₑ-, and 6- to 10-membered aryl or 5- to 10-membered heteroaryl optionally substituted with one or more Qi substituents;
R₆ₐ and R_{6b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy, or together R₆ₐ and R_{6b} form oxo or thio;
R_{6c}, R_{6d} and R₆ₑ are each independently selected from the group consisting of hydrogen, C₁-C₆ alkyl, C₁-C₆ haloalkyl and C₁-C₆ alkoxy;
each R₂ is independently selected from the group consisting of -CH₂OH, -CH₂SH, -CH₂Cl, -SCH₂Cl, -SCH₂F, -SCH₂CF₃, -OH, -OCH₂CN, -OCH₂Cl, -OCH₂F, -OCH₃, -OCH₂CH₃, -SCH₂CN, 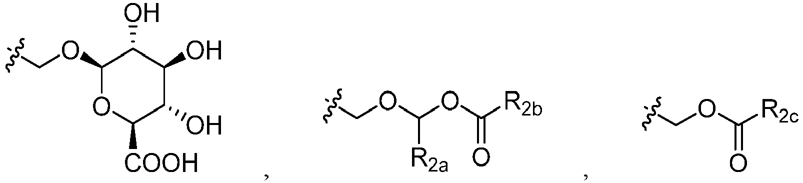 and 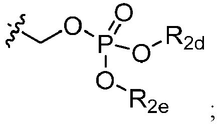
   each R₂ₐ is independently hydrogen or C₁-C₆ alkyl;
   each R_{2b} is independently C₁-C₆ alkyl or C₁-C₆ alkoxy;
   each R_{2c} is independently selected from the group consisting of hydrogen, C₁-C₆ alkyl, -CH₂OH and C₁-C₆ alkoxy;
   R_{2d} and R₂ₑ are each independently hydrogen or C₁-C₆ alkyl;
   each R₃ is independently hydrogen or a halogen;
   m and n are each independently an integer of 1 to 6;
   the Qi substituents are each independently selected from the group consisting of halogen, hydroxy, sulfhydryl, deuterium, oxo, thio, cyano, amino, carboxyl, C₁-C₆ alkyl and C₁-C₆ alkoxy;
   Rᵢ and Rⱼ are each independently selected from the group consisting of hydrogen, hydroxy, C₁-C₆ alkyl and C₁-C₆ alkoxy;
   Rₖ is independently selected from the group consisting of hydrogen, C₁-C₆ alkyl, C₁-C₆ haloalkyl, C₁-C₆ alkoxy, hydroxy and -NRᵢRⱼ;
   provided that when R₅ₐ is hydrogen or alkyl, R_{5b} is not hydrogen or alkyl.

In certain embodiments, R₁ₐ is hydrogen.

In certain embodiments, X₁ is selected from the group consisting of -(CR₅ₐR_{5b})m-, and 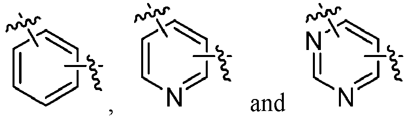 optionally substituted with one or more Qi substituents.

In certain embodiments, R₅ₐ and R_{5b} are both fluorine.

In certain embodiments, together R₅ₐ and R_{5b} form oxo or thio, preferably oxo.

In certain embodiments, X₂ is selected from the group consisting of -(CR₆ₐR_{6b})n-, and 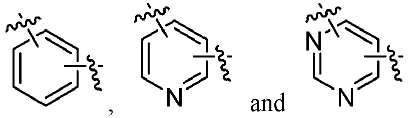 optionally substituted with one or more Qi substituents.

In certain embodiments, R₆ₐ and R_{6b} are each independently selected from the group consisting of hydrogen, halogen, hydroxy, sulfhydryl, deuterium, cyano, C₁-C₆ alkyl, -NRᵢRⱼ, -C(O)ORₖ and C₁-C₆ alkoxy, or together R₆ₐ and R_{6b} form oxo or thio.

In certain embodiments, each R₂ is independently selected from the group consisting of -CH₂OH, -CH₂SH, -OH and

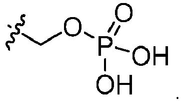

In certain embodiments, R₃ is hydrogen.

In certain embodiments, R₃ is fluorine.

In certain embodiments, k has any value between 1 and 10, preferably between 2 and 5. k may be an integer or a decimal.

In certain embodiments, the linker is stable outside a cell, such that the ADC remains intact when present in extracellular conditions but is capable of being cleaved upon internalization in a cell. In certain embodiments, the glucocorticoid receptor agonist drug moiety is cleaved from the antibody moiety when the ADC enters a cell that expresses an antigen specific to the antibody moiety of the ADC, and cleavage releases an unmodified form of the glucocorticoid receptor agonist.

In certain embodiments, the cleavable moiety in the linker is a cleavable peptide moiety. In certain embodiments, an ADC that comprises a cleavable peptide moiety exhibits a lower aggregation level and an improved antibody-to-drug ratio relative to ADCs that comprise other cleavable moieties. In certain embodiments, adding a cleavable moiety increases cytotoxicity and/or potency relative to a non-cleavable linker. In certain embodiments, the cleavable peptide moiety is cleavable by an enzyme, and the linker is an enzyme-cleavable linker. In certain embodiments, the enzyme is cathepsin, and the linker is a cathepsin-cleavable linker. In certain embodiments, the enzyme-cleavable linker (e.g., the cathepsin-cleavable linker) exhibits one or more of the improved properties described above, as compared to other cleavage mechanisms.

In certain embodiments, the linker comprises amino acid unit L₁, and the amino acid unit Li preferably comprises a peptide residue consisting of 2 to 7 amino acids selected from the group consisting of phenylalanine, glycine, valine, lysine, citrulline, serine, glutamic acid, aspartic acid, homolysine, n-methyl-valine and 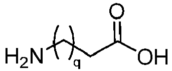 (q is an integer of 1-6); exemplary amino acid units include, but are not limited to, valine-citrulline (Val-Cit), alanine-phenylalanine (Ala-Phe),phenylalanine-lysine (Phe-Lys), phenylalanine-homolysine (Phe-Homolys), n-methyl-valine-citrulline (Me-Val-Cit), alanine-alanine (Ala-Ala), glycine-glutamic acid (Gly-Glu), glutamic acid-alanine-alanine (Glu-Ala-Ala), glycine-lysine (Gly-Lys), glycine-valine-citrulline (Gly-Val-Cit) andglycine-glycine-glycine (Gly-Gly-Gly) and

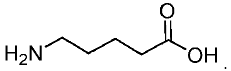

In certain embodiments, the linker comprises a stretcher unit, which is a chemical structural fragment, one end of which is covalently linked to an antibody by carbon atoms, and the other end is linked to an amino acid unit, a disulfide moiety, a sulfonamide moiety or a non-peptide chemical moiety. Exemplary stretcher units include, but are not limited to,

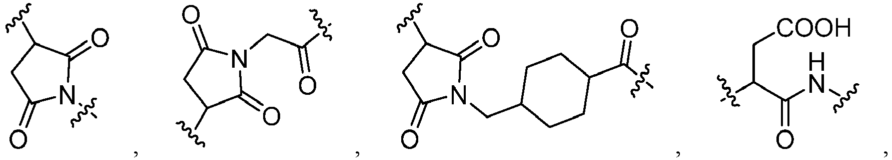

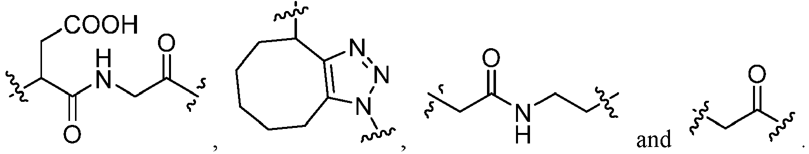

In certain embodiments, the stretcher unit is selected from the group consisting of 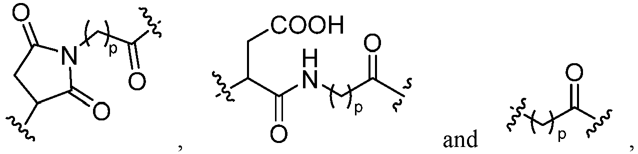 wherein each p is independently 1, 2, 3, 4, 5 or 6.

In certain embodiments, the linker is selected from the group consisting of 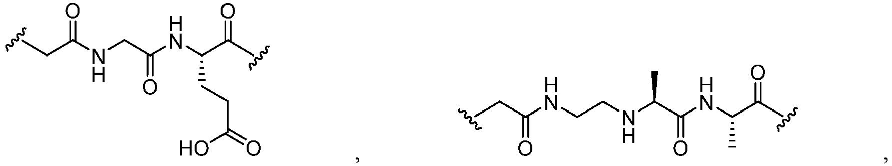 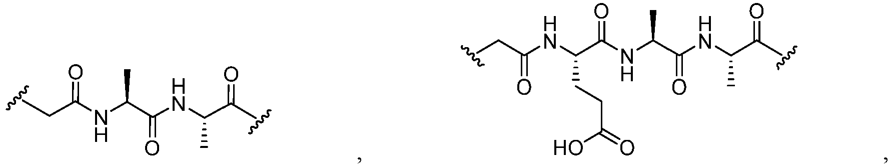 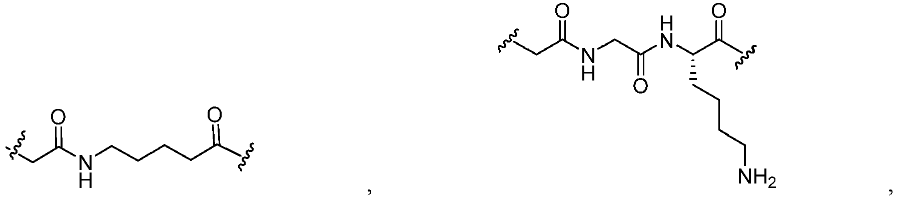 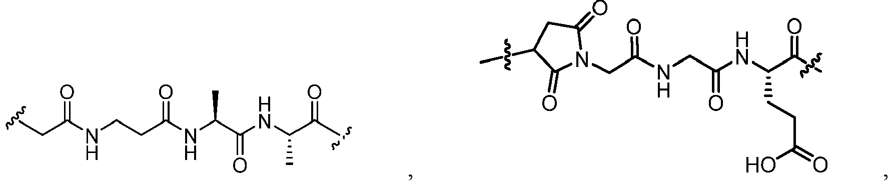 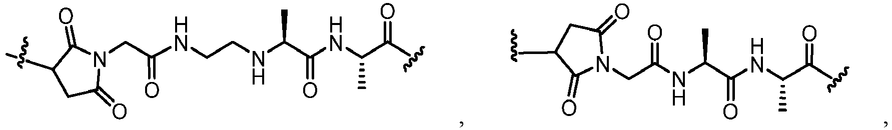 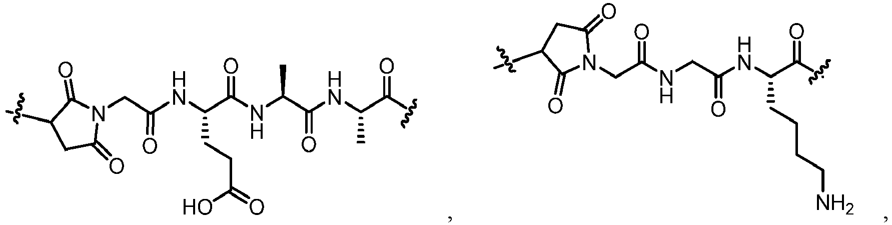 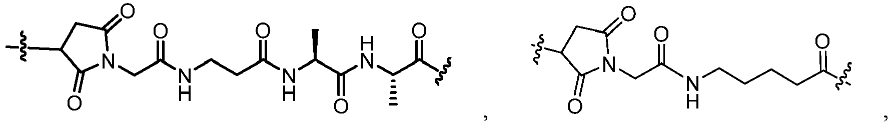 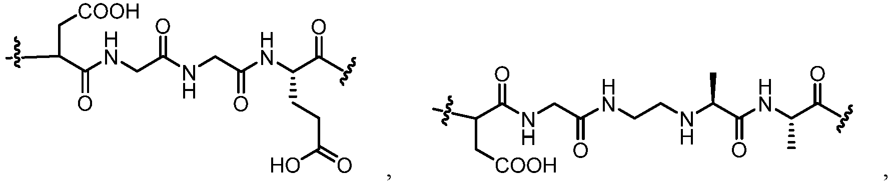 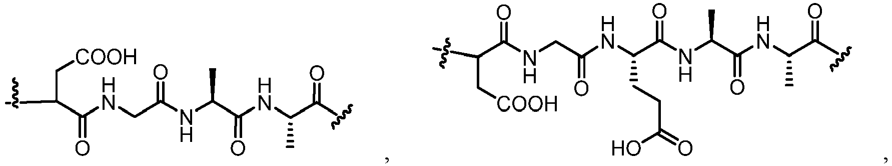 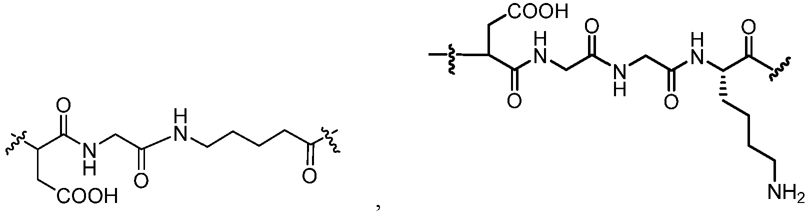 and

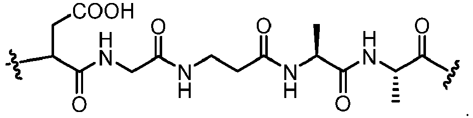

In certain embodiments, the linker is selected from the group consisting of 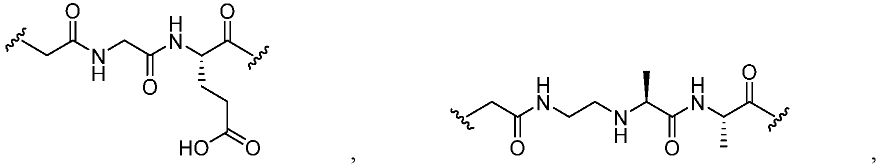 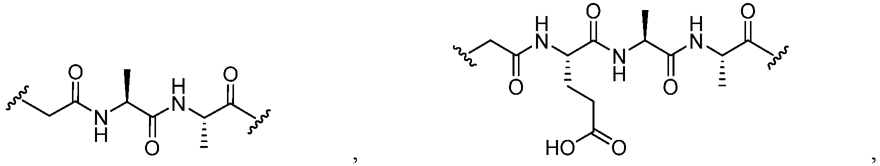 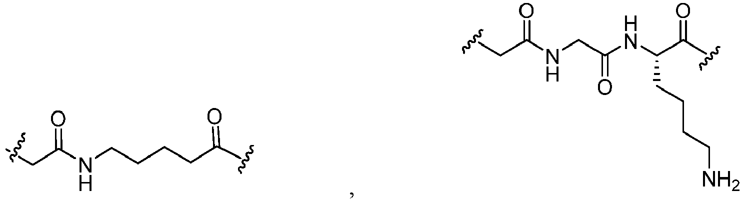 and

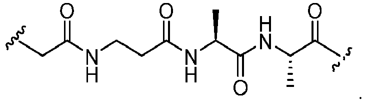

In certain embodiments, the antibody-drug conjugate is selected from the group consisting of: 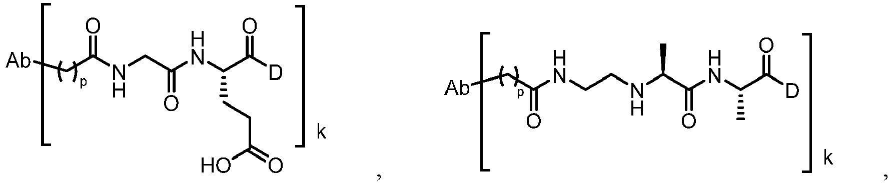 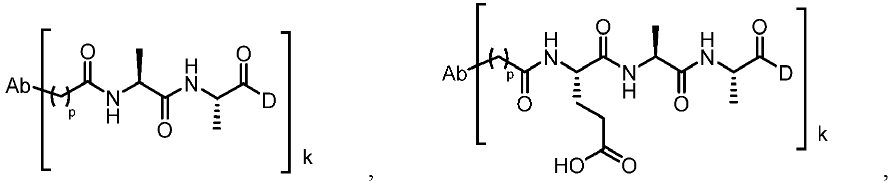 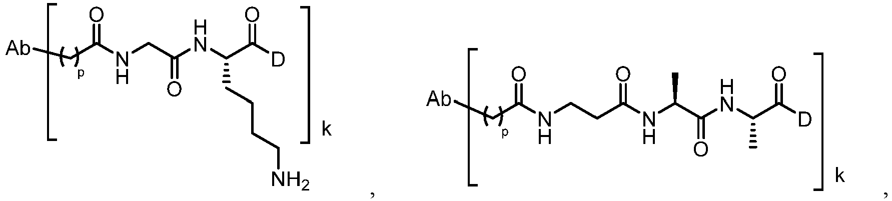 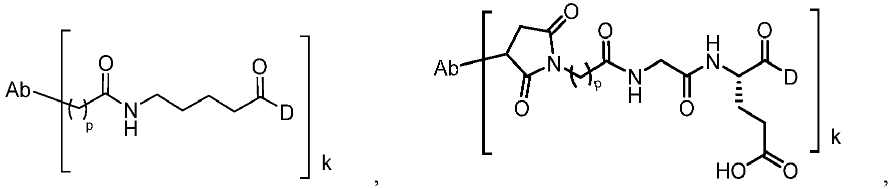 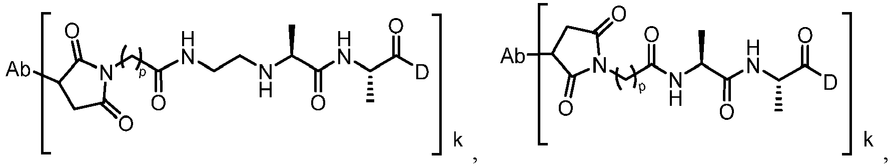 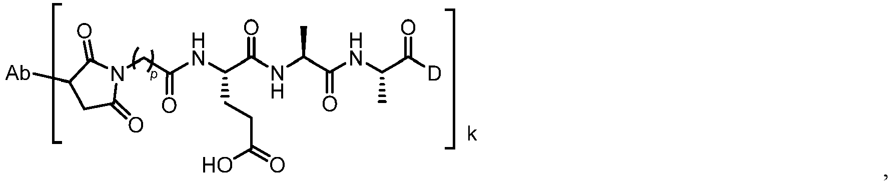 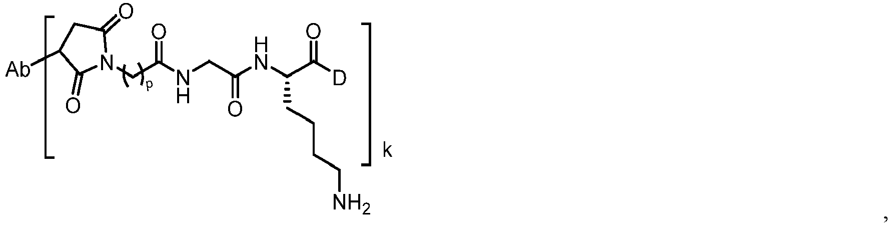 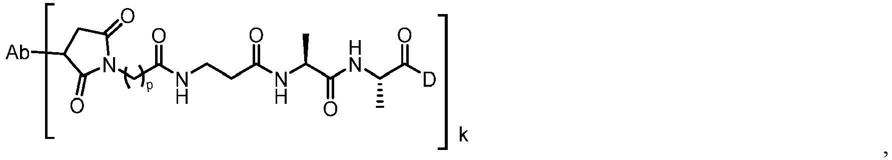 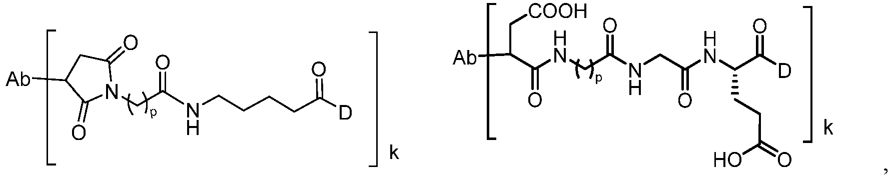 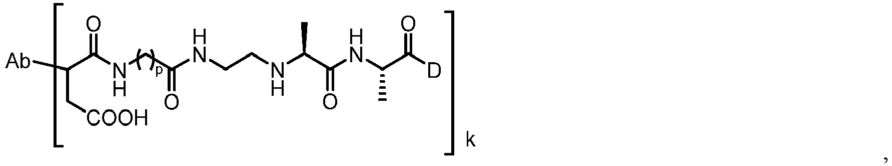 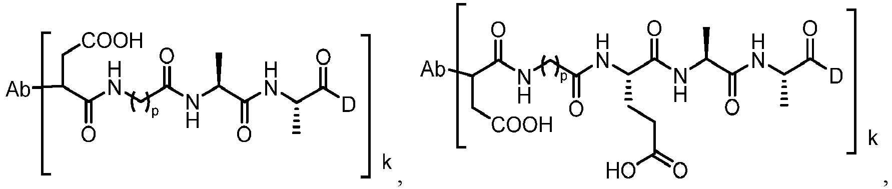 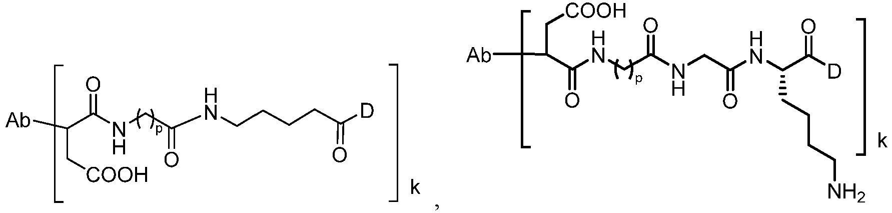 and 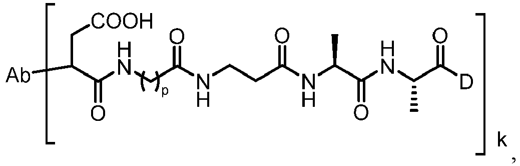 wherein Ab, D and k are as previously defined and each p is independently 1, 2, 3, 4, 5 or 6.

In certain embodiments, the antibody-drug conjugate is selected from the group consisting of 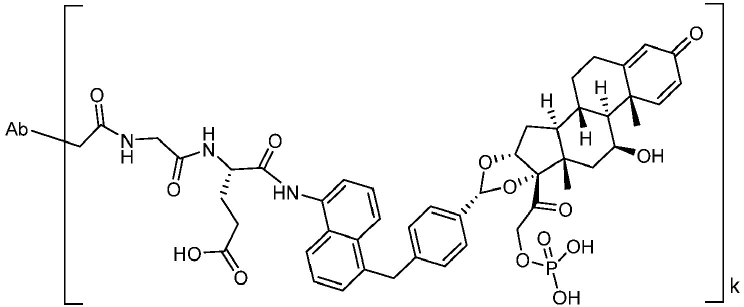 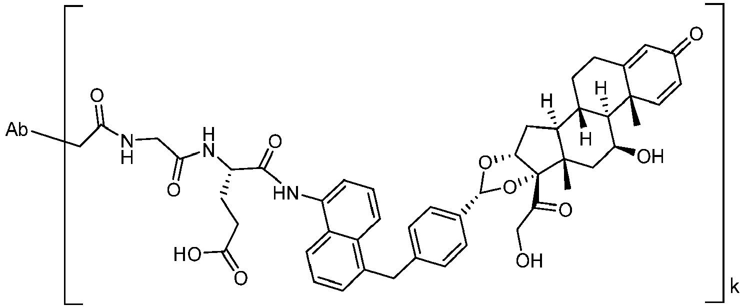 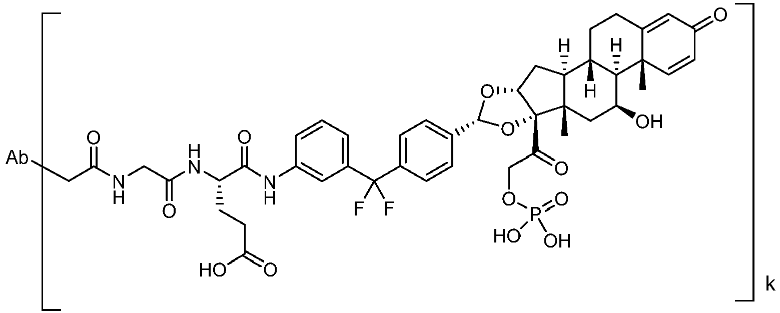 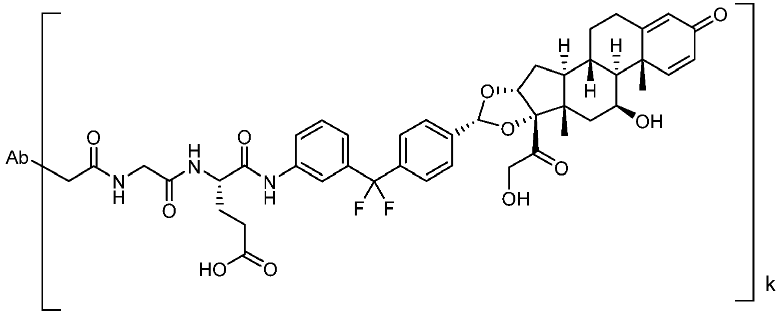 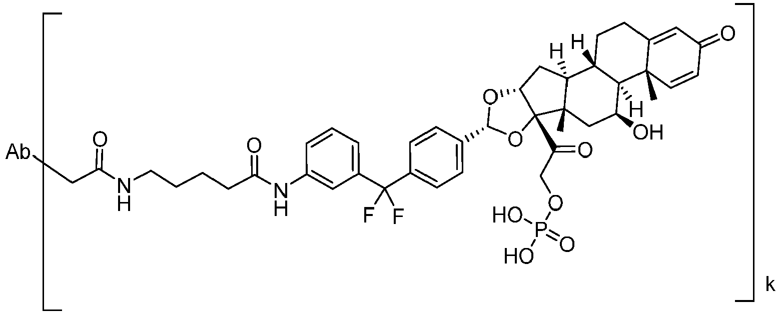 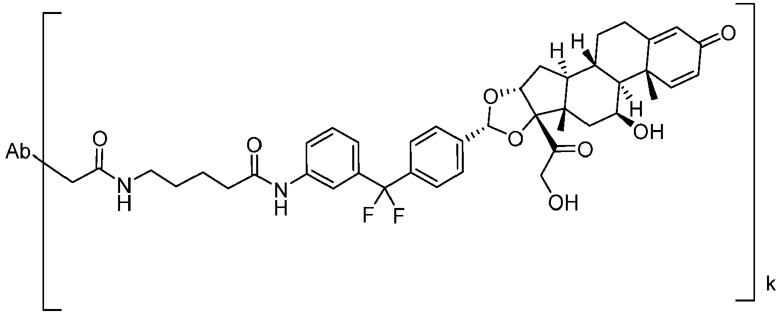 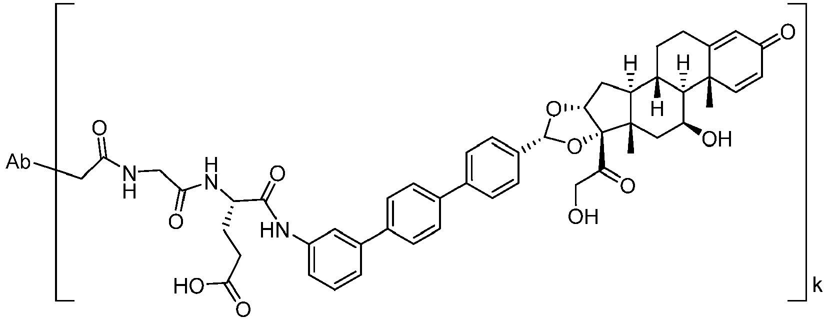 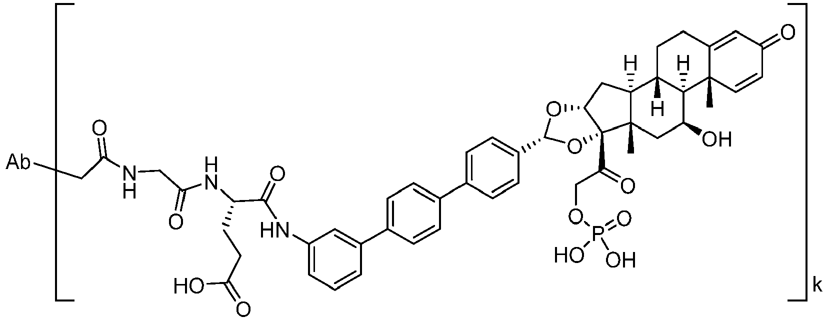 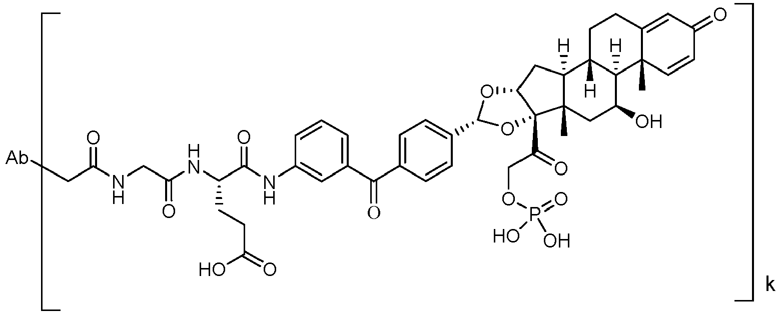 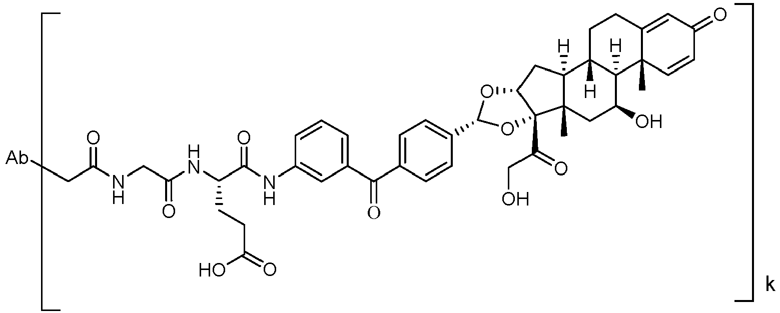 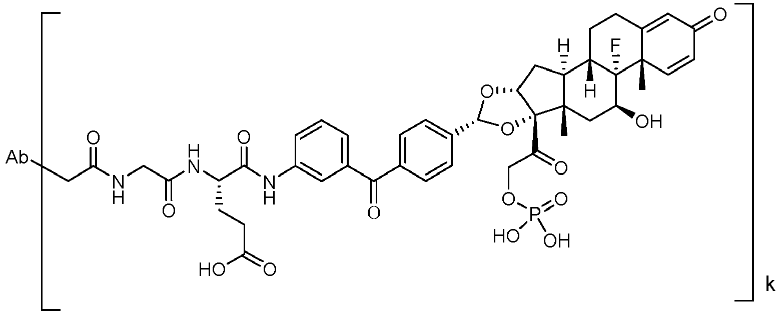 and 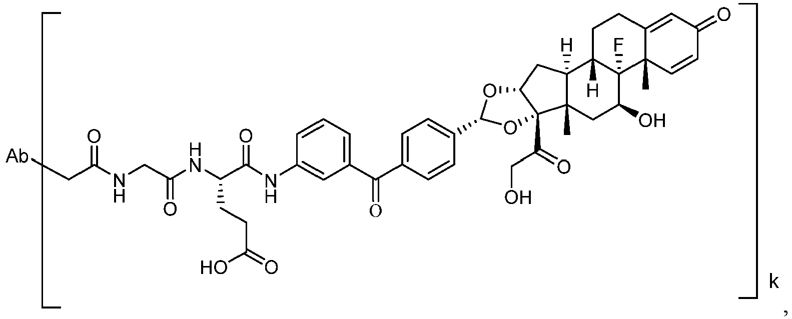 wherein k is selected from the group consisting of 1 to 10 and may be an integer or a decimal.

In another aspect, the antibody or the antigen-binding fragment thereof in the antibody-drug conjugate (ADC) of the present disclosure is selected from the group consisting of a murine antibody, a chimeric antibody, a humanized antibody and a fully human-derived antibody or an antigen-binding fragment thereof.

In certain embodiments, the antibody or the antigen-binding fragment thereof is selected from the group consisting of an anti-TNFα antibody, an anti-IL-4R antibody, an anti-IL-6/IL-6R antibody, an anti-IL-13R antibody, an anti-IL-17/IL-17R antibody, an anti-IL-23/IL23R antibody, an anti-IL-36R antibody, an anti-CD20 antibody, an anti-CD22 antibody, an anti-CD28 antibody, an anti-CD40 antibody and an anti-TSLP antibody or antigen-binding fragments thereof.

In certain embodiments, the antibody or the antigen-binding fragment thereof binds to human and/or mouse TNFα. The antibody and the antigen-binding fragment that bind to TNFα are known in the art.

In certain embodiments, the anti-TNFα antibody or the antigen-binding fragment does not bind to TNF-β.

The anti-TNFα antibody and the antigen-binding fragment thereof includes, for example, adalimumab, infliximab, certolizumab pegol, afelimomab, nerelimomab, ozoralizumab, placulumab and golimumab or antigen-binding fragments thereof. Additional anti-TNFα antibodies and antigen-binding fragments are provided in, for example, WO 2013/087912, WO 2014/152247 and WO 2015/073884, each of which is incorporated herein by reference in its entirety.

The anti-TNFα antibody and the antigen-binding fragment thereof further includes antibodies and antigen-binding fragments thereof that competitively inhibit the binding of adalimumab, infliximab, certolizumab pegol, afelimomab, nerelimomab, ozoralizumab, placulumab or golimumab to TNFα. The anti-TNFα antibody and the antigen-binding fragment thereof further includes antibodies and antigen-binding fragments that bind to the same TNFα epitope as adalimumab, infliximab, certolizumab pegol, afelimomab, nerelimomab, ozoralizumab, placulumab or golimumab.

In certain embodiments, the anti-TNFα antibody or the antigen-binding fragment thereof competitively inhibits the binding of adalimumab to TNFα. In certain embodiments, the anti-TNFα antibody or the antigen-binding fragment thereof binds to the same TNFα epitope as adalimumab. In certain embodiments, the anti-TNFα antibody or the antigen-binding fragment thereof is adalimumab or an antigen-binding fragment thereof. In certain embodiments, the anti-TNFα antibody or the antigen-binding fragment thereof is adalimumab.

In certain embodiments, the anti-TNFα antibody or the antigen-binding fragment thereof comprises a sequence of adalimumab, infliximab, certolizumab pegol, afelimomab, nerelimomab, ozoralizumab, placulumab or golimumab, for example, the complementarity determining regions (CDRs), the variable heavy chain domain (VH) and/or the variable light chain domain (VL).

The present disclosure further provides a compound of formula (III-A) or (III-B) or a pharmaceutically acceptable salt thereof, 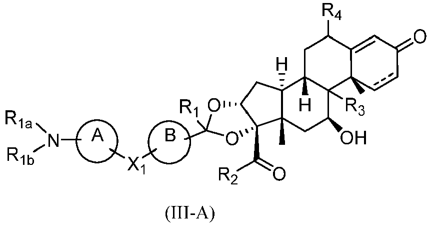 or 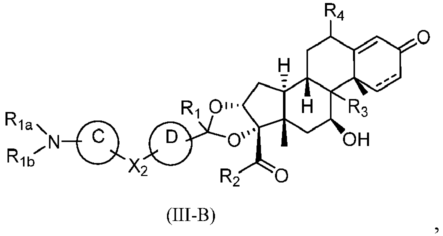 wherein,
-̅ -̅ -̅ represents a single bond or a double bond;
ring A is aryl or heteroaryl optionally substituted with one or more Qi substituents;
ring B is aryl or heteroaryl optionally substituted with one or more Qi substituents;
X₁ is -(CR₅ₐR_{5b})m- or is aryl or heteroaryl optionally substituted with one or more Qi substituents;
R₅ₐ and R_{5b} are each independently selected from the group consisting of hydrogen, halogen, hydroxy, sulfhydryl, deuterium, nitro, cyano, and the following groups optionally substituted with one or more Qi substituents: alkyl, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, alkoxy, alkylthio, alkenyl and alkynyl, or together R₅ₐ and R_{5b} form oxo or thio;
ring C and ring D are each independently selected from the group consisting of aryl and heteroaryl or fused heteroaryl optionally substituted with one or more Qi substituents, and at least one of ring C and ring D is selected from the group consisting of fused cycloaryl and fused heteroaryl optionally substituted with one or more Qi substituents;
X₂ is selected from the group consisting of -(CR₆ₐR_{6b})n-, aryl or heteroaryl optionally substituted with one or more Qi substituents, -O-, -S-, -S(O)-, -S(O)(O)-, -NR_{6c}-,
-CH₂S-, -CH₂O-, -NHCR_{6d}R₆ₑ-, -CR_{6f}=CR_{6g}- and -C=C-, or X₂ is absent;
R₆ₐ and R_{6b} are each independently selected from the group consisting of hydrogen, halogen, hydroxy, sulfhydryl, deuterium, nitro, cyano, and the following groups optionally substituted with one or more Qi substituents: alkyl, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, alkoxy, alkylthio, alkenyl and alkynyl, or R₆ₐ and R_{6b}, together with the carbon atom to which they are attached, form 3- to 10-membered cycloalkyl, or together R₆ₐ and R_{6b} form oxo or thio;
R_{6c}, R_{6d}, R₆ₑ, R_{6f} and R_{6g} are each independently selected from the group consisting of hydrogen, C₁-C₆ alkyl, C₁-C₆ haloalkyl and C₁-C₆ alkoxy;
each R₁ is independently selected from the group consisting of hydrogen, alkyl and alkoxy, wherein the alkyl and alkoxy are each independently optionally substituted with one or more substituents selected from the group consisting of alkyl, alkoxy, halogen, deuterium, amino, cyano, nitro, hydroxy and hydroxyalkyl;
each R₂ is independently selected from the group consisting of -CH₂OH, -CH₂SH, -CH₂Cl, -SCH₂Cl, -SCH₂F, -SCH₂CF₃, -OH, -OCH₂CN, -OCH₂Cl, -OCH₂F, -OCH₃, -OCH₂CH₃, -SCH₂CN, 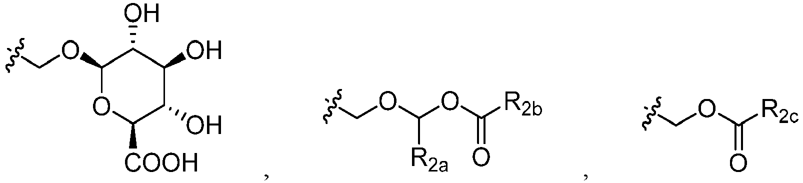 and 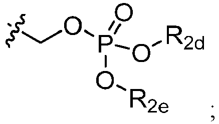
each R₂ₐ is independently hydrogen or C₁-C₆ alkyl;
each R_{2b} is independently C₁-C₆ alkyl or C₁-C₆ alkoxy;
each R_{2c} is independently selected from the group consisting of hydrogen, C₁-C₆ alkyl, -CH₂OH and C₁-C₆ alkoxy;
R_{2d} and R₂ₑ are each independently hydrogen or C₁-C₆ alkyl;
each R₃ is independently hydrogen or a halogen;
each R₄ is independently selected from the group consisting of hydrogen, halogen and hydroxy;
m and n are each independently selected from an integer of 1 to 6;
the Qi substituents are each independently selected from the group consisting of C₁-C₆ alkyl, halogen, deuterium, hydroxy, sulfhydryl, -NRᵢRⱼ, oxo, thio, -C(O)Rₖ, -C(O)ORₖ,
-S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthio, C₂-C₆ alkenyl, C₂-C₆ alkynyl, 3- to 10-membered cycloalkyl, 3- to 10-membered heterocyclyl, 6- to 10-membered aryl, 5- to 10-membered heteroaryl, 8-to 12-membered fused cycloaryl, and 5- to 12-membered fused heteroaryl;
Rᵢ and Rⱼ are each independently selected from the group consisting of hydrogen, hydroxy, C₁-C₆ alkyl and C₁-C₆ alkoxy;
Rₖ is independently selected from the group consisting of hydrogen, C₁-C₆ alkyl, C₁-C₆ haloalkyl, C₁-C₆ alkoxy, hydroxy and -NRᵢRⱼ, wherein the alkyl, alkoxy and haloalkyl are each independently optionally substituted with one or more substituents selected from the group consisting of C₁-C₆ alkyl, halogen, hydroxy, sulfhydryl, -NRᵢRⱼ, oxo, thio, carboxyl, nitro, cyano, C₁-C₆ alkoxy, C₁-C₆ alkylthio, C₂-C₆ alkenyl, C₂-C₆ alkynyl, 3- to 10-membered cycloalkyl, 3- to 10-membered heterocyclyl, 6- to 10-membered aryl, and 5- to 10-membered heteroaryl;
each R₁ₐ is independently selected from the group consisting of hydrogen, alkyl and alkoxy, and the alkyl and alkoxy are each independently optionally substituted with one or more substituents selected from the group consisting of alkyl, alkoxy, halogen, deuterium, amino, cyano, nitro, hydroxy and hydroxyalkyl;
each R_{1b} is independently selected from the group consisting of hydrogen, PG-, H-L₁-, PG-L₁-, 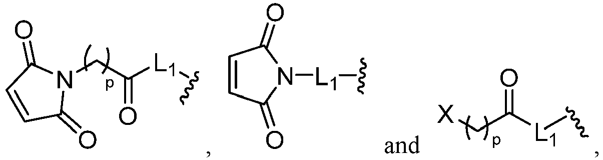 or
R₁ₐ and R_{1b}, together with the nitrogen atom to which they are attached, form: 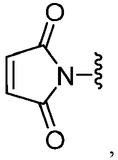 or R₁ₐ and R_{1b}, together with the nitrogen atom to which they are attached, form a nitro group;
each p is independently 1, 2, 3, 4, 5 or 6;
Li is an amino acid unit, preferably -glycine-glutamic acid- or 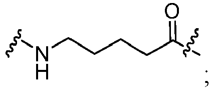
X is a halogen;
PG is an amino protecting group;
provided that when R₅ₐ is hydrogen or alkyl, R_{5b} is not hydrogen or alkyl.

In certain embodiments, each R₁ₐ is independently selected from the group consisting of hydrogen, C₁-C₆ alkyl and C₁-C₆ alkoxy, and the alkyl and alkoxy are each independently optionally substituted with one or more substituents selected from the group consisting of C₁-C₆ alkyl, C₁-C₆ alkoxy, halogen, deuterium, amino, cyano and hydroxy.

In certain embodiments, each R₁ₐ is independently selected from the group consisting of hydrogen, C₁-C₆ alkyl and C₁-C₆ alkoxy.

In certain embodiments, Li is selected from the group consisting of -glycine-glutamic acid- and

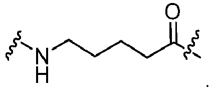

In certain embodiments, each R_{1b} is independently selected from the group consisting of hydrogen, PG-, H-L₁-, PG-L₁-, 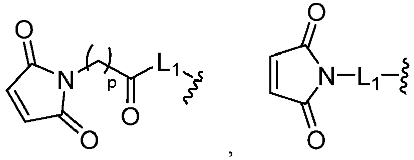 and

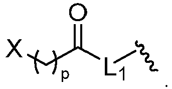

In certain embodiments, PG is Boc or Cbz.

In certain embodiments, ring A is 6- to 10-membered aryl or 5- to 10-membered heteroaryl optionally substituted with one or more Qi substituents, and the heteroaryl comprises at least one nitrogen atom.

In certain embodiments, ring A is 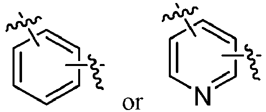 optionally substituted with one or more Qi substituents.

In certain embodiments, ring B is 6- to 10-membered aryl or 5- to 10-membered heteroaryl optionally substituted with one or more Qi substituents, and the heteroaryl comprises at least one nitrogen atom.

In certain embodiments, ring B is 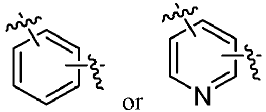 optionally substituted with one or more Qi substituents.

In certain embodiments, X₁ is -(CR₅ₐR_{5b})m- or is 6- to 10-membered aryl or 5- to 10-membered heteroaryl optionally substituted with one or more Qi substituents, and the heteroaryl comprises at least one nitrogen atom;
In certain embodiments, R₅ₐ and R_{5b} are each independently selected from the group consisting of hydrogen, halogen, hydroxy, sulfhydryl, deuterium, nitro, cyano, and the following groups optionally substituted with one or more Qi substituents: C₁-C₆ alkyl, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, C₁-C₆ alkoxy, C₁-C₆ alkylthio, C₂-C₆ alkenyl and C₂-C₆ alkynyl, or together R₅ₐ and R_{5b} form oxo or thio.

In certain embodiments, R₅ₐ and R_{5b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ, -S(O)Rₖ, -S(O)ORₖ, -S(O)(O)Rₖ, -S(O)(O)ORₖ, C₁-C₆ alkoxy, C₁-C₆ alkylthio, C₂-C₆ alkenyl and C₂-C₆ alkynyl, or together R₅ₐ and R_{5b} form oxo or thio.

In certain embodiments, R₅ₐ and R_{5b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ, -S(O)Rₖ, -S(O)(O)Rₖ, C₁-C₆ alkoxy, C₂-C₆ alkenyl and C₂-C₆ alkynyl, or together R₅ₐ and R_{5b} form oxo or thio.

In certain embodiments, R₅ₐ and R_{5b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy, or together R₅ₐ and R_{5b} form oxo or thio. In certain embodiments, ring C and ring D are each independently selected from the group consisting of 6- to 10-membered aryl, 5- to 10-membered heteroaryl, 8- to 12-membered fused cycloaryl and 5- to 12-membered fused heteroaryl optionally substituted with one or more Qi substituents, and the heteroaryl or fused heteroaryl comprises at least one nitrogen atom.

In certain embodiments, ring C is selected from the group consisting of the following groups optionally substituted with one or more Qi substituents:

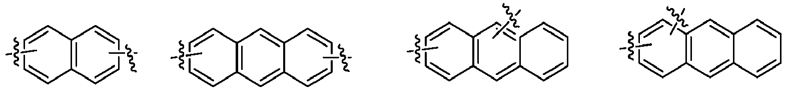

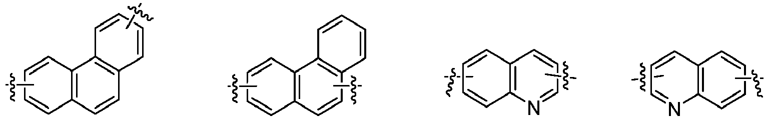

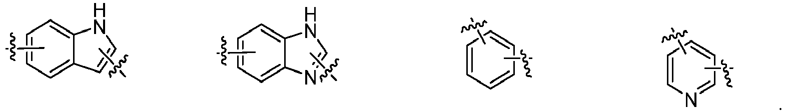

In certain embodiments, ring C is selected from the group consisting of: 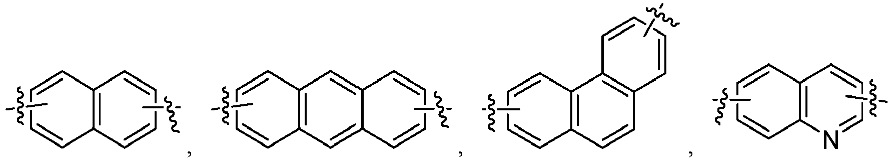 and 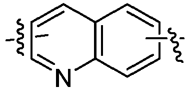 optionally substituted with one or more Qi substituents.

In certain embodiments, ring D is 6- to 10-membered aryl or 5- to 10-membered heteroaryl optionally substituted with one or more Qi substituents, and the heteroaryl comprises at least one nitrogen atom.

In certain embodiments, ring D is 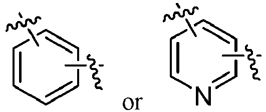 optionally substituted with one or more Qi substituents.

In certain embodiments, X₂ is -(CR₆ₐR_{6b})n- or is 6- to 10-membered aryl or 5- to 10-membered heteroaryl optionally substituted with one or more Qi substituents, and the heteroaryl comprises at least one nitrogen atom.

In certain embodiments, R₆ₐ and R_{6b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ, -S(O)Rₖ, -S(O)ORₖ, -S(O)(O)Rₖ, -S(O)(O)ORₖ, C₁-C₆ alkoxy, C₁-C₆ alkylthio, C₂-C₆ alkenyl and C₂-C₆ alkynyl, or R₆ₐ and R_{6b}, together with the carbon atom to which they are attached, form 3- to 10-membered cycloalkyl, or together R₆ₐ and R_{6b} form oxo or thio.

In certain embodiments, R₆ₐ and R_{6b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ, -S(O)Rₖ, -S(O)(O)Rₖ, C₁-C₆ alkoxy, C₂-C₆ alkenyl and C₂-C₆ alkynyl, or R₆ₐ and R_{6b}, together with the carbon atom to which they are attached, form 3- to 10-membered cycloalkyl, or together R₆ₐ and R_{6b} form oxo or thio.

In certain embodiments, R₆ₐ and R_{6b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy, or together R₆ₐ and R_{6b} form oxo or thio. In certain embodiments, each R₁ is independently selected from the group consisting of hydrogen, C₁-C₆ alkyl and C₁-C₆ alkoxy, preferably hydrogen.

In certain embodiments, each R₄ is independently hydrogen.

In certain embodiments, the Qi substituents are each independently selected from the group consisting of halogen, hydroxy, sulfhydryl, deuterium, cyano, amino, carboxyl, C₁-C₆ alkyl and C₁-C₆ alkoxy.

In certain embodiments, Rₖ is independently selected from the group consisting of hydrogen, C₁-C₆ alkyl, C₁-C₆ haloalkyl, C₁-C₆ alkoxy, hydroxy and -NRᵢRⱼ.

In certain embodiments, the compound of formula (III-A) or (III-B) is a compound of formula (III-A') or (III-B'): 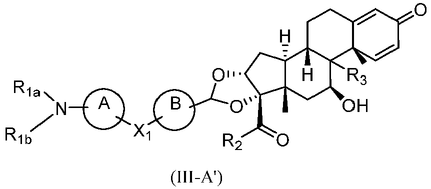 or 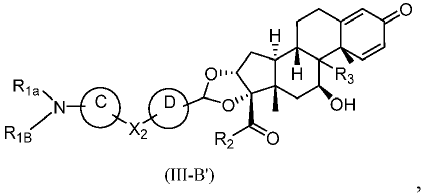 wherein,
ring A is 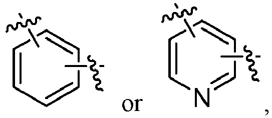 and the ring A is optionally substituted with one or more Qi substituents;
ring B is 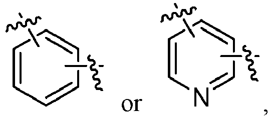 and the ring B is optionally substituted with one or more Qi substituents;
X₁ is selected from the group consisting of -(CR₅ₐR_{5b})m- and 6- to 10-membered aryl or 5- to 10-membered heteroaryl optionally substituted with one or more Qi substituents, and the heteroaryl comprises at least one nitrogen atom;
R₅ₐ and R_{5b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy, or together R₅ₐ and R_{5b} form oxo or thio;
ring C is selected from the group consisting of 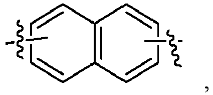 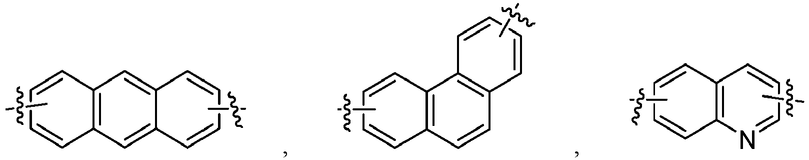 and 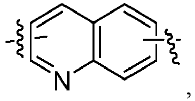 and the ring C is optionally substituted with one or more Qi substituents;
ring D is 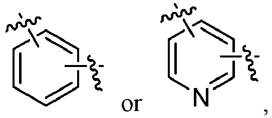 and the ring D is optionally substituted with one or more Qi substituents;
X₂ is selected from the group consisting of -(CR₆ₐR_{6b})n-, -O-, -S-, -NR_{6c}-, -CH₂S-, -CH₂O-, -NHCR_{6d}R₆ₑ-, and 6- to 10-membered aryl or 5- to 10-membered heteroaryl optionally substituted with one or more Qi substituents;
R₆ₐ and R_{6b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy, or together R₆ₐ and R_{6b} form oxo or thio;
R_{6c}, R_{6d} and R₆ₑ are each independently selected from the group consisting of hydrogen, C₁-C₆ alkyl, C₁-C₆ haloalkyl and C₁-C₆ alkoxy;
each R₂ is independently selected from the group consisting of -CH₂OH, -CH₂SH, -CH₂Cl, -SCH₂Cl, -SCH₂F, -SCH₂CF₃, -OH, -OCH₂CN, -OCH₂Cl, -OCH₂F, -OCH₃, -OCH₂CH₃, -SCH₂CN, 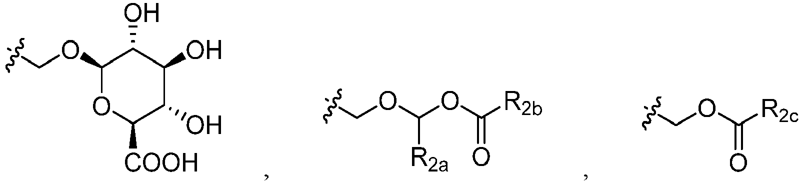 and 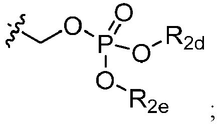
each R₂ₐ is independently hydrogen or C₁-C₆ alkyl;
each R_{2b} is independently C₁-C₆ alkyl or C₁-C₆ alkoxy;
each R_{2c} is independently selected from the group consisting of hydrogen, C₁-C₆ alkyl, -CH₂OH and C₁-C₆ alkoxy;
R_{2d} and R₂ₑ are each independently hydrogen or C₁-C₆ alkyl;
each R₃ is independently hydrogen or a halogen;
m and n are each independently an integer of 1 to 6;
the Qi substituents are each independently selected from the group consisting of halogen, hydroxy, sulfhydryl, deuterium, oxo, thio, cyano, amino, carboxyl, C₁-C₆ alkyl and C₁-C₆ alkoxy;
Rᵢ and Rⱼ are each independently selected from the group consisting of hydrogen, hydroxy, C₁-C₆ alkyl and C₁-C₆ alkoxy;
Rₖ is independently selected from the group consisting of hydrogen, C₁-C₆ alkyl, C₁-C₆ haloalkyl, C₁-C₆ alkoxy, hydroxy and -NRᵢRⱼ;
each R₁ₐ is independently selected from the group consisting of hydrogen, C₁-C₆ alkyl and C₁-C₆ alkoxy;
each R_{1b} is independently selected from the group consisting of hydrogen, PG-, H-L₁-, PG-L₁-, 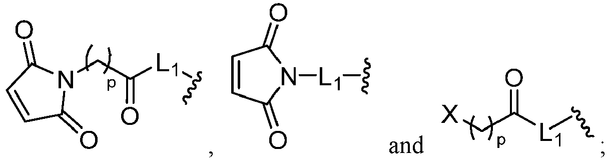
each p is independently 1, 2, 3, 4, 5 or 6;
Li is an amino acid unit selected from the group consisting of -glycine-glutamic acid-and 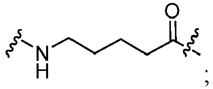
X is a halogen;
PG is an amino protecting group;
provided that when R₅ₐ is hydrogen or alkyl, R_{5b} is not hydrogen or alkyl.

In certain embodiments, R₁ₐ is hydrogen.

In certain embodiments, each R_{1b} is independently selected from the group consisting of hydrogen, PG-, H-L₁-, PG-L₁-, 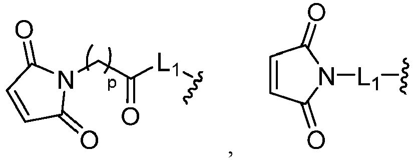 and 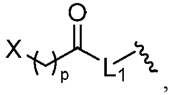 preferably hydrogen or

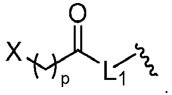

In certain embodiments, R₁ₐ and R_{1b} are both hydrogen.

In certain embodiments, X₁ is selected from the group consisting of -(CR₅ₐR_{5b})m-, and 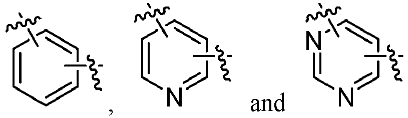 optionally substituted with one or more Qi substituents.

In certain embodiments, R₅ₐ and R_{5b} are both fluorine.

In certain embodiments, together R₅ₐ and R_{5b} form oxo or thio, preferably oxo.

In certain embodiments, X₂ is selected from the group consisting of -(CR₆ₐR_{6b})n-, and 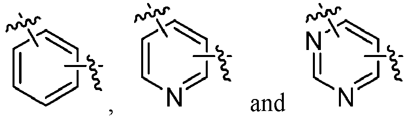 optionally substituted with one or more Qi substituents.

In certain embodiments, R₆ₐ and R_{6b} are each independently selected from the group consisting of hydrogen, halogen, hydroxy, sulfhydryl, deuterium, cyano, C₁-C₆ alkyl, -NRᵢRⱼ, -C(O)ORₖ and C₁-C₆ alkoxy, or together R₆ₐ and R_{6b} form oxo or thio.

In certain embodiments, each R₂ is independently selected from the group consisting of -CH₂OH, -CH₂SH, -OH and

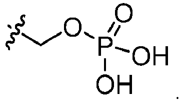

In certain embodiments, R₃ is hydrogen.

In certain embodiments, R₃ is fluorine.

In certain embodiments, each p is independently 1 or 2, preferably 1.

In certain embodiments, the compound of formula (III-A) or (III-B) is selected from the group consisting of 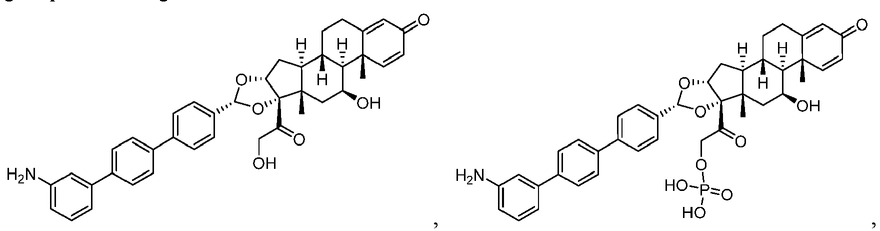 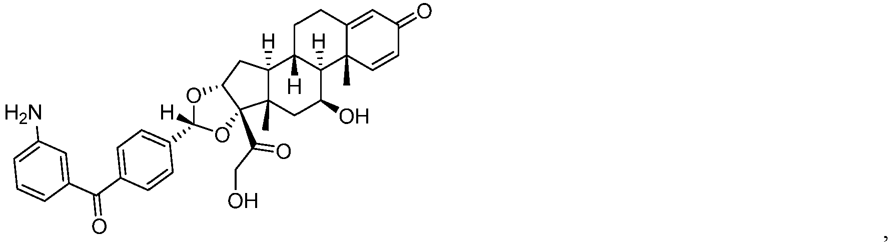 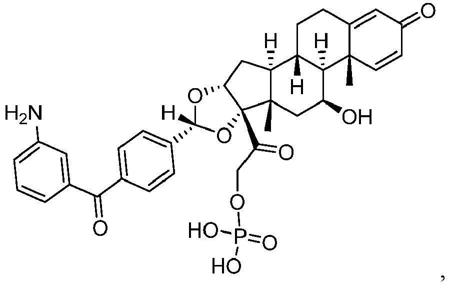 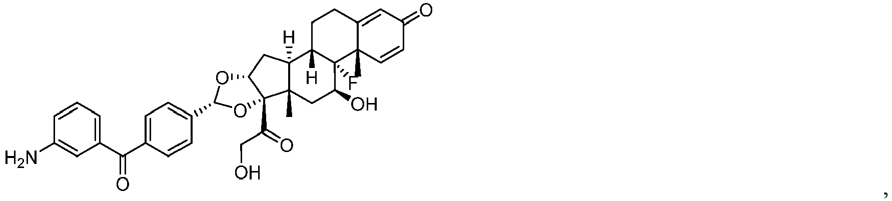 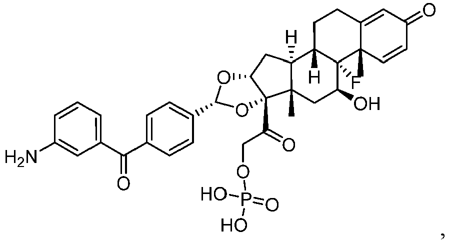 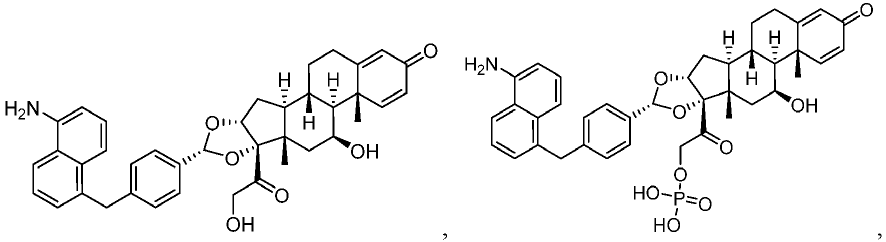 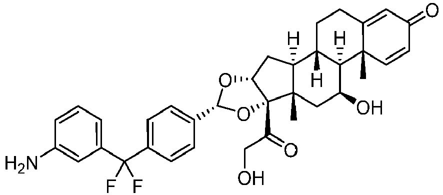 and 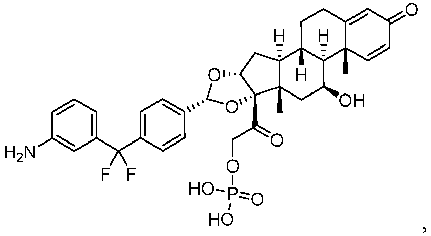 or pharmaceutically acceptable salts thereof.

In certain embodiments, the compound of formula (III-A) or (III-B) is selected from the group consisting of 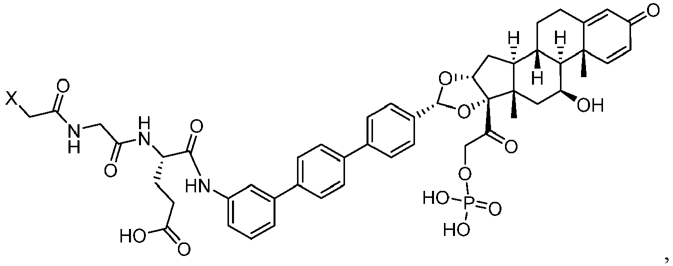 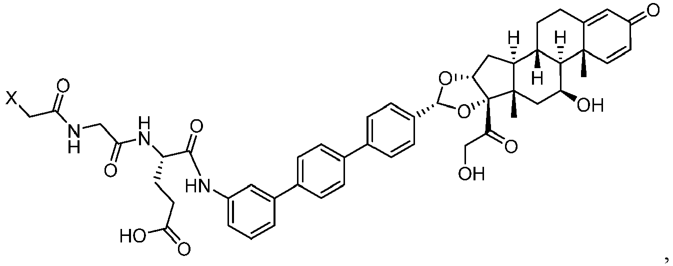 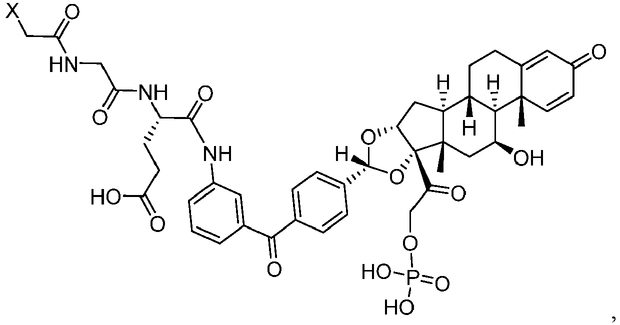 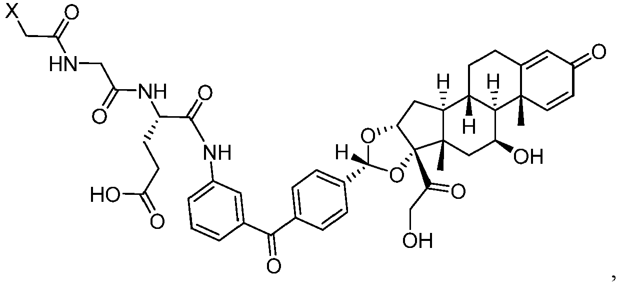 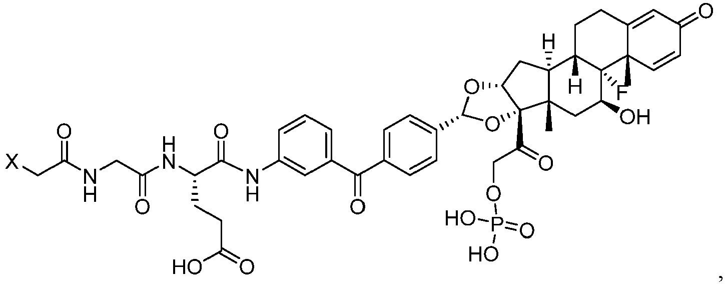 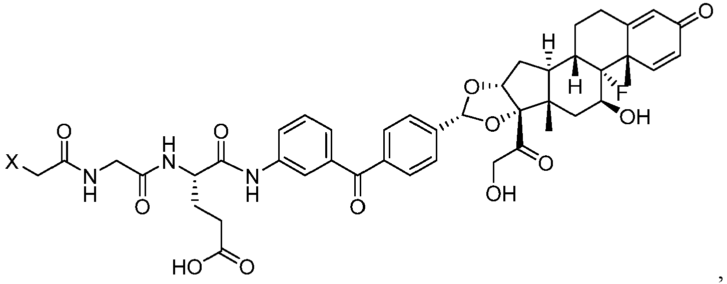 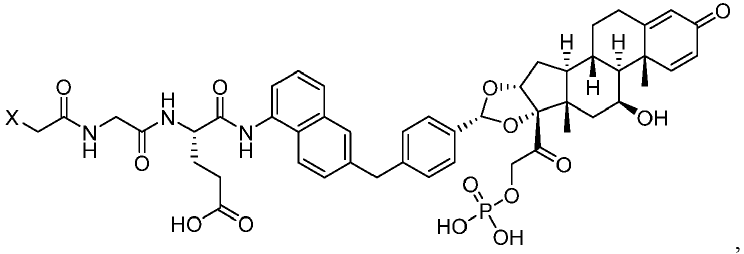 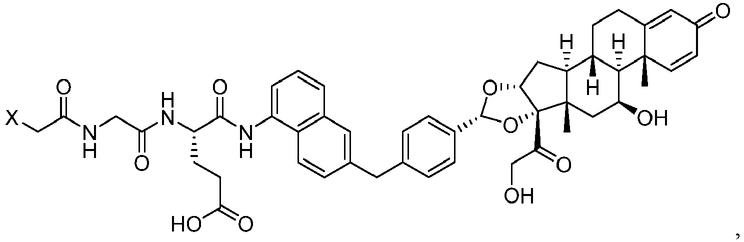 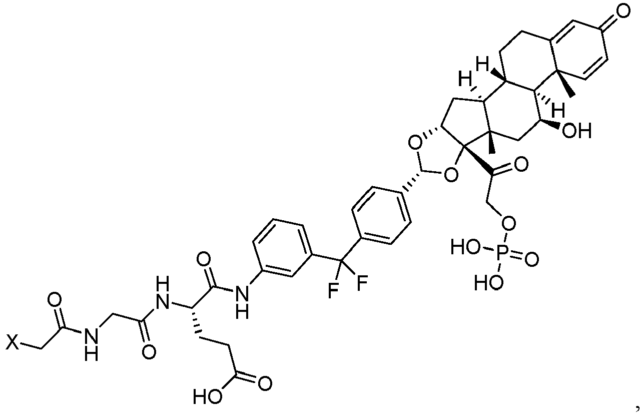 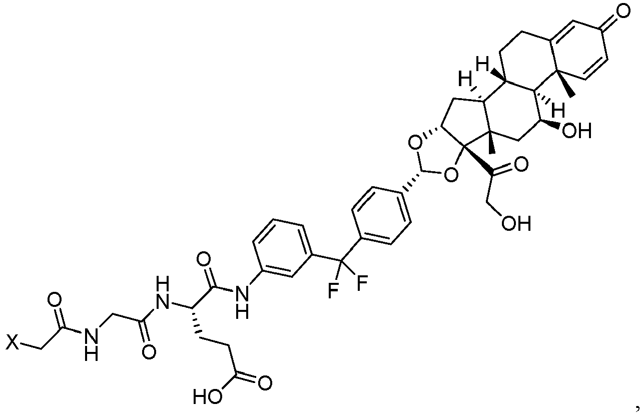 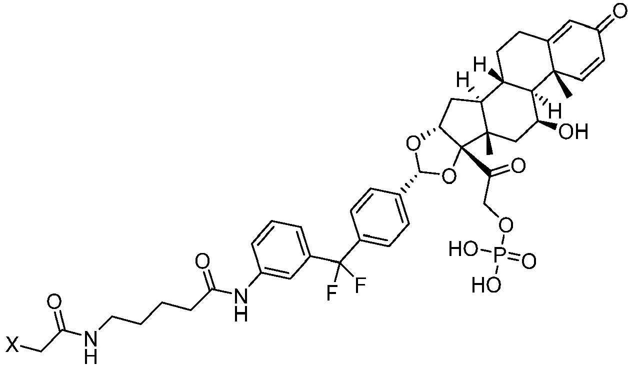 and 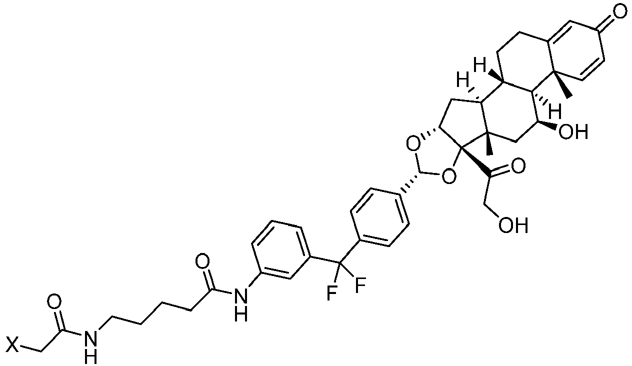 or pharmaceutically acceptable salts thereof, wherein X is a halogen, preferably chlorine or bromine, and more preferably bromine.

In the structures of the present disclosure, -̅ -̅ -̅ represents a single bond or a double bond.

As will be appreciated by those skilled in the art, for example, when one of R₅ₐ and R_{5b} is selected from the group consisting of oxo and thio, the other is absent.

The present disclosure further provides a pharmaceutical composition comprising at least one of the antibody-drug conjugates described above, and a pharmaceutically acceptable carrier, diluent or excipient.

In certain embodiments, a unit dose of the pharmaceutical composition is 0.001 mg-1000 mg.

In certain embodiments, the pharmaceutical composition comprises 0.01%-99.99% of the antibody-drug conjugate described above based on the total weight of the composition. In certain embodiments, the pharmaceutical composition comprises 0.1%-99.9% of the antibody-drug conjugate described above. In certain embodiments, the pharmaceutical composition comprises 0.5%-99.5% of the antibody-drug conjugate described above. In certain embodiments, the pharmaceutical composition comprises 1%-99% of the antibody-drug conjugate described above. In certain embodiments, the pharmaceutical composition comprises 2%-98% of the antibody-drug conjugate described above.

In certain embodiments, the pharmaceutical composition comprises 0.01%-99.99% of the pharmaceutically acceptable carrier, diluent or excipient based on the total weight of the composition. In certain embodiments, the pharmaceutical composition comprises 0.1%-99.9% of the pharmaceutically acceptable carrier, diluent or excipient. In certain embodiments, the pharmaceutical composition comprises 0.5%-99.5% of the pharmaceutically acceptable carrier, diluent or excipient. In certain embodiments, the pharmaceutical composition comprises 1%-99% of the pharmaceutically acceptable carrier, diluent or excipient. In certain embodiments, the pharmaceutical composition comprises 2%-98% of the pharmaceutically acceptable carrier, diluent or excipient.

The antibody-drug conjugate and/or the pharmaceutical composition comprising the antibody-drug conjugate of the present disclosure can be used to lyse cells expressing surface TNFα *(in vitro* or *in vivo)* and/or to treat diseases or disorders characterized by increased TNFα (e.g., increased TNFα in synovial fluid). In certain embodiments, the antibody-drug conjugate and/or the composition can be used to inhibit cytokine release *(in vitro* or *in vivo)* and/or to treat autoimmune or inflammatory diseases. In certain embodiments, the antibody-drug conjugate and/or the composition are/is used to treat Crohn's disease. In certain embodiments, the antibody-drug conjugate and/or the composition are/is used to treat ulcerative colitis. In certain embodiments, the antibody-drug conjugate and/or the composition are/is used to treat rheumatoid arthritis (RA). In certain embodiments, the antibody-drug conjugate and/or the composition are/is used to treat juvenile idiopathic arthritis (JA). In certain embodiments, the antibody-drug conjugate and/or the composition are/is used to treat psoriatic arthritis (PsA). In certain embodiments, the antibody-drug conjugate and/or the composition are/is used to treat a spondyloarthropathy, such as ankylosing spondylitis (AS) or axial spondyloarthritis (axSpA). In certain embodiments, the antibody-drug conjugate and/or the composition are/is used to treat adult Crohn's disease (CD). In certain embodiments, the antibody-drug conjugate and/or the composition are/is used to treat pediatric Crohn's disease. In certain embodiments, the antibody-drug conjugate and/or the composition are/is used to treat ulcerative colitis (UC). In certain embodiments, the antibody-drug conjugate and/or the composition are/is used to treat plaque psoriasis (Ps). In certain embodiments, the antibody-drug conjugate and/or the composition are/is used to treat hidradenitis suppurativa (HS). In certain embodiments, the antibody-drug conjugate and/or the composition are/is used to treat uveitis. In certain embodiments, the antibody-drug conjugate and/or the composition are/is used to treat Behcet's disease. In certain embodiments, the antibody-drug conjugate and/or the composition are/is used to treat psoriasis, including plaque psoriasis.

The present disclosure further provides a method for delivering a glucocorticoid receptor agonist to a TNFα-expressing cell, which comprises the step of contacting the TNFα-expressing cell with the antibody-drug conjugate of the present disclosure.

The present disclosure further provides a method for determining the anti-inflammatory activity of an antibody-drug conjugate. Such methods may comprise the step of contacting a TNFα-expressing cell with an antibody-drug conjugate as described herein. Some embodiments comprise contacting a TNFα-expressing cell with an antibody-drug conjugate as described herein and determining reduced release of pro-inflammatory cytokines from the cell as compared to a control cell. Some embodiments comprise an *in vitro* method for determining the anti-inflammatory activity of an antibody-drug conjugate.

Some embodiments comprise a screening method (e.g., an *in vitro* method) that comprises contacting, directly or indirectly, a cell (e.g., a TNFα-expressing cell) with an antibody-drug conjugate and determining if the antibody-drug conjugate modulates an activity or function of the cell, as reflected, for example, by changes in cell morphology or viability, expression of a marker, differentiation or de-differentiation, cell respiration, mitochondrial activity, membrane integrity, maturation, proliferation, viability, apoptosis or cell death. One example of a direct interaction is a physical interaction, while an indirect interaction includes, for example, the action of a composition upon an intermediate molecule that in turn acts upon the referenced entity (e.g., a cell or cell culture).

The present disclosure further provides a pharmaceutical composition comprising at least one of the compounds of formula (III-A) or (III-B) or the pharmaceutically acceptable salt thereof described above, and a pharmaceutically acceptable carrier, diluent or excipient. The compound or the pharmaceutically acceptable salt thereof and the pharmaceutical composition thereof can be used to treat immune diseases.

The present disclosure further provides a kit comprising the antibody-drug conjugate or the pharmaceutical composition of the present disclosure.

### Definition of terms

Unless defined otherwise, all technical and scientific terms used herein have the same meaning as commonly understood by those of ordinary skill in the art to which the present disclosure belongs. Although any methods and materials similar or equivalent to those of the present disclosure can also be used to implement or test the present disclosure, preferred methods and materials are described herein. In describing and claiming the present disclosure, the following terms are used in accordance with the definitions below.

When a trade name is used in the present disclosure, the applicant intends to include the formulation of the commercial product under the trade name, and the non-patent drug and active drug component of the commercial product under the trade name. Unless otherwise stated, the terms used in the specification and claims have the following meanings.

The term "linker", "linker unit" or "linker fragment" refers to a chemical structural fragment or bond, which is linked to a ligand at one end and linked to a drug at the other end, and also may be linked to other linkers and then linked to the drug.

The linker may comprise one or more linker components. Exemplary linker components include 6-maleimidocaproyl (MC), maleimidopropionyl (MP), valine-citrulline (Val-Cit or vc), alanine-phenylalanine (ala-phe), p-aminobenzyloxycarbonyl (PAB), and those derived from coupling to a linker reagent: N-succinimidyl 4-(2-pyridylthio)pentanoate (SPP), N-succinimidyl 4-(N-maleimidomethyl)cyclohexane-1 carboxylate (SMCC, also referred to herein as MCC), and N-succinimidyl(4-iodo-acetyl)aminobenzoate (SIAB). The linker can include a stretcher unit, a spacer unit, an amino acid unit and an extension unit. The linker may be synthesized using methods known in the art, such as those described in US2005-0238649A1. The linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers can be used (Chari et al., Cancer Research 52: 127-131(1992); U.S. Patent No. 5,208,020).

The term "stretcher unit" refers to a chemical structural fragment, one end of which is covalently linked to an antibody by carbon atoms, and the other end is linked to an amino acid unit, a disulfide moiety, a sulfonamide moiety or a non-peptide chemical moiety.

The term "spacer unit" is a bifunctional compound structural fragment that can be used to couple an amino acid unit to a glucocorticoid to form an antibody-drug conjugate, in such a way that the glucocorticoid is selectively linked to the amino acid unit.

The term "amino acid" refers to an organic compound that contains amino and carboxyl in the molecular structure and in which both amino and carboxyl are directly linked to a -CH- structure. The general formula is H₂NCHRCOOH, where R is H, substituted or unsubstituted alkyl, and the like. Amino acids are classified as α, β, γ, δ, ε...-amino acids according to the position of the carbon atom to which the amino is linked in the carboxylic acid. In the biological field, the amino acids that constitute native proteins have their specific structural characteristics; that is, their amino groups are attached directly to the α-carbon atoms, namely α-amino acids, including Gly (Glycine), Ala (Alanine), Val (Valine), Leu (Leucine), Ile (Isoleucine), Phe (Phenylalanine), Trp (Tryptophan), Tyr (Tyrosine), Asp (Aspartic acid), His (Histidine), Asn (Asparagine), Glu (Glutamic acid), Lys (Lysine), Gln (Glutamine), Met (Methionine), Arg (Arginine), Ser (Serine), Thr (Threonine), Cys (Cysteine), Pro (Proline), etc. Non-natural amino acids are, for example, citrulline. As is well known to those skilled in the art, non-natural amino acids do not constitute natural proteins and are therefore not involved in the synthesis of antibodies in the present disclosure. The three-letter and single-letter codes for amino acids used in the present disclosure are as described in J. biol. chem, 243, p3558 (1968).

| For short | Abbreviation | Name | Structure |
|---|---|---|---|
| G | Gly | Glycine | 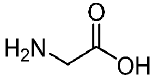 |
| A | Ala | Alanine | 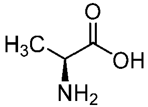 |
| V | Val | Valine | 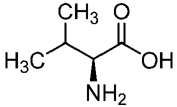 |
| L | Leu | Leucine | 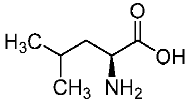 |
| I | Ile | Isoleucine | 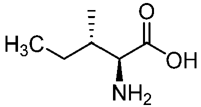 |
| F | Phe | Phenylalanine | 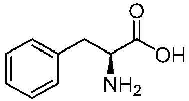 |
| W | Trp | Tryptophan | 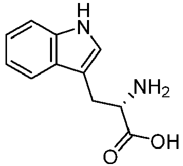 |
| Y | Tyr | Tyrosine | 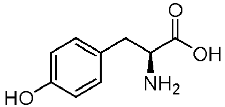 |
| D | Asp | Aspartic acid | 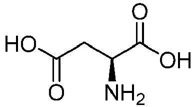 |
| H | His | Histidine | 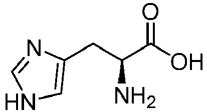 |
| N | Asn | Asparagine | 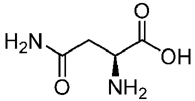 |
| E | Glu | Glutamic acid | 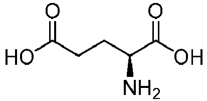 |
| K | Lys | Lysine | 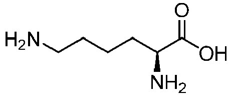 |
| Q | Gln | Glutamine | 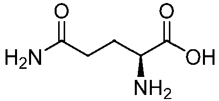 |
| C | Cit | Citrulline | 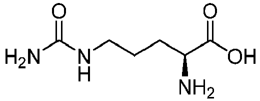 |

The term "antibody-drug conjugate" means that a ligand is linked to a biologically active drug by a stable linker unit. In the present disclosure, "antibody-drug conjugate" (ADC) means that a monoclonal antibody or an antibody fragment is linked to a biologically active glucocorticoid by a stable linker unit, wherein the antibody or the antibody fragment, through a specific group therein (such as an interchain disulfide bond), can bind to the glucocorticoid molecule comprising a linker.

The term "drug loading" refers to the average number of drugs that each antibody-drug conjugate molecule carries in a population of antibody-drug conjugates, and can also be expressed as a ratio of the number of drugs to the number of antibodies. The drug loading may range from 1 to 20, preferably from 1 to 10 glucocorticoids (D) linked to each antibody (Ab). In the embodiments of the present disclosure, the drug loading is represented by k, and may illustratively be 1, 2, 3, 4, 5, 6, 7, 8, 9, 10, 11, 12, 13, 14, 15, 16, 17, 18, 19, 20, or an average of any two values, preferably an average of 1 to 10, and more preferably an average of 1 to 8, or 2 to 8, or 2 to 7, or 3 to 8, or 3 to 7, or 3 to 6, or 4 to 7, or 4 to 6, or 4 to 5. The average number of drugs per ADC molecule after coupling reactions can be characterized by conventional methods such as UV/visible spectroscopy, mass spectrometry, ELISA assays, monoclonal antibody molecule size variant assay (CE-SDS) and HPLC.

The monoclonal antibody molecular size variant assay (CE-SDS) of the present disclosure may be used for quantitatively determining the purity of a recombinant monoclonal antibody product by adopting capillary electrophoresis-sodium dodecyl sulfate (CE-SDS) ultraviolet assay based on the molecular weight under reduced and non-reduced conditions and according to a capillary electrophoresis method (Chinese Pharmacopoeia 0542, 2015 Edition).

In one embodiment of the present disclosure, the glucocorticoid is coupled to the N-terminal amino of the ligand and/or ε-amino of the lysine residue through a linker unit, and generally, the number of drug molecules that can be coupled to the antibody in the coupling reaction will be less than the theoretical maximum.

The loading of the antibody-drug conjugate can be controlled by the following non-limiting methods, including:
(1) controlling a molar ratio of a linking reagent to a monoclonal antibody,
(2) controlling reaction time and temperature, and
(3) selecting different reaction reagents.

The term "antibody" refers to an immunoglobulin, which is of a tetrapeptide chain structure formed by connection between two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, otherwise called isotypes of immunoglobulins, namely IgM, IgD, IgG, IgA and IgE, with their corresponding heavy chains being µ chain, δ chain, γ chain, α chain and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3 and IgG4. Light chains are divided into κ or λ chains according to differences in the constant regions. Each of the five classes of Ig may have a κ chain or λ chain.

In the heavy and light chains of the antibody, the sequences of about 110 amino acids near the N-terminus vary considerably and thus are referred to as variable regions (Fv regions); the remaining amino acid sequences near the C-terminus are relatively stable and thus are referred to as constant regions. The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (LCVR) or heavy chain variable region (HCVR) consists of 3 CDRs and 4 FRs arranged from the amino-terminus to the carboxy-terminus in the following order: FR1, CDR1, FR2, CDR2, FR3, CDR3 and FR4. The 3 CDRs of the light chain refer to LCDR1, LCDR2 and LCDR3, and the 3 CDRs of the heavy chain refer to HCDR1, HCDR2 and HCDR3.

The antibody of the present disclosure includes a murine antibody, a chimeric antibody, a humanized antibody and a fully human-derived antibody, preferably a humanized antibody and a fully human-derived antibody.

The term "murine antibody" used herein refers to an antibody prepared from mice according to the knowledge and skill in the art. During the preparation, a test subject is injected with a specific antigen, and then hybridomas expressing antibodies with desired sequences or functional properties are isolated.

The term "chimeric antibody" refers to an antibody obtained by fusing a variable region of a murine antibody to a constant region of a human antibody, which can reduce an immune response induced by the murine antibody. The chimeric antibody is established by firstly establishing hybridoma secreting murine specific monoclonal antibody, then cloning a variable region gene from the mouse hybridoma cells, cloning a constant region gene of human antibody as required, connecting the mouse variable region gene and the human constant region gene into a chimeric gene, inserting the chimeric gene into an expression vector, and finally expressing chimeric antibody molecules in a eukaryotic system or prokaryotic system.

The term "humanized antibody", also known as a CDR-grafted antibody, refers to an antibody produced by grafting murine CDR sequences into a human antibody variable region framework, i.e., a different type of human germline antibody framework sequence. Such an antibody can overcome the heterogeneous reaction induced by the chimeric antibody because of carrying a large amount of mouse protein components. Such framework sequences can be obtained from public DNA databases or published references that include germline antibody gene sequences. For example, germline DNA sequences of genes of the human heavy and light chain variable regions can be found in the "VBase" human species sequence database, as well as in Kabat, E. A. et al., 1991 Sequences of Proteins of Immunological Interest, 5th edition. To avoid the decrease in activity caused by the decrease in immunogenicity, the FR sequence in the human antibody variable region can be subjected to minimum reverse mutation or back mutation to maintain activity. The humanized antibody of the present disclosure also includes the humanized antibody formed after further affinity maturation on the CDRs by phage display. Literature further describing methods used in humanization of accessible mouse antibodies includes, for example, Queen et al., Proc., Natl. Acad. Sci. USA, 88, 2869, 1991, and literature describing humanization using the method provided by Winter and coworkers thereof includes Jones et al., Nature, 321, 522 (1986); Riechmann et al., Nature, 332, 323-327 (1988), Verhoeyen et al, Science, 239, 1534 (1988).

The term "fully human-derived antibody", "fully human antibody" or "completely human-derived antibody", also known as "fully human-derived monoclonal antibody", may have both humanized variable regions and constant regions, thus eliminating immunogenicity and toxic and side effects. The development of monoclonal antibodies has four stages, namely murine monoclonal antibodies, chimeric monoclonal antibodies, humanized monoclonal antibodies and fully human-derived monoclonal antibodies. The antibody of the present disclosure is the fully human-derived monoclonal antibody. Major relevant technologies for the preparation of the fully human antibody include human hybridoma technology, EBV-transformed B-lymphocyte technology, phage display technology, transgenic mouse antibody preparation technology, single B-cell antibody preparation technology, and the like.

The term "antigen-binding fragment" refers to one or more fragments of an antibody that retain the ability to specifically bind to an antigen. It is shown that a fragment of a full-length antibody can be used to perform the antigen-binding function of the antibody. The examples of the binding fragment included in the "antigen-binding fragment" include (i) Fab fragments, monovalent fragments consisting of VL, VH, CL and CH1 domains; (ii) F(ab')₂ fragments, bivalent fragments comprising two Fab fragments connected by disulfide bridges in the hinge regions; (iii) Fd fragments consisting of VH and CH1 domains; (iv) Fv fragments consisting of VH and VL domains of a single arm of an antibody; (v) single domains or dAb fragments (Ward et al., (1989) Nature 341: 544-546) consisting of VH domains; and (vi) isolated complementarity determining regions (CDRs) or (vii) combinations of two or more isolated CDRs which may optionally be linked by synthetic linkers. In addition, while the two domains of the Fv fragment, VL and VH, are encoded by separate genes, they can be recombinantly joined by a synthetic linker, so that they can generate a single protein chain in which the VL and VH regions are paired to form a monovalent molecule (referred to as single-chain Fv (scFv); see, e.g., Bird et al., (1988) Science, 242:423-426; and Huston et al., (1988) Proc. Natl. Acad. Sci USA 85:5879-5883).

Such single-chain antibodies are also intended to be included in the term "antigen-binding fragment" of an antibody. Such antibody fragments are obtained by conventional techniques known to those skilled in the art, and screened for utility in the same manner as for intact antibodies. Antigen-binding moieties may be produced by a recombinant DNA technique or by enzyme catalysis or chemical cleavage of intact immunoglobulins. The antibody may be of different isotypes, e.g., an IgG (e.g., subtype IgG1, IgG2, IgG3 or IgG4), IgA1, IgA2, IgD, IgE or IgM antibody.

Fab is an antibody fragment having a molecular weight of about 50,000 and having antigen-binding activity, among fragments obtained by treating an IgG antibody molecule with a protease papain (cleaving the amino acid residue at position 224 of H chain), in which about half and whole N-terminal side of H chain is combined with L chain by a disulfide bond.

F(ab')2 is an antibody fragment having a molecular weight of about 100,000 and having antigen-binding activity and comprising two Fab regions linked at the hinge position, which is obtained by digesting a portion below two disulfide bonds in the IgG hinge region with the enzyme pepsin.

Fab' is an antibody fragment having a molecular weight of about 50,000 and having an antigen-binding activity, which is obtained by cleaving the disulfide bond in the hinge region of the F(ab')2 described above.

In addition, the Fab' can be produced by inserting DNA encoding the Fab' fragment of the antibody into a prokaryotic expression vector or a eukaryotic expression vector and introducing the vector into a prokaryote or a eukaryote to express the Fab'.

The term "single-chain antibody", "single-chain Fv" or "scFv" refers to a molecule comprising an antibody heavy chain variable domain (or region; VH) and an antibody light chain variable domain (or region; VL) linked by a linker. Such scFv molecules may have a general structure: NH₂-VL-linker-VH-COOH or NH₂-VH-linker-VL-COOH. Suitable linkers in the prior art consist of repeated GGGGS amino acid sequences or variants thereof, for example, 1-4 repeated variants (Holliger et al. (1993), Proc. Natl. Acad. Sci. USA 90:6444-6448). Other linkers that can be used in the present disclosure are described in Alfthan et al. (1995), Protein Eng. 8:725-731; Choi et al. (2001), Eur. J. Immunol. 31:94-106; Hu et al. (1996), Cancer Res. 56:3055-3061; Kipriyanov et al. (1999), J. Mol. Biol. 293:41-56; and Roovers et al. (2001), Cancer Immunol.

The term "CDR" refers to one of the 6 hypervariable regions within the variable domain of an antibody which primarily contribute to antigen binding. One of the most common definitions for the 6 CDRs is provided in Kabat E.A. et al., (1991) Sequences of proteins of immunological interest. NIH Publication 91-3242. As used herein, the Kabat definition of CDRs may be only applied to CDR1, CDR2 and CDR3 of the light chain variable domain (CDR L1, CDR L2, CDR L3 or L1, L2, L3), and CDR2 and CDR3 of the heavy chain variable domain (CDR H2, CDR H3 or H2, H3). Generally, there are three CDRs (HCDR1, HCDR2 and HCDR3) in each heavy chain variable region and three CDRs (LCDR1, LCDR2 and LCDR3) in each light chain variable region. The amino acid sequence boundaries of the CDRs can be determined using any of a variety of well-known schemes, including "Kabat" numbering scheme (see Kabat et al. (1991), "Sequences of Proteins of Immunological Interest", 5th edition, Public Health Service, National Institutes of Health, Bethesda, MD), "Chothia" numbering scheme (see Al-Lazikani et al. (1997) JMB 273: 927-948) and ImMunoGen Tics (IMGT) numbering scheme (see Lefranc M.P., Immunologist, 7, 132-136(1999); Lefranc, M.P. et al., Dev. Comp. Immunol., 27, 55-77(2003)), and the like. For example, for the classical format, according to the Kabat scheme, the CDR amino acid residues in the heavy chain variable domain (VH) are numbered 31-35 (HCDR1), 50-65 (HCDR2) and 95-102 (HCDR3); the CDR amino acid residues in the light chain variable domain (VL) are numbered 24-34 (LCDR1), 50-56 (LCDR2) and 89-97 (LCDR3). According to the Chothia scheme, the CDR amino acids in VH are numbered 26-32 (HCDR1), 52-56 (HCDR2) and 95-102 (HCDR3); and the amino acid residues in VL are numbered 26-32 (LCDR1), 50-52 (LCDR2) and 91-96 (LCDR3). According to the combination of CDR definitions provided in the Kabat scheme and the Chothia scheme, the CDR is composed of amino acid residues 26-35 (HCDR1), 50-65 (HCDR2) and 95-102 (HCDR3) in the human VH and amino acid residues 24-34 (LCDR1), 50-56 (LCDR2) and 89-97 (LCDR3) in the human VL. According to the IMGT scheme, the CDR amino acid residues in VH are roughly numbered 26-35 (CDR1), 51-57 (CDR2) and 93-102 (CDR3), and the CDR amino acid residues in VL are roughly numbered 27-32 (CDR1), 50-52 (CDR2) and 89-97 (CDR3). According to the IMGT scheme, the CDRs of the antibody can be determined using the program IMGT/DomainGap Align.

The term "antibody framework" refers to a portion of a variable domain VL or VH, which serves as a framework for the antigen-binding loops (CDRs) of the variable domain. It is essentially a variable domain without CDRs.

The term "epitope" or "antigenic determinant" refers to a site on an antigen to which an immunoglobulin or antibody specifically binds. Epitopes generally comprise at least 3, 4, 5, 6, 7, 8, 9, 10, 11, 12, 13, 14 or 15 contiguous or non-contiguous amino acids in a unique spatial conformation (see, e.g., Epitope Mapping Protocols in Methods in Molecular Biology, Vol. 66, G.E.Morris, Ed. (1996)).

The terms "specific binding", "selective binding", "selectively bind to" and "specifically bind to" refer to the binding of an antibody to an epitope on a predetermined antigen. Generally, the antibody binds with an affinity (KD) of less than about 10⁻⁷ M, e.g., less than about 10⁻⁸ M, 10⁻⁹ M, or 10⁻¹⁰ M or less.

The term "nucleic acid molecule" refers to a DNA molecule and an RNA molecule. The nucleic acid molecule may be single-stranded or double-stranded, and is preferably double-stranded DNA. A nucleic acid is "operably linked" when it is placed into a functional relationship with another nucleic acid sequence. For example, a promoter or enhancer is operably linked to a coding sequence if it affects the transcription of the coding sequence.

The term "vector" refers to a nucleic acid molecule capable of transporting another nucleic acid to which it has been linked. In one embodiment, the vector is a "plasmid" that refers to a circular double-stranded DNA loop into which additional DNA segments may be ligated. In another embodiment, the vector is a viral vector, wherein additional DNA segments may be ligated into the viral genome. The vectors disclosed herein are capable of autonomously replicating in a host cell into which they are introduced (e.g., bacterial vectors having a bacterial origin of replication and episomal mammalian vectors) or capable of integrating into the genome of a host cell after being introduced into the host cell and thus replicating with the host genome (e.g., non-episomal mammalian vectors).

Methods for producing and purifying antibodies and antigen-binding fragments are well known in the prior art, for example, those described in chapters 5-8 and 15 of "Antibodies: A Laboratory Manual", Cold Spring Harbor Press. Likewise, antigen-binding fragments can be prepared by conventional methods. The antibody or antigen-binding fragment described in the present invention is genetically engineered to contain one or more additional human-derived FRs in the non-human-derived CDRs. Human FR germline sequences can be obtained by aligning the IMGT human antibody variable region germline gene database with MOE software, or obtained from Immunoglobulin Journal, 2001ISBN012441351.

The term "host cell" refers to a cell into which an expression vector has been introduced. Host cells may include bacterial, microbial, plant or animal cells. Bacteria susceptible to transformation include members of the *Enterobacteriaceae* family, such as strains of *Escherichia coli* or *Salmonella;* members of the *Bacillaceae* family, such as *Bacillus subtilis; Pneumococcus; Streptococcus* and *Haemophilus influenzae.* Suitable microorganisms include *Saccharomyces cerevisiae* and *Pichia pastoris.* Suitable animal host cell lines include CHO (Chinese hamster ovary cell line) and NS0 cells.

The engineered antibody or the antigen-binding fragment of the present disclosure can be prepared and purified by conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems will result in glycosylation of antibodies, particularly at the highly conserved N-terminal site of the Fc region. Positive clones are expanded in a serum-free medium of a bioreactor to produce antibodies. The culture medium with the secreted antibody can be purified by conventional techniques. For example, purification is performed using an A or G Sepharose FF column containing an adjusted buffer. Non-specifically bound fractions are washed away. The bound antibody is eluted by the pH gradient method, and the antibody fragments are detected by SDS-PAGE and collected. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

The amino acid sequence "identity" refers to the percentage of amino acid residues shared by a first sequence and a second sequence, wherein in aligning the amino acid sequences and when necessary, gaps are introduced to achieve maximum percent sequence identity, and any conservative substitution is not considered as part of the sequence identity. For the purpose of determining percent amino acid sequence identity, alignments can be achieved in a variety of ways that are within the skills in the art, for example, using publicly available computer software such as BLAST, BLAST-2, ALIGN, ALIGN-2 or Megalign (DNASTAR) software. Those skilled in the art can determine parameters suitable for measuring alignment, including any algorithms required to achieve maximum alignment of the full length of the aligned sequences.

The term "anti-TNFα antibody" or "antibody that binds to TNFα" refers to an antibody that is capable of binding to TNFα, e.g., with sufficient affinity such that the antibody can be used as a therapeutic agent targeting TNFα. The extent of binding of an anti-TNFα antibody to an unrelated non-TNFα protein can be less than about 10% of the binding of the antibody to TNFα as measured, e.g., by a radioimmunoassay (RIA). In certain embodiments, an antibody that binds to TNFα has a dissociation constant (Kd) of ≤1 µM, ≤100 nM, ≤10 nM, ≤1 nM, or ≤0.1 nM.

The term "peptide" refers to a compound fragment between an amino acid and a protein. It is formed by connecting 2 or more amino acid molecules by peptide bonds, and is a structural and functional fragment of the protein, such as hormones and enzymes, which are essentially peptides.

The term "sugar" refers to biomacromolecules consisting of C, H and O elements. They can be classified into monosaccharides, disaccharides, polysaccharides and the like.

The term "fluorescent probe" refers to a class of fluorescent molecules that have characteristic fluorescence in the ultraviolet-visible-near infrared region and whose fluorescent properties (excitation and emission wavelengths, intensity, lifetime, polarization, etc.) can sensitively change as the properties of the environment they are in, such as polarity, refractive index and viscosity, change. These fluorescent molecules non-covalently interact with nucleic acids (DNA or RNA), proteins or other macromolecular structures to change one or more fluorescent properties, and therefore can be used to study the properties and behavior of macromolecular substances.

The term "alkyl" refers to a saturated aliphatic hydrocarbon group which is a linear or branched group containing 1 to 20 carbon atoms, preferably an alkyl group containing 1 to 12 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, and the like. More preferred is an alkyl group containing 1 to 6 carbon atoms; non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl may be substituted or unsubstituted, and when it is substituted, the substituent may be substituted at any available point of attachment, and the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl and a carboxylate group.

The term "alkylene" refers to a saturated linear or branched aliphatic hydrocarbon group having 2 residues derived from the parent alkane by removal of two hydrogen atoms from the same carbon atom or two different carbon atoms. It is a linear or branched group containing 1 to 20 carbon atoms, preferably alkylene containing 1 to 12 carbon atoms, and more preferably alkylene containing 1 to 6 carbon atoms. Non-limiting examples of alkylene include, but are not limited to, methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), and the like. The alkylene may be substituted or unsubstituted, and when it is substituted, the substituent may be substituted at any available point of attachment.

The term "alkenylene" refers to a linear alkenyl group having 2 to 8 carbon atoms, preferably 2 to 6 carbon atoms, and more preferably 2 to 4 carbon atoms, and having at least one double bond at any position, including, for example, ethenylene, allylene, propenylene, butenylene, prenylene, butadienylene, pentenylene, pentadienylene, hexenylene, hexadienylene, and the like.

The term "alkynylene" refers to a linear alkynylene group having 2 to 8 carbon atoms, preferably 2 to 6 carbon atoms, and more preferably 2 to 4 carbon atoms, and having at least one triple bond at any position, including, for example, ethynylene, propynylene, butynelene, pentynylene, hexynylene, and the like.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl. "Carbocycle" refers to a ring system in cycloalkyl.

The term "spiro cycloalkyl" refers to a 5- to 20-membered polycyclic group in which monocyclic rings share one carbon atom (referred to as the spiro atom). It may contain one or more double bonds, but none of the rings has a fully conjugated π-electron system. It is preferably 6- to 14-membered, and more preferably 7- to 10-membered. According to the number of the spiro atoms shared among the rings, the spiro cycloalkyl may be monospiro cycloalkyl, bispiro cycloalkyl or polyspiro cycloalkyl, preferably monospiro cycloalkyl and bispiro cycloalkyl, and more preferably 4-membered/4-membered, 4-membered/5-membered, 4-membered/6-membered, 5-membered/5-membered or 5-membered/6-membered monospiro cycloalkyl. "Spiro carbocycle" refers to a ring system in spiro cycloalkyl. Non-limiting examples of spiro cycloalkyl include:

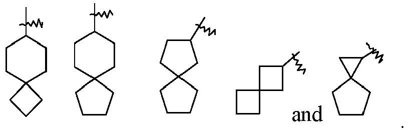

The term "fused cycloalkyl" refers to a 5- to 20-membered all-carbon polycyclic group in which each ring shares a pair of adjacent carbon atoms with other rings in the system, wherein one or more of the rings may contain one or more double bonds, but none of them has a fully conjugated π-electron system. It is preferably 6- to 14-membered, and more preferably 7- to 10-membered. According to the number of constituent rings, the fused cycloalkyl may be bicyclic, tricyclic, tetracyclic or polycyclic fused cycloalkyl, preferably bicyclic or tricyclic fused cycloalkyl, and more preferably 5-membered/5-membered or 5-membered/6-membered bicycloalkyl. "Fused carbocycle" refers to a ring system in fused cycloalkyl. Non-limiting examples of fused cycloalkyl include:

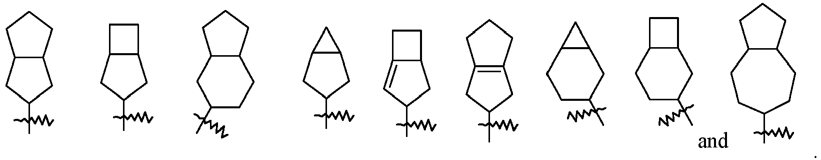

The term "bridged cycloalkyl" refers to a 5- to 20-membered all-carbon polycyclic group in which any two rings share two carbon atoms that are not directly connected to each other. It may contain one or more double bonds, but none of the rings has a fully conjugated π-electron system. It is preferably 6- to 14-membered, and more preferably 7- to 10-membered. According to the number of constituent rings, the bridged cycloalkyl may be bicyclic, tricyclic, tetracyclic or polycyclic bridged cycloalkyl, preferably bicyclic, tricyclic or tetracyclic bridged cycloalkyl, and more preferably bicyclic or tricyclic bridged cycloalkyl. Non-limiting examples of bridged cycloalkyl include:

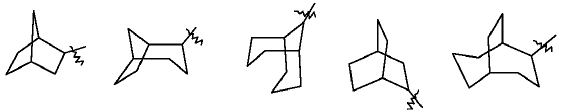

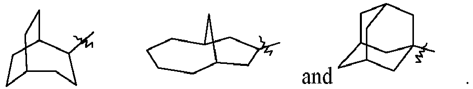

The cycloalkyl ring may be fused to an aryl, heteroaryl or heterocycloalkyl ring, wherein the ring attached to the parent structure is cycloalkyl; non-limiting examples include indanyl, tetrahydronaphthyl, benzocycloheptyl, and the like. The cycloalkyl may be optionally substituted or unsubstituted, and when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl and a carboxylate group.

The term "heterocyclyl" refers to a saturated or partially unsaturated monocyclic or polycyclic hydrocarbon substituent containing 3 to 20 ring atoms, one or more of which are heteroatoms selected from the group consisting of nitrogen, oxygen and S(O)ₘ (where m is an integer of 0 to 2), excluding a ring moiety of -O-O-, -O-S- or -S-S-, and the remaining ring atoms are carbon atoms. It preferably contains 3 to 12 ring atoms, of which 1 to 4 are heteroatoms; more preferably, it contains 3 to 6 ring atoms. Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, and the like, preferably piperidinyl and pyrrolidinyl. Polycyclic heterocyclyl includes spiro heterocyclyl, fused heterocyclyl, and bridged heterocyclyl. "Heterocycle" refers to a ring system in heterocyclyl.

The term "spiro heterocyclyl" refers to a 5- to 20-membered polycyclic heterocyclyl group in which monocyclic rings share one atom (referred to as the spiro atom), wherein one or more ring atoms are heteroatoms selected from the group consisting of nitrogen, oxygen and S(O)ₘ (where m is an integer of 0 to 2), and the remaining ring atoms are carbon atoms. It may contain one or more double bonds, but none of the rings has a fully conjugated π-electron system. It is preferably 6- to 14-membered, and more preferably 7- to 10-membered. According to the number of spiro atoms shared among the rings, the spiro heterocyclyl may be monospiro heterocyclyl, bispiro heterocyclyl or polyspiro heterocyclyl, preferably monospiro heterocyclyl and bispiro heterocyclyl, and more preferably 4-membered/4-membered, 4-membered/5-membered, 4-membered/6-membered, 5-membered/5-membered or 5-membered/6-membered monospiro heterocyclyl. "Spiro heterocycle" refers to a ring system in spiro heterocyclyl. Non-limiting examples of spiro heterocyclyl include:

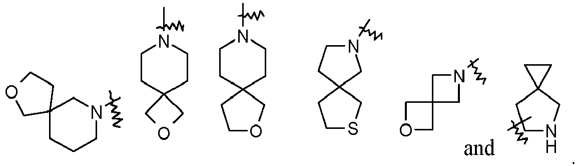

The term "fused heterocyclyl" refers to a 5- to 20-membered polycyclic heterocyclyl group in which each ring shares a pair of adjacent atoms with the other rings in the system, wherein one or more of the rings may contain one or more double bonds, but none of them has a fully conjugated π-electron system, and one or more of the ring atoms are heteroatoms selected from the group consisting of nitrogen, oxygen or S(O)ₘ (where m is an integer of 0 to 2), and the remaining ring atoms are carbon atoms. It is preferably 6- to 14-membered, and more preferably 7- to 10-membered. According to the number of constituent rings, the fused heterocyclyl may be bicyclic, tricyclic, tetracyclic or polycyclic fused heterocyclyl, preferably bicyclic or tricyclic fused heterocyclyl, and more preferably 5-membered/5-membered or 5-membered/6-membered bicyclic fused heterocyclyl. "Fused heterocycle" refers to a ring system in fused heterocyclyl. Non-limiting examples of fused heterocyclyl include:

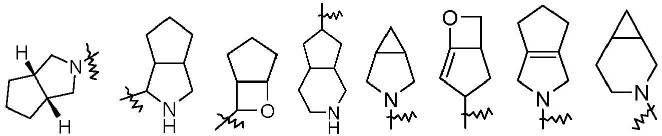

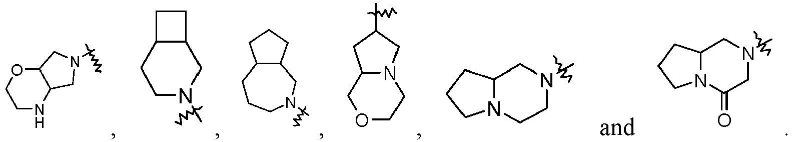

The term "bridged heterocyclyl" refers to a 5- to 14-membered polycyclic heterocyclyl group in which any two rings share two atoms that are not directly connected to each other. It may contain one or more double bonds, but none of the rings has a fully conjugated π-electron system, wherein one or more of the ring atoms are heteroatoms selected from the group consisting of nitrogen, oxygen and S(O)ₘ (where m is an integer of 0 to 2), and the remaining ring atoms are carbon atoms. It is preferably 6- to 14-membered, and more preferably 7- to 10-membered. According to the number of constituent rings, the bridged heterocyclyl may be bicyclic, tricyclic, tetracyclic or polycyclic bridged heterocyclyl, preferably bicyclic, tricyclic or tetracyclic bridged heterocyclyl, and more preferably bicyclic or tricyclic bridged heterocyclyl. Non-limiting examples of bridged heterocyclyl include:

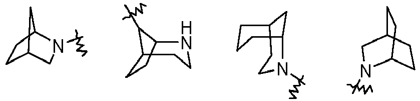

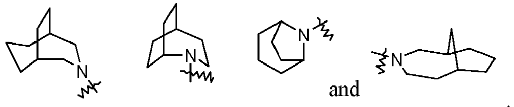

The heterocyclyl ring may be fused to an aryl, heteroaryl or cycloalkyl ring, wherein the ring attached to the parent structure is heterocyclyl; its non-limiting examples include: 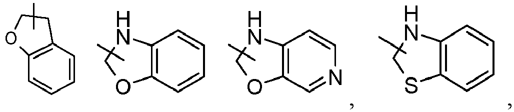 etc.

The heterocyclyl may be optionally substituted or unsubstituted, and when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl and a carboxylate group.

The term "aryl" refers to a 6- to 14-membered, preferably 6- to 10-membered all-carbon monocyclic or fused polycyclic (i.e., rings that share a pair of adjacent carbon atoms) group having a conjugated π-electron system, such as phenyl and naphthyl. The aryl ring may be fused to a heteroaryl, heterocyclyl or cycloalkyl ring, wherein the ring attached to the parent structure is the aryl ring. "Aryl ring" refers to a ring system in aryl. Non-limiting examples of aryl include: 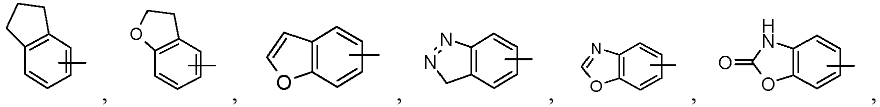 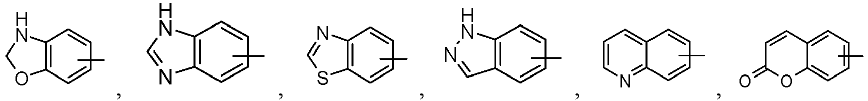 and

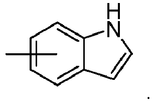

The aryl may be substituted or unsubstituted, and when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl and a carboxylate group, preferably phenyl.

The term "fused cycloaryl" may be an unsaturated aromatic fused ring structure containing 8-14 ring atoms, preferably 8-12 ring atoms, formed by connecting two or more ring structures that share two adjacent atoms with each other, for example, including all unsaturated fused cycloaryl groups such as naphthalene and phenanthrene, and partially saturated fused cycloaryl groups such as benzo 3-8 membered saturated monocyclic cycloalkyl and benzo 3-8 membered partially saturated monocyclic cycloalkyl. "Fused aryl ring" refers to a ring system in fused cycloaryl. Specific examples of fused cycloaryl include 2,3-dihydro-1H-indenyl, IH-indenyl, 1,2,3,4-tetrahydronaphthyl, 1,4-dihydronaphthyl, and the like.

The term "heteroaryl" refers to a heteroaromatic system containing 1 to 4 heteroatoms and 5 to 14 ring atoms, wherein the heteroatoms are selected from the group consisting of oxygen, sulfur and nitrogen. The heteroaryl is preferably 5- to 12-membered, e.g., imidazolyl, furyl, thienyl, thiazolyl, pyrazolyl, oxazolyl, pyrrolyl, tetrazolyl, pyridyl, pyrimidinyl, thiadiazole, pyrazinyl, or the like, preferably imidazolyl, pyrazolyl, pyrimidinyl or thiazolyl; and more preferably pyrazolyl or thiazolyl. The heteroaryl ring may be fused to an aryl, heterocyclyl or cycloalkyl ring, wherein the ring attached to the parent structure is the heteroaryl ring. "Heteroaryl ring" refers to a ring system in heteroaryl. Non-limiting examples of heteroaryl include:

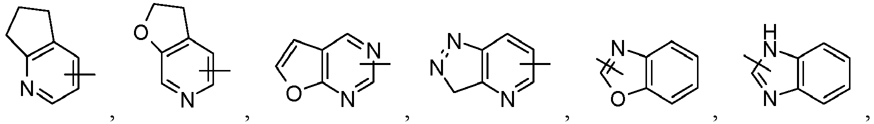

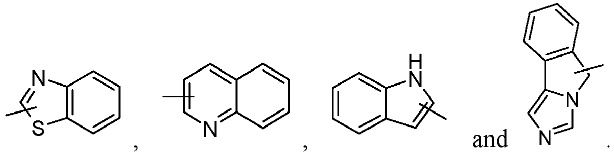

The heteroaryl may be optionally substituted or unsubstituted, and when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl and a carboxylate group.

The term "fused heteroaryl" may be an unsaturated aromatic fused ring structure containing 5-14 ring atoms (at least one heteroatom) formed by connecting two or more ring structures that share two adjacent atoms with each other, including the case where a carbon atom, a nitrogen atom and a sulfur atom may be oxidized, preferably "5-12 membered fused heteroaryl", "7-12 membered fused heteroaryl", "9-12 membered fused heteroaryl", and the like, for example, benzofuranyl, benzoisothiafuranyl, benzothienyl, indolyl, isoindolyl, benzoxazolyl, benzimidazolyl, indazolyl, benzotriazolyl, quinolyl, 2-quinolinone, 4-quinolinone, 1-isoquinolinone, isoquinolinyl, acridinyl, phenanthridinyl, benzopyridazinyl, phthalazinyl, quinazolinyl, quinoxalinyl, phenazinyl, pteridinyl, purinyl, naphthyridinyl, phenazine, phenothiazine, and the like. "Fused heteroaryl ring" refers to a ring system in fused heteroaryl.

The fused heteroaryl may be optionally substituted or unsubstituted, and when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl and a carboxylate group.

The term "alkoxy" refers to -O-(alkyl) and -O-(unsubstituted cycloalkyl), wherein the alkyl is as defined above. Non-limiting examples of alkoxy include: methoxy, ethoxy, propoxy, butoxy, cyclopropyloxy, cyclobutoxy, cyclopentyloxy and cyclohexyloxy. The alkoxy may be optionally substituted or unsubstituted, and when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl and a carboxylate group.

The term "alkylthio" refers to -S-(alkyl) and -S-(unsubstituted cycloalkyl), wherein the alkyl is as defined above. Non-limiting examples of alkylthio include: methylthio, ethylthio, propylthio, butylthio, cyclopropylthio, cyclobutylthio, cyclopentylthio and cyclohexylthio. The alkylthio may be optionally substituted or unsubstituted, and when it is substituted, the substituent is preferably one or more of the following groups; it is substituted with one or more substituents independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio and heterocycloalkylthio.

The term "hydroxyalkyl" refers to an alkyl group substituted with hydroxy, wherein the alkyl group is as defined above.

The term "haloalkyl" refers to an alkyl group substituted with halogen, wherein the alkyl group is as defined above.

The term "deuterated alkyl" refers to an alkyl group substituted with a deuterium atom, wherein the alkyl group is as defined above.

The term "hydroxy" refers to the -OH group.

The term "oxo" refers to the =O group. For example, a carbon atom is connected to an oxygen atom via a double bond to form a ketone or aldehyde group.

The term "thio" refers to the =S group. For example, the carbon atom is connected to a sulfur atom via a double bond to form thiocarbonyl-C(S)-.

The term "halogen" refers to fluorine, chlorine, bromine, or iodine.

The term "amino" refers to -NH₂.

The term "cyano" refers to -CN.

The term "nitro" refers to -NO₂.

The term "carboxyl" refers to -C(O)OH.

The term "aldehyde" refers to -CHO.

The term "carboxylate group" refers to -C(O)O(alkyl) or -C(O)O(cycloalkyl), wherein the alkyl and cycloalkyl are as defined above.

The term "acyl halide" refers to a compound containing a -C(O)-halogen group.

The term "sulfonyl" refers to -S(O)(O)-.

The term "sulfinyl" refers to -S(O)-.

"Amino protecting group" is a suitable group known in the art for amino protection; see the literature ("Protective Groups in Organic Synthesis", 5Th. Ed. T. W. Greene & P. G. M. Wuts) for the amino protecting groups. Preferably, the amino protecting group may be (C₁₋₁₀ alkyl or aryl)acyl, e.g., formyl, acetyl, benzoyl, or the like; (C₁₋₆ alkyl or C₆₋₁₀ aryl)sulfonyl; (C₁₋₆ alkoxy or C₆₋₁₀ aryloxy)carbonyl, e.g., Boc or Cbz; or substituted or unsubstituted alkyl, e.g., trityl (Tr), 2,4-dimethoxybenzyl (DMB), *p*-methoxybenzyl (PMB) or benzyl (Bn).

"Optional" or "optionally" means that the event or circumstance subsequently described may, but does not necessarily, occur, and that the description includes instances where the event or circumstance occurs or does not occur. For example, "a heterocycloalkyl group optionally substituted with alkyl" means that alkyl may, but does not necessarily, exist, and that the description includes instances where the heterocycloalkyl group is or is not substituted with alkyl.

The term "pharmaceutical composition" refers to a mixture containing one or more of the compounds or the physiologically/pharmaceutically acceptable salts or pro-drugs thereof described herein, and other chemical components, for example, physiologically/pharmaceutically acceptable carriers and excipients. The pharmaceutical composition is intended to promote the administration to an organism, so as to facilitate the absorption of the active ingredient, thereby exerting biological activity.

The term "drug carrier" for the drug of the present disclosure refers to a system that can alter the manner in which the drug gets into a human body and the distribution of the drug in the human body, control the release rate of the drug, and deliver the drug to a targeted organ. The drug carrier release and targeted system can reduce drug degradation and loss, reduce side effects and improve bioavailability. For example, polymeric surfactants that can be used as carriers can self-assemble due to their unique amphiphilic structures to form various forms of aggregates, such as micelles, microemulsions, gels, liquid crystals and vesicles, as preferred examples. The aggregates have the capability of encapsulating drug molecules and have good permeability for membranes, and therefore can be used as excellent drug carriers.

The term "excipient" is an addition, besides the main drug, to a pharmaceutical formulation. It may also be referred to as an auxiliary material. For example, binders, fillers, disintegrants and lubricants in tablets; the matrix part in semisolid ointment and cream preparations; preservatives, antioxidants, corrigents, fragrances, cosolvents, emulsifiers, solubilizers, osmotic pressure regulators, colorants and the like in liquid formulations can all be referred to as excipients.

The term "diluent", also referred to as a filler, is used primarily to increase the weight and volume of the tablet. The addition of the diluent not only ensures a certain volume, but also reduces the dose deviation of the main ingredients, and improves the drug's compression moldability and the like. When the drug in the tablet form contains oily components, an absorbent is necessarily added to absorb the oily components so as to maintain a "dry" state and thus facilitate the preparation of the tablet.

The compound of the present disclosure may contain one or more asymmetric centers and thus enantiomers and diastereomers may be generated. The enantiomers and diastereomers may be defined in terms of absolute stereochemistry as (R)- or (*S*)-, or other stereoisomeric forms of (*D*)- or (*L*)- for amino acids. The present disclosure includes all possible isomers as well as racemic and optically pure forms thereof. Optically active (+) and (-), (*R*)- and (*S*)-, or (*D*)- and (*L*)-isomers may be prepared by using chiral synthons or chiral reagents, or may be prepared by using conventional methods such as chromatography and fractional crystallization. Conventional methods for the preparation/separation of enantiomers include chiral synthesis from suitable optically pure precursors or resolution of the racemate (or the racemate of a salt or derivative) by using, for example, chiral high performance liquid chromatography (HPLC). When a compound described herein contains an olefinic double bond or other geometric asymmetric centers, it is meant that the compound includes both *E* and *Z* geometric isomers, unless otherwise specified. Moreover, all tautomeric forms are also intended to be included.

In the chemical structure of the compound of the present disclosure, a bond 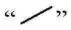 represents an unspecified configuration-that is, if chiral isomers exist in the chemical structure, the bond 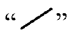 may be 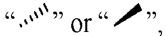 or contains both the configurations of 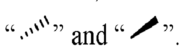 In the chemical structure of the compound of the present disclosure, a bond 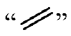 is not specified with a configuration, that is, it may be in a Z configuration or an E configuration, or contains both configurations.

"Stereoisomer" refers to compounds composed of identical atoms bonded by the same bond but with different three-dimensional structures, which are not interchangeable. The present disclosure contemplates various stereoisomers and mixtures thereof, including "enantiomers" that refer to a pair of stereoisomers that are non-superimposable mirror images of one another.

"Tautomer" refers to the transfer of a proton from one atom of a molecule to another atom of the same molecule. Tautomers of any of the compounds are included in the present disclosure.

Any isotopically-labeled derivative of the compound or the pharmaceutically acceptable salt or the isomer thereof of the present disclosure is encompassed by the present disclosure. Atoms that can be isotopically labeled include, but are not limited to, hydrogen, carbon, nitrogen, oxygen, phosphorus, fluorine, chlorine, iodine, and the like. They may be separately replaced by the isotopes ²H (D), ³H, ¹¹C, ¹³C, ¹⁴C, ¹⁵N, ¹⁸F, ³¹P, ³²P, ³⁵S, ³⁶Cl and ¹²⁵I, etc. Unless otherwise stated, when a position is specifically designated as deuterium (D), that position shall be understood to be deuterium having an abundance that is at least 3000 times greater than the natural abundance of deuterium (which is 0.015%) (i.e., incorporating at least 45% deuterium).

### BRIEF DESCRIPTION OF THE DRAWINGS

FIG. 1: the anti-inflammatory activity of small-molecule steroids in the lipopolysaccharide's stimulation of human PBMCs' cytokine secretion.
FIG. 2: the activity of anti-TNF-ADCs in a membrane-bound TNFα-mediated GRE reporter gene system.
FIG. 3: the activity of anti-TNF-ADCs in lipopolysaccharide's stimulation of human monocytes' cytokine secretion.
FIG. 4: the activity of anti-TNF-ADCs (4 nM) in LPS-induced human monocytes' cytokine release.
FIG. 5: the experimental process of Test Example 5.
FIG. 6: the activity of anti-TNF-ADCs in a mouse collagen antibody-induced arthritis (CAIA) model.
FIG. 7: the activity of anti-TNF-ADCs in a mouse collagen antibody-induced arthritis (CAIA) model at different time points.
FIG. 8: the experimental process of Test Example 6.
FIG. 9: the activity of anti-TNF-ADCs in a delayed-type hypersensitivity (DTH) model.

### DETAILED DESCRIPTION

The preparation of the compound and the pharmaceutically acceptable salt thereof of the present disclosure is further described below in conjunction with examples, which are not intended to limit the scope of the present disclosure.

Experimental methods without conditions specified in the examples of the present disclosure generally followed conventional conditions, or conditions recommended by the manufacturers of the starting materials or commercial products. Reagents without specific origins indicated were commercially available conventional reagents.

NMR shifts (δ) were given in 10⁻⁶ (ppm). NMR analysis was performed on a Bruker AVANCE-400 nuclear magnetic resonance instrument, with deuterated dimethyl sulfoxide (DMSO-*d*⁶), deuterated chloroform (CDCl₃) and deuterated methanol (CD₃OD) as solvents and tetramethylsilane (TMS) as an internal standard.

MS analysis was performed on a Shimadzu 2010 Mass Spectrometer or Agilent 6110A MSD Mass Spectrometer.

HPLC analysis was performed on Shimadzu LC-20A systems, Shimadzu LC-2010HT series, or an Agilent 1200 LC high-performance liquid chromatograph (Ultimate XB-C18 3.0 × 150 mm chromatography column or Xtimate C18 2.1 × 30 mm chromatography column).

Chiral HPLC analysis used Chiralpak IC-3 100 × 4.6 mm I.D., 3 µm, Chiralpak AD-3 150 × 4.6 mm I.D., 3 µm, Chiralpak AD-3 50 × 4.6 mm I.D., 3 µm, Chiralpak AS-3 150 × 4.6 mm I.D., 3 µm, Chiralpak AS-3 100 × 4.6 mm I.D., 3 µm, ChiralCel OD-3 150 × 4.6 mm I.D., 3 µm, Chiralcel OD-3 100 × 4.6 mm I.D., 3 µm, ChiralCel OJ-H 150 × 4.6 mm I.D., 5 µm, and Chiralcel OJ-3 150 × 4.6 mm I.D., 3 µm chromatography columns. Yantai Huanghai HSGF254 or Qingdao GF254 silica gel plates, 0.15-0.2 mm layer thickness, were adopted for thin-layer chromatography (TLC) analysis and 0.4-0.5 mm layer thickness for TLC separation and purification. Yantai Huanghai silica gel of 100-200 mesh, 200-300 mesh or 300-400 mesh was generally used as a carrier in column chromatography.

Preparative chiral chromatography used a DAICEL CHIRALPAK IC (250 mm × 30 mm, 10 µm) or Phenomenex-Amylose-1 (250 mm × 30 mm, 5 µm) column.

The CombiFlash preparative flash chromatograph used was CombiFlash Rf150 (TELEDYNE ISCO).

The mean inhibition of kinase and the IC₅₀ value were determined on a NovoStar microplate reader (BMG, Germany).

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Pressurized hydrogenation reactions were conducted using a Parr 3916EKX hydrogenator and a Qinglan QL-500 hydrogenator, or an HC2-SS hydrogenator. Hydrogenation reactions generally involved 3 cycles of vacuumization and hydrogen purging.

Microwave reactions were conducted on a CEM Discover-S 908860 microwave reactor.

In the examples, a solution was an aqueous solution unless otherwise specified.

In the examples, the reaction temperature was room temperature, i.e., 20 °C to 30 °C, unless otherwise specified.

The monitoring of the reaction progress in the examples was conducted by thin-layer chromatography (TLC). The developing solvent for reactions, the eluent system of column chromatography for compound purification and the developing solvent system of thin-layer chromatography include: A: dichloromethane/methanol system, B: n-hexane/ethyl acetate system, C: petroleum ether/ethyl acetate system, and D: petroleum ether/ethyl acetate/methanol system. The volume ratio of the solvents was adjusted according to the polarity of the compound, or by adding a small amount of basic or acidic reagents such as triethylamine and acetic acid.

Preparation of 1.0 M Tris buffer, pH = 8.30 ± 0.1:
6.0 g of tris was weighed into a 50 mL volumetric flask and dissolved by shaking in 40 mL of purified water, 1.2 mL of concentrated hydrochloric acid was added dropwise to adjust the pH to 8.30, and the solution was brought to volume with purified water. Preparation of buffer solution A:
KH₂PO₄ (8.50 g), K₂HPO₄ (8.56 g), NaCl (5.86 g) and EDTA (1.50 g) were added to a 2.0 L vessel and completely dissolved in 1.6 L of water for injection by half an hour's stirring, and the solution was then brought to volume (2.0 L) with water for injection. Measurement showed the pH was 6.30 ± 0.1.

The abbreviations used in the following experiments have the following meanings: DAST: diethylaminosulfur trifluoride; THF: tetrahydrofuran; NMP: *N-*methylpyrrolidone; DCM: dichloromethane; *m*-CPBA: m-chloroperoxybenzoic acid; DIEA: *N,N-*diisopropylethylamine; TEA: triethylamine; Boc: tert-butyloxycarbonyl; MeOH: methanol; Et₂O: diethyl ether.

### Example 1

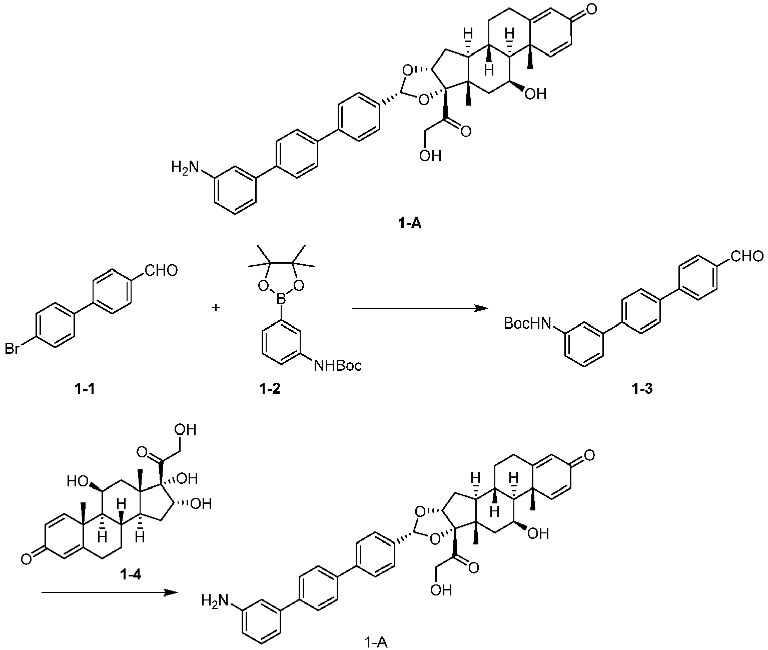

### Step 1

Compound **1-1** (500 mg, 1.9 mmol, 1.0 eq), compound **1-2** (730 mg, 2.3 mmol, 1.2 eq), Pd(PPh₃)₄ (660 mg, 0.57 mmol, 0.3 eq), K₂CO₃ (960 mg, 6.9 mmol, 3.6 eq) and DMF (35 mL, 70 V) were added to a 100 mL single-necked flask, purged with nitrogen three times and stirred at 80 °C until the reaction was complete. EA (50 mL) and H₂O (130 mL) were added to the reaction mixture. The aqueous phase was separated and extracted with EA (30 mL × 2). The organic phases were combined, washed with H₂O (50 mL × 3), a saturated solution of LiCl (50 mL) and then a saturated solution of NaCl (30 mL), dried over anhydrous Na₂SO₄, filtered, and then concentrated by rotary evaporation to remove the solvent. The residue was purified by column chromatography to give a crude product. The crude product was triturated with PE and EA (2:1), and the triturate was filtered to give compound **1-3** (220 mg, 31% yield).

Ms (ESI): m/z 318 [M-55]⁺.

### Step 2

Compound **1-3** (100 mg, 0.268 mmol, 1.1 eq), compound **1-4** (92 mg, 0.243 mmol, 1.0 eq), anhydrous MgSO₄ (146 mg, 1.215 mmol, 5.0 eq) and anhydrous CH₃CN (5 mL) were added to a 50 mL three-necked flask and stirred in a nitrogen atmosphere at room temperature for 0.7 h. The mixture was cooled to 0 °C in an ice bath, and CF₃SO₃H (182 mg, 1.215 mmol, 5.0 eq) was slowly added dropwise. After the addition, the reaction was naturally warmed to room temperature. After the reaction was complete, it was quenched with EA and a saturated solution of sodium bicarbonate in an ice bath, and the pH was adjusted to be greater than 8. Then the aqueous phase was extracted with EA, and the organic phases were combined, washed with saturated brine, dried over anhydrous sodium sulfate, and concentrated. The crude product was purified by prep-HPLC to give compound **1-A** (64.5 mg, 42% yield).

Ms (ESI): m/z 632.3 [M+H]⁺.

¹H NMR (400 MHz, DMSO) δ 7.73 (dd, *J=* 8.3, 2.5 Hz, 4H), 7.64 (d, *J=* 8.3 Hz, 2H), 7.57 (d, *J* = 8.2 Hz, 2H), 7.33 (d, *J* = 10.1 Hz, 1H), 7.11 (t, *J* = 7.8 Hz, 1H), 6.89 (s, 1H), 6.83 (d, *J* = 7.8 Hz, 1H), 6.57 (d, *J* = 7.9 Hz, 1H), 6.17 (d, *J* = 10.1 Hz, 1H), 5.95 (s, 1H), 5.52 (s, 1H), 5.17 (s, 2H), 5.12 (t, *J* = 5.9 Hz, 1H), 4.97 (d, *J* = 5.1 Hz, 1H), 4.82 (d, *J* = 2.8 Hz, 1H), 4.56 (dd, *J* = 19.6, 6.4 Hz, 1H), 4.32 (s, 1H), 4.22 (dd, *J* = 19.6, 5.5 Hz, 1H), 2.61-2.53 (m, 1H), 2.34 (d, *J* = 10.4 Hz, 1H), 2.20-2.01 (m, 2H),1.90 - 1.60 (m, 5H), 1.41 (s, 3H), 1.10-1.01 (m, 2H), 0.89 (s, 3H).

### Example 2

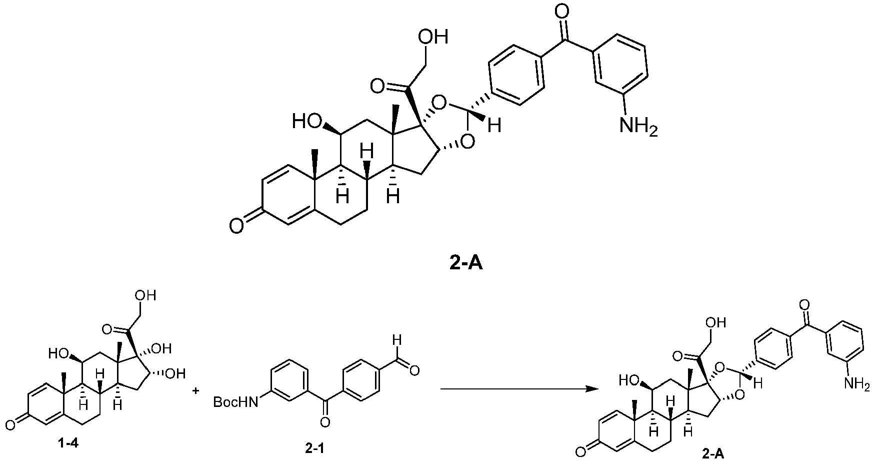

In a nitrogen atmosphere, compound **1-4** (176 mg, 0.47 mmol, 1.0 eq) and anhydrous magnesium sulfate (282 mg, 2.34 mmol, 5.0 eq) were added to a 25 mL reaction flask, and anhydrous acetonitrile (5 mL) was added. The reaction was stirred at room temperature for 90 min. Compound **2-1** (160 mg, 0.49 mmol, 1.05 eq) was then added to the above mixture. The mixture was cooled to 0-5 °C in an ice bath, and trifluoromethanesulfonic acid (351 mg, 2.34 mmol, 5.0 eq) was added using a syringe. The reaction was stirred in the ice bath until completion. The reaction mixture was filtered with celite, and the filter cake was rinsed with ethyl acetate. Ethyl acetate and water were added to the filtrate. The organic phase was separated, washed once with saturated brine, dried, and concentrated under reduced pressure to give a crude product. The crude product was purified by prep-HPLC to give compound **2-A** (88 mg, 32.2% yield).

Ms (ESI):m/z 584.42 [M+1]⁺.

### Example 3

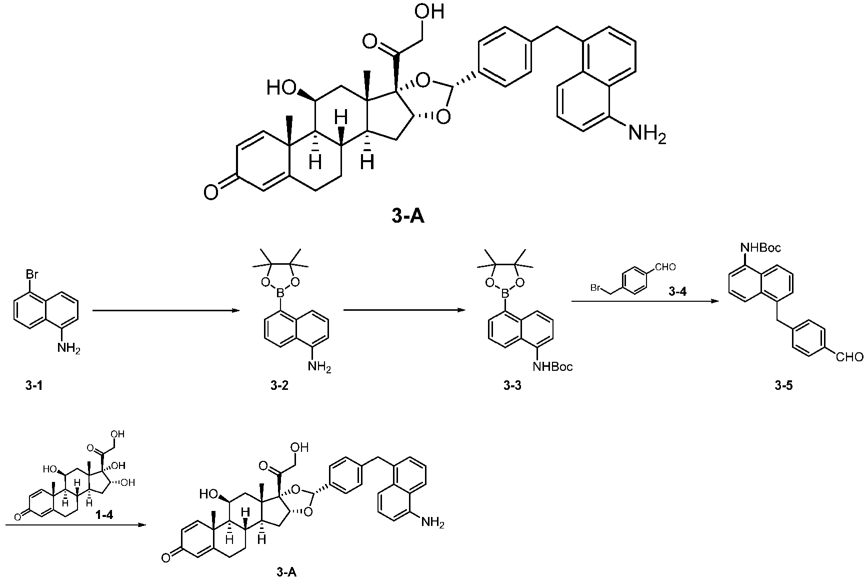

### Step 1

In a nitrogen atmosphere, compound **3-1** (10.0 g, 45.03 mmol), (Bpin)₂ (bis(pinacolato)diboron, 18.3 g, 72.05 mmol), X-Phos (1.36 g, 2.70 mmol) and KOAc (8.84 g, 90.06 mmol) were dissolved in 1,4-dioxane (150 mL). Pd₂(dba)₃ (1.65 g, 1.80 mmol) was added, and the reaction was heated to 90 °C. The reaction mixture was cooled to room temperature and filtered. The filtrate was concentrated and separated by column chromatography (PE/EA) to give compound **3-2** (13 g).

MS-ESI: m/z 270.2 [M+H]⁺.

### Step 2

Compound **3-2** (13 g, 45.03 mmol) was dissolved in toluene (100 mL), and Boc₂O (13.4 mL, 58.54 mmol) was added. The reaction was heated to 100 °C. The reaction mixture was cooled to room temperature, concentrated, and separated by column chromatography (PE/EA) to give compound **3-3** (16 g, 96.2% yield over two steps).

MS-ESI: m/z 396.1 [M+Na]⁺.

### Step 3

In a nitrogen atmosphere, compound **3-3** (2.0 g, 5.42 mmol), compound **3-4** (2.16 g, 10.84 mmol) and K₂CO₃ (3.75 g, 27.10 mmol) were dissolved in tetrahydrofuran (50 mL). Pd(dppf)Cl₂-DCM (441 mg, 0.54 mmol) was added, and the reaction was heated to 80 °C. The reaction mixture was cooled to room temperature, quenched with water, and extracted with ethyl acetate. After concentration, the residue was separated by column chromatography (PE/EA) to give compound **3-5** (1.33 g, 67.9% yield). MS-ESI: m/z 384.1 [M+Na]⁺.

### Step 4

In a nitrogen atmosphere, compound **1-4** (123 mg, 0.327 mmol) and MgSO₄ (197 mg, 1.635 mmol) were dissolved in acetonitrile (10 mL). The mixture was allowed to react at room temperature for 1 h. A solution of compound **3-5** (130 mg, 0.360 mmol) in acetonitrile (10 mL) was added. The mixture was cooled to 0 °C, and trifluoromethanesulfonic acid (145 µL, 1.635 mmol) was slowly added dropwise. After the addition, the reaction was naturally warmed to room temperature. After filtration, the filtrate was concentrated and separated by prep-HPLC (CH₃CN/H₂O) to give compound **3-A** (90 mg, 44.4% yield).

MS-ESI: m/z 620.3 [M+H]⁺.

¹H NMR (400 MHz, DMSO) δ 7.96 (dd, *J* = 7.0, 2.2 Hz, 1H), 7.38 - 7.26 (m, 5H), 7.20 (d, *J* = 8.2 Hz, 3H), 7.17 - 7.09 (m, 1H), 6.67 (d, *J* = 7.2 Hz, 1H), 6.14 (d, *J* = 10.1 Hz, 1H), 5.91 (s, 1H), 5.36 (s, 1H), 4.90 (d, *J=* 4.9 Hz, 1H), 4.75 (brs, 1H), 4.47 (d, *J=* 19.4 Hz, 1H), 4.32 (s, 2H), 4.27 (brs, 1H), 4.15 (d, *J* = 19.4 Hz, 1H), 2.58 - 2.50 (m, 3H), 2.34 - 2.23 (m, 1H), 2.08 (dd, *J* = 16.2, 6.0 Hz, 1H), 2.03 - 1.93 (m, 1H), 1.81 - 1.53 (m, 5H), 1.38 (s, 3H), 1.23 (s, 1H), 1.03 (ddd, *J* = 27.9, 11.3, 2.6 Hz, 2H), 0.84 (s, 3H).

### Example 4

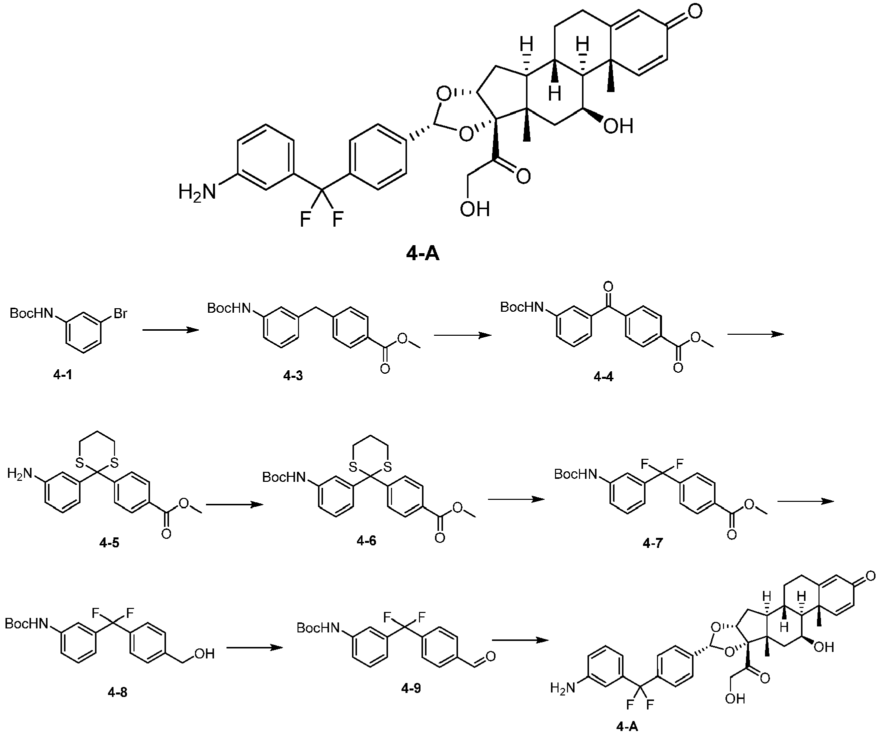

### Step 1

To a 1000 mL single-necked flask, compound **4-1** (48 g, 176.4 mmol, 1.0 eq), bis(pinacolato)diboron (71.7 g, 282.2 mmol, 1.6 eq), potassium acetate (34.6 g, 352.8 mmol, 2.0 eq), PdCl₂(dppf) (6.4 g, 8.82 mmol, 0.05 eq) and dioxane (500 mL) were added. The mixture was heated to 95 °C and stirred in a nitrogen atmosphere until the reaction was complete. The reaction was stopped, and the reaction mixture was cooled. Compound **4-2** (80.8 g, 352.8 mmol, 2.0 eq), potassium carbonate (48.8 g, 352.8 mmol, 2.0 eq) and PdCl₂(dppf) (6.4 g, 8.82 mmol, 0.05 eq) were added, and water (100 mL) was then added. The mixture was stirred, heated to 80 °C and stirred in a nitrogen atmosphere until the reaction was complete. After the reaction mixture was cooled, ethyl acetate and water were added, and the mixture was stirred and separated. After drying over anhydrous sodium sulfate and concentration, the crude product was purified by column chromatography to give compound **4-3** (about 54 g, 90% yield).

Ms (ESI): m/z 342.1 [M+1]⁺.

### Step 2

To a 500 mL three-necked flask, compound **4-3** (7.0 g, 20.5 mmol, 1.0 eq), potassium permanganate (9.7 g, 61.6 mmol, 3.0 eq) and tetra-tert-butylammonium bromide (20.0 g, 61.6 mmol, 3.0 eq) were added, and dichloroethane (140 mL) was then added. The mixture was stirred at room temperature in a nitrogen atmosphere until the reaction was complete. The reaction was stopped, and the reaction mixture was cooled in iced water. 10% sodium bisulfite and acetic acid were added. The organic layer was separated, dried over anhydrous sodium sulfate, filtered under reduced pressure, and concentrated. The crude product was purified by column chromatography to give compound **4-4** (about 6.2 g, 85% yield).

Ms (ESI): m/z 378.1 [M+23]⁺.

### Step 3:

To a 500 mL three-necked flask, compound **4-4** (20.0 g, 56.0 mmol, 1.0 eq), propanedithiol (12.1 g, 112.0 mmol, 2.0 eq) and boron trifluoride diethyl etherate (23.8 g, 168.0 mmol, 3.0 eq) were added, and chloroform (100 mL) was then added. The mixture was heated at reflux and stirred in a nitrogen atmosphere until the reaction was complete. The reaction was stopped, and the reaction mixture was cooled in iced water. Water was added, and the mixture was stirred to completely dissolve the solid. The organic layer was separated, dried over anhydrous sodium sulfate, filtered under reduced pressure, and concentrated. Petroleum ether was added to the crude product, and the mixture was stirred and filtered under reduced pressure to give compound **4-5** (about 22 g). The product was directly used in the next step.

Ms (ESI): m/z 346.0 [M+1]⁺.

### Step 4:

To a 100 mL three-necked flask, compound **4-5** (22.0 g, 56.0 mmol, 1.0 eq) and di-tert-butyl dicarbonate (24.4 g, 112.0 mmol, 2.0 eq) were added, and ethanol (60 mL) was then added. The mixture was heated to 50 °C and stirred in a nitrogen atmosphere until the reaction was complete. The reaction was stopped, and the reaction mixture was concentrated and purified by column chromatography to give compound **4-6** (about 18.5 g, 71% yield).

Ms (ESI): m/z 468.1 [M+23]⁺.

### Step 5:

To a 100 mL three-necked flask, compound **4-6** (4.7 g, 13.6 mmol, 1.0 eq) and DAST (6.6 g, 40.9 mmol, 3.0 eq) were added, and dichloromethane (50 mL) was then added. The mixture was heated to 50 °C and stirred in a nitrogen atmosphere until the reaction was complete. The reaction was stopped. The reaction was quenched with water under cooling in iced water. The organic layer was separated, dried over anhydrous sodium sulfate, filtered under reduced pressure, concentrated, and purified by column chromatography to give compound **4-7** (about 3.9 g, 76% yield).

Ms (ESI): m/z 400.1 [M+23]⁺.

### Step 6:

To a 100 mL three-necked flask, compound **4-7** (2.0 g, 5.3 mmol, 1.0 eq) was added and then dissolved in tetrahydrofuran (25 mL). In a nitrogen atmosphere, the solution was cooled to about 0 °C, and a 1.0 M solution of lithium aluminum hydride in tetrahydrofuran (8.0 mL, 8.0 mmol, 1.5 eq) was slowly added dropwise. The mixture was stirred at about 0 °C until the reaction was complete. The reaction was stopped. The reaction was quenched with water (0.8 mL) under cooling in iced water, and a 3.0 M aqueous solution of potassium hydroxide (0.8 mL) was added. Then water (1.6 mL) was added, and the mixture was stirred for 15 min and filtered under reduced pressure. The filtrate was dried over anhydrous sodium sulfate, filtered under reduced pressure, and concentrated to give compound **4-8** (about 2.2 g). The product was directly used in the next step.

Ms (ESI): m/z 372.1 [M+23]⁺.

### Step 7:

To a 100 mL three-necked flask, compound **4-8** (2.2 g, 5.3 mmol, 1.0 eq) was added and then dissolved in ethyl acetate (25 mL). In a nitrogen atmosphere, the solution was cooled to about 5 °C, and the Dess-Martin oxidant (6.7 g, 15.9 mmol, 3.0 eq) was added. The mixture was stirred at about 10 °C until the reaction was complete. The reaction was stopped, and the reaction mixture was filtered under reduced pressure. The filtrate was concentrated, and the crude product was purified by column chromatography to give compound **4-9** (about 1.5 g, 82% yield).

Ms (ESI): m/z 370.1 [M+23]⁺.

### Step 8:

To a 100 mL three-necked flask, compound **1-4** (1.2 g, 3.0 mmol, 1.0 eq) and compound **4-9** (1.1 g, 3.17 mmol, 1.05 eq) were added, anhydrous magnesium sulfate (1.8 g, 15.0 mmol, 5.0 eq) was added, and acetonitrile (25 mL) was then added. The mixture was stirred. In a nitrogen atmosphere, the mixture was cooled to below 0 °C, and trifluoromethanesulfonic acid (2.3 g, 15.0 mmol, 5.0 eq) was added. The mixture was stirred at about 0 °C until the reaction was complete. The reaction was stopped, and the reaction mixture was filtered under reduced pressure. The filtrate was directly purified by a preparative method to give compound **4-A** (about 1.5 g, 70% yield).

Ms (ESI): m/z 606.3 [M+1]⁺.

¹H-NMR (400 MHz, MeOD) δ 7.53 (m, 4H), 7.44 (m, 2H), 7.28 (m, 3H), 6.23 (dd, 1H), 5.99 (t, 1H), 5.52 (s, 1H), 5.08 (d, 1H), 4.63 (d, 1H), 4.37 (m, 2H), 2.65 (td, 1H), 2.36 (d, 1H), 2.25 (m, 1H), 2.13 (m, 1H), 1.95 (dd, 1H), 1.80 (m, 4H), 1.49 (s, 3H), 1.11 (dt, 1H), 1.10 (m, 4H).

### Example 5

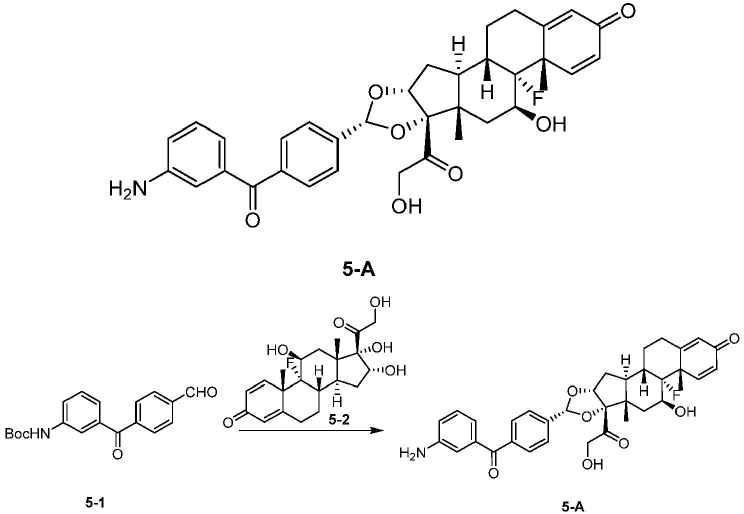

Compound **5-1** (85.3 mg, 0.262 mmol, 1.1 eq), compound **5-2** (94 mg, 0.238 mmol, 1.0 eq, Macklin/C10492138/P > 98%), anhydrous MgSO₄ (143 mg, 1.19 mmol, 5.0 eq) and anhydrous CH₃CN (4 mL) were added to a 25 mL Schlenk reaction flask and stirred in a nitrogen atmosphere at room temperature for 0.7 h. The mixture was cooled to 0 °C in an ice bath, and CF₃SO₃H (179 mg, 1.19 mmol, 5.0 eq) was slowly added dropwise. After the dropwise addition, the reaction was naturally warmed and completed. The reaction was quenched with EA and a saturated solution of sodium bicarbonate in an ice bath. Then the aqueous phase was extracted with EA, and the organic phases were combined, washed with saturated brine, dried over anhydrous sodium sulfate, and concentrated to give a crude product. The crude product was purified by prep-HPLC to give compound **5-A** (67.1 mg, 42.6% yield).

Ms (ESI): m/z 602.3 [M+H]⁺.

¹H NMR (400 MHz, DMSO) δ 7.71 (d, *J* = 8.1 Hz, 2H), 7.59 (d, *J* = 8.2 Hz, 2H), 7.29 (d, *J* = 10.2 Hz, 1H), 7.16 (t, *J* = 7.7 Hz, 1H), 6.92 (s, 1H), 6.87 - 6.76 (m, 2H), 6.22 (d, *J* = 10.2 Hz, 1H), 6.02 (s, 1H), 5.59 (s, 1H), 5.47 (d, *J* = 2.9 Hz, 1H), 5.39 (s, 2H), 5.13 (t, *J* = 5.9 Hz, 1H), 5.00 (d, *J* = 4.3 Hz, 1H), 4.57 (dd, *J* = 19.6, 6.5 Hz, 1H), 4.30-4.15 (m, 2H), 2.71-2.56 (m, 2H), 2.38-2.31 (m, 1H), 2.20-2.12 (m, 1H), 2.09-2.01 (m, 1H), 1.90-1.80 (m, 1H), 1.78 - 1.64 (m, 3H), 1.50 (s, 3H), 1.45-1.34 (m, 1H), 0.89 (s, 3H).

### Example 6

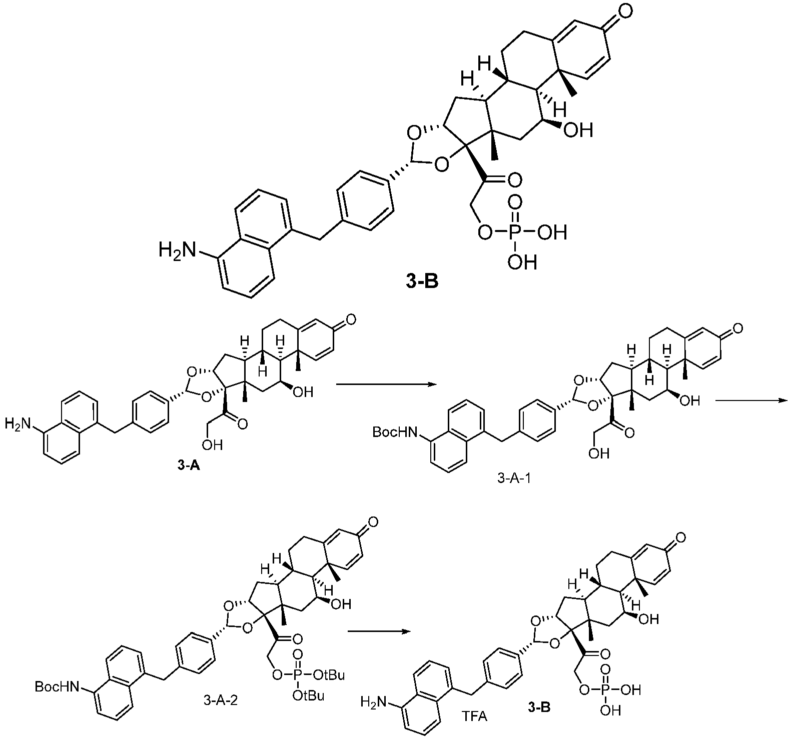

### Step 1

In a nitrogen atmosphere, compound **3-A** (90.0 mg, 0.145 mmol, 1.0 eq) was added to 2 mL of toluene, and BOC anhydride (63.3 mg, 0.290 mmol, 2.0 eq) was then added. The system was heated at 100 °C until the reaction was complete. The reaction mixture was directly concentrated to dryness under reduced pressure and purified by column chromatography to give compound **3-A-1** (48.0 mg).

MS-ESI: m/z 742.3 [M+Na]⁺.

### Step 2

In a nitrogen atmosphere, compound **3-A-1** (45.0 mg, 0.063 mmol) and tetrazole (66.0 mg, 0.945 mmol) were dissolved in N,N-dimethylacetamide (1.5 mL), and di-tert-butyl N,N-diethylphosphoramidite (187.0 mg, 0.756 mmol) was added. The mixture was allowed to react at room temperature for 2 h. The reaction was cooled to 0 °C, and H₂O₂ (50.0 mg, 0.82 mmol) was slowly added. After the addition, the mixture was stirred at room temperature until the reaction was complete. 2 mL of water was added, and the mixture was filtered. The filter cake was dried to give compound **3-A-2** (about 40.0 mg). The product was directly used in the next step.

MS-ESI: m/z 943.3 [M+Na]⁺.

### Step 3

In a nitrogen atmosphere, compound **3-A-2** (40.0 mg, 0.043 mmol) was dissolved in dichloromethane (1.0 mL). The solution was cooled to 0 °C, and trifluoroacetic acid (0.3 mL) was added. The mixture was allowed to react at room temperature for 3 h. After concentration, the residue was separated by prep-HPLC (CH₃CN/H₂O + 0.1% trifluoroacetic acid) to give compound **3-B** (about 12.0 mg).

ESI: m/z 700.3 [M+H]⁺.

### Example 7

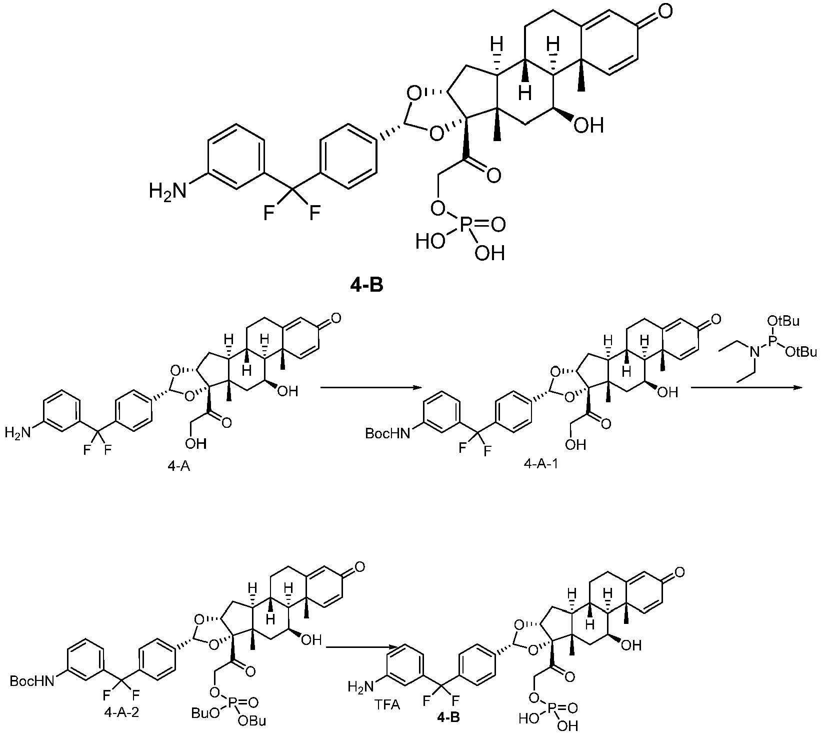

### Step 1

In a nitrogen atmosphere, compound **4-A** (45.0 mg, 0.074 mmol, 1.0 eq) was added to 2 mL of toluene, and BOC anhydride (32.0 mg, 0.158 mmol, 2.0 eq) was then added. The system was heated at 100 °C until the reaction was complete. The reaction mixture was directly concentrated under reduced pressure and purified by column chromatography to give compound **4-A-1** (45.2 mg).

MS-ESI: m/z 728.2 [M+Na]⁺.

### Step 2

In a nitrogen atmosphere, compound **4-A-1** (45.0 mg, 0.063 mmol) and tetrazole (66.0 mg, 0.945 mmol) were dissolved in N,N-dimethylacetamide (1.0 mL), and di-tert-butyl N,N-diethylphosphoramidite (249.0 mg, 0.756 mmol) was added. The mixture was allowed to react at room temperature for 2 h. The reaction was cooled to 0 °C, and H₂O₂ (50.0 mg, 0.82 mmol) was slowly added. After the addition, the mixture was stirred at room temperature until the reaction was complete. 2 mL of water was added, and the mixture was filtered. The filter cake was dried to give compound **4-A-2** (about 43.0 mg). The product was directly used in the next step.

MS-ESI: m/z 920.3 [M+Na]⁺.

### Step 3

In a nitrogen atmosphere, compound **4-A-2** (40.0 mg, 0.042 mmol) was dissolved in dichloromethane (1.0 mL). The solution was cooled to 0 °C, and trifluoroacetic acid (0.3 mL) was added. The mixture was allowed to react at room temperature for 3 h. After concentration, the residue was separated by prep-HPLC (CH₃CN/H₂O + 0.1% trifluoroacetic acid) to give compound **4-B** (about 15.0 mg).

ESI: m/z 686.2 [M+H]⁺.

### Example 8

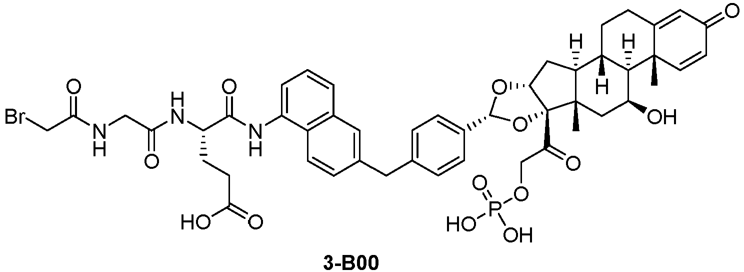

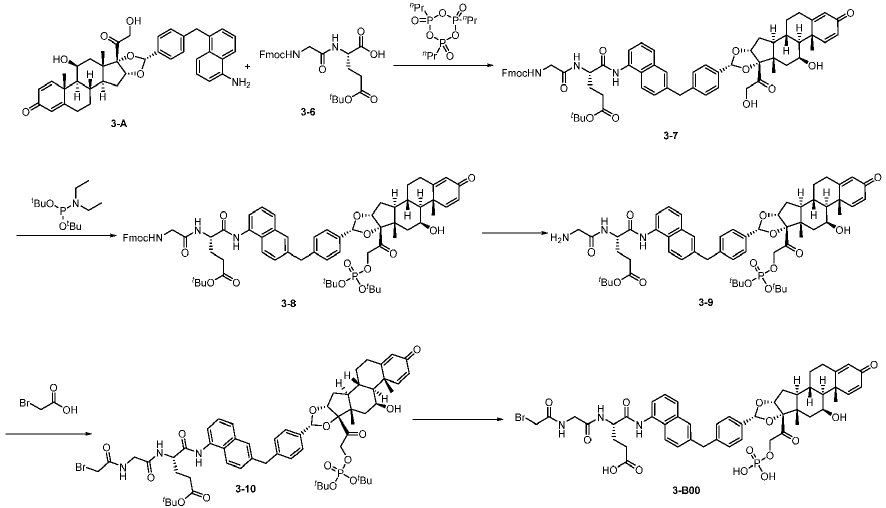

### Step 1

In a nitrogen atmosphere, compounds **3-A** (2.60 g, 4.20 mmol) and **3-6** (2.03 g, 4.20 mmol) were dissolved in N,N-dimethylacetamide (30 mL), and triethylamine (1.27 g, 12.60 mmol) was added. The mixture was cooled to 0 °C, and T₃P (5.35 g, 8.40 mmol, 50% in DMF) was slowly added. The reaction was completed at room temperature. The reaction mixture was directly purified by prep-HPLC to give the product **3-7** (965 mg, 21.2% yield).

MS-ESI: m/z 1106.5 [M+Na]⁺.

### Step 2

In a nitrogen atmosphere, compound **3-7** (805 mg, 0.778 mmol) and tetrazole (818 mg, 11.670 mmol) were dissolved in N,N-dimethylacetamide (10 mL), and di-tert-butyl N,N-diethylphosphoramidite (2.6 mL, 9.336 mmol) was added. The mixture was allowed to react at room temperature for 2 h. The reaction was cooled to 0 °C, and H₂O₂ (437 µL, 4.279 mmol, 30% in water) was slowly added. After the addition, the mixture was stirred at room temperature for 1 h. After concentration, the residue was separated by column chromatography (CH₃CN/H₂O) to give compound **3-8** (692 mg, 73.1% yield).

### Step 3

In a nitrogen atmosphere, compound **3-8** (830 mg, 0.650 mmol) was dissolved in acetonitrile (20 mL), and piperidine (302 µL, 3.250 mmol) was added. The mixture was allowed to react at room temperature. After concentration, the residue was triturated with 15 mL of petroleum ether three times to give compound **3-9** (645 mg). The product was directly used in the next step.

MS-ESI: m/z 1054.5 [M+H]⁺.

### Step 4

In a nitrogen atmosphere, 2-bromoacetic acid (170 mg, 1.224 mmol) and EEDQ (305 mg, 1.224 mmol) were dissolved in N,N-dimethylacetamide (5 mL). The mixture was allowed to react at room temperature for 1 h. A solution of compound **3-9** (645 mg, 0.612 mmol) in N,N-dimethylacetamide (5 mL) was added, and the mixture was allowed to react at room temperature. The reaction mixture was diluted with dichloromethane (200 mL) and washed with a 1 M aqueous solution of hydrobromic acid, a saturated solution of sodium bicarbonate and then saturated brine. The organic phase was concentrated to give compound **3-10** (830 mg). The product was directly used in the next step.

MS-ESI: m/z 1196.4 [M+Na]⁺.

### Step 5

In a nitrogen atmosphere, compound **3-10** (830 mg, 0.706 mmol) was dissolved in dichloromethane (8 mL). The solution was cooled to 0 °C, and trifluoroacetic acid (4 mL) was added. The mixture was allowed to react at room temperature. After concentration, the residue was separated by prep-HPLC (CH₃CN/H₂O + 0.1% trifluoroacetic acid) to give compound **3-B00** (220 mg, 33.6% yield over three steps).

MS-ESI: m/z 1028.2 [M+Na]⁺.

¹H NMR (400 MHz, DMSO) δ 9.98 (s, 1H), 8.55 (t, *J* = 5.4 Hz, 1H), 8.36 (d, *J* = 7.4 Hz, 1H), 7.97-7.81 (m, 2H), 7.55-7.46 (m, 2H), 7.45-7.38 (m, 2H), 7.36-7.26 (m, 3H), 7.23-7.16 (m, 2H), 6.14 (dd, *J=* 10.0, 1.1 Hz, 1H), 5.90 (s, 1H), 5.44 (s, 1H), 4.97-4.79 (m, 3H), 4.65-4.50 (m, 2H), 4.42 (s, 2H), 4.27 (brs, 1H), 3.94 (s, 2H), 3.86 (d, *J* = 5.5 Hz, 2H), 2.41-2.22 (m, 4H), 2.17-1.88 (m, 6H), 1.81-1.56 (m, 6H), 1.37 (s, 3H), 1.06-0.92 (m, 2H), 0.85 (s, 3H).

### Example 9

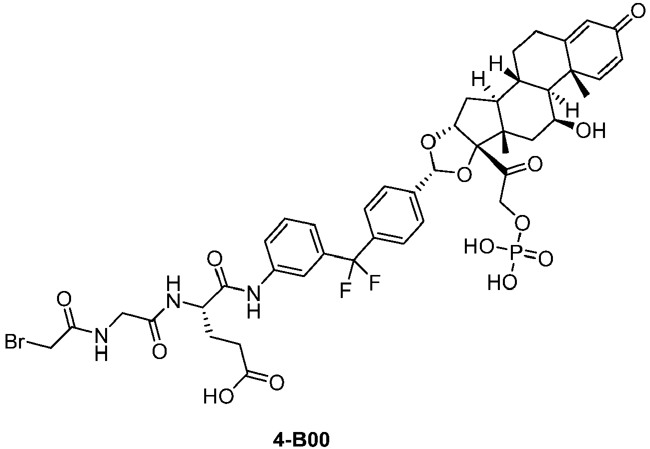

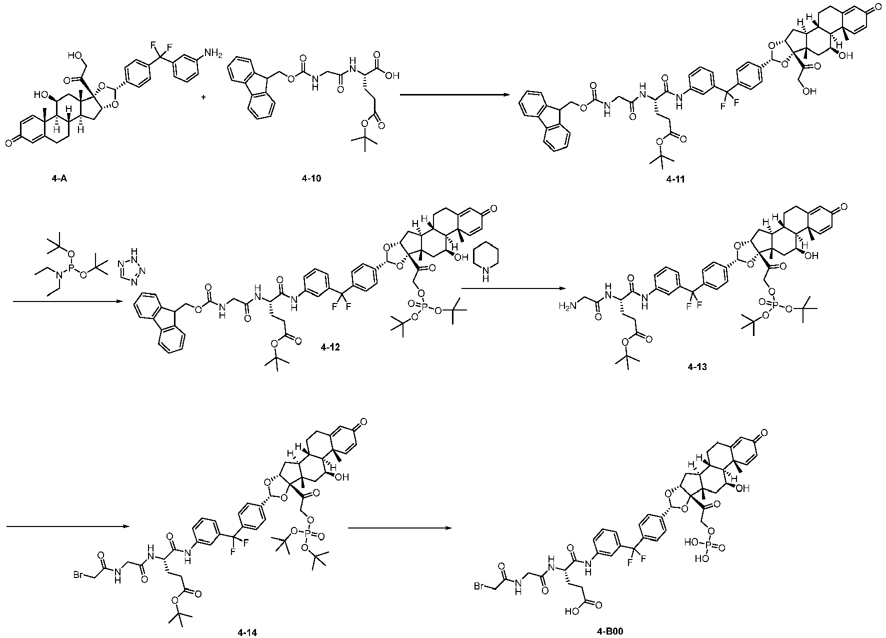

### Step 1

To a 25 mL three-necked flask, compound **4-A** (0.329 g, 0.544 mmol, 1.05 eq) was added, and compound **4-10** (0.25 g, 0.52 mmol, 1.0 eq), triethylamine (0.25 g, 1.56 mmol, 3.0 eq) and DMF (2 mL) were added. After the addition, the flask was cooled in an ice bath for 5-10 min to reduce the internal temperature to -5 °C, and then T3P (50% DMF) (0.9 mL, 1.82 mmol, 3.5 eq) was slowly added. After the addition, the mixture was naturally warmed and stirred until the reaction was complete. The reaction mixture was directly purified by pre-HPLC to give compound **4-11** (223 mg, 40% yield).

Ms (ESI): m/z 1092.4 [M+Na]⁺.

### Step 2

To a 50 mL three-necked flask, compound **4-11** (0.22 g, 0.206 mmol, 1.0 eq) was added, and the starting material tetrazole (0.20 g, 2.87 mmol, 14.0 eq), di-tert-butyl N,N-diethylphosphoramidite (0.616 g, 2.47 mmol, 12.0 eq) and DMF (2.6 mL) were added. After the addition, the mixture was allowed to react at room temperature for 2 h, cooled to 0 °C in an ice bath, and then H₂O₂ (30%) (0.13 g, 0.57 mmol, 5.5 eq) was slowly added. After the addition, the mixture was stirred at room temperature until the reaction was complete. The reaction mixture was directly purified by pre-HPLC to give compound **4-12** (184.1 mg, 70.8% yield).

Ms (ESI): m/z 1284.6 [M+Na]⁺.

### Step 3

To a 25 mL single-necked flask, compound **4-12** (0.285 g, 0.233 mmol, 1.0 eq) was added, and the starting material piperidine (0.17 g, 1.96 mmol, 8.5 eq) and acetonitrile (5 mL) were added. After the addition, the mixture was stirred at room temperature. The reaction mixture was concentrated under reduced pressure and triturated with 5 mL of petroleum ether. The triturate was stirred at room temperature and then filtered. The filter cake was washed with 2 mL of petroleum ether twice to give compound **4-13** (209 mg, 91% yield).

Ms (ESI): m/z 1004.4 [M+H]⁺.

### Step 4

To a 25 mL single-necked flask, the starting material 2-bromoacetic acid (0.074 g, 0.523 mmol, 2.6 eq) was added, and the starting material EEDQ (0.13 g, 0.523 mmol, 2.6 eq) and DMF (1 mL) were added. After the addition, the mixture was stirred at room temperature for 1 h, and a solution of compound **4-13** (0.21 g, 0.201 mmol, 1.0 eq) in DMF (0.5 mL) was added. After the addition, the mixture was stirred at room temperature until the reaction was complete. The reaction mixture was first diluted with dichloromethane (40 mL), then washed with 1 M HBr (10 mL × 2), then washed with saturated sodium bicarbonate (20 mL × 2), and finally washed with saturated brine. The organic phase was dried over anhydrous sodium sulfate, filtered, and concentrated under reduced pressure using an oil pump to give compound **4-14** (230 mg, crude).

Ms (ESI): m/z 1182.4 [M+Na]⁺.

### Step 5

To a 25 mL single-necked flask, compound **4-14** (0.240 g, 0.201 mmol, 1.0 eq) and DCM (2 mL) were added. After the addition, the mixture was cooled to 0 °C in an ice bath, and trifluoroacetic acid (1 mL) was slowly added. After the addition, the mixture was stirred at room temperature until the reaction was complete. The reaction mixture was concentrated under reduced pressure in an ice bath, then purified by pre-HPLC, and lyophilized to give compound **4-B00** (78 mg, 39.2% yield).

Ms (ESI): m/z 1014.2 [M+Na]⁺.

¹H NMR (400 MHz, DMSO) δ 10.15 (s, 1H), 8.53 (t, *J* = 5.2 Hz, 1H), 8.29 (br, d, *J* = 7.5 Hz, 1H), 7.83 (s, 1H), 7.73 (d, *J* = 7.8 Hz, 1H), 7.68-7.58 (m, 2H), 7.57-7.47 (m, 2H), 7.43 (t, *J* = 7.9 Hz, 1H), 7.32 (d, *J* = 9.9 Hz, 1H), 7.20 (d, *J* = 8.2 Hz, 1H), 6.17 (d, *J* = 10.1 Hz, 1H), 5.92 (s, 1H), 5.58 (s, 1H), 4.95-4.80 (m, 3H), 4.58 (br, dd, *J* = 18.4, 8.2 Hz, 1H), 4.40-4.37 (m, 1H), 4.33-4.27 (m, 1H), 3.93 (s, 2H), 3.82-3.78 (m, 2H), 2.30-2.25 (m, 3H), 2.16-2.07 (m, 1H), 2.10-1.90 (m, 2H), 1.88-1.59 (m, 6H), 1.39 (s, 3H), 1.18-1.05 (m, 2H), 0.89 (s, 3H).

### Example 10

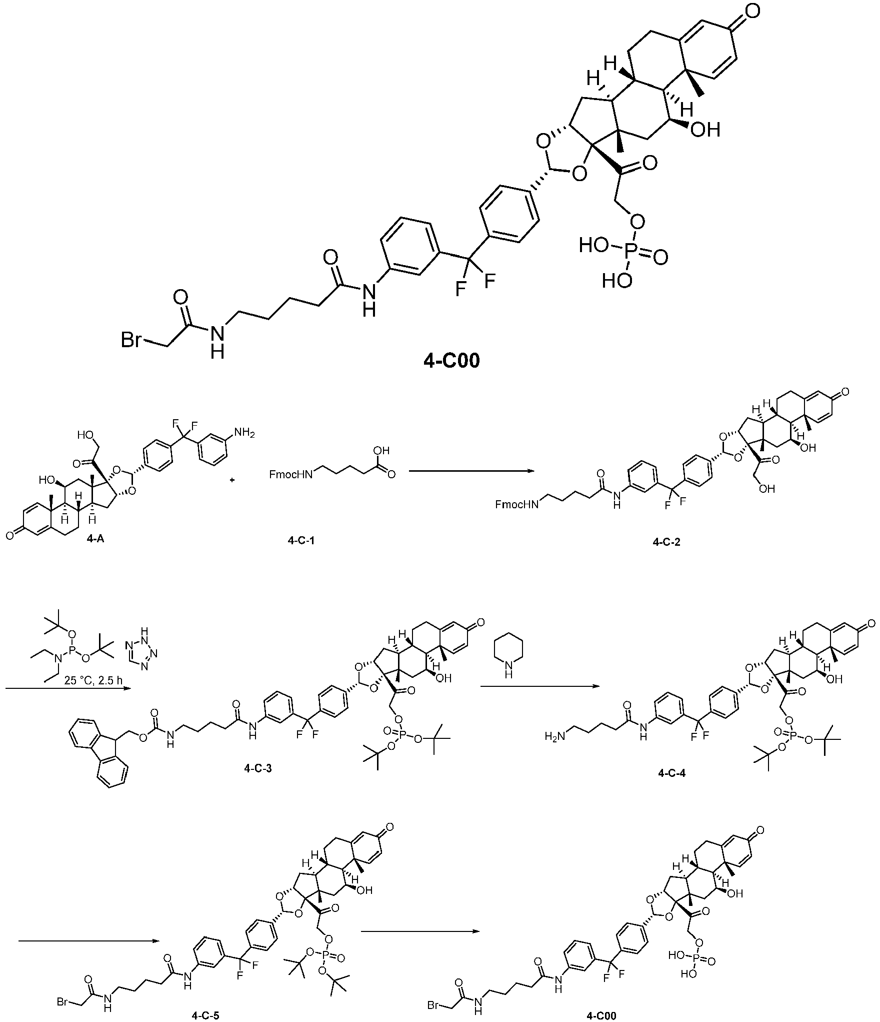

### Step 1

To a 25 mL three-necked flask, compound **4-A** (0.260 g, 0.430 mmol, 1.05 eq), compound **4-C-1** (0.193 g, 0.551 mmol, 1.32 eq), triethylamine (0.125 g, 1.23 mmol, 3.0 eq) and DMF (2 mL) were added. After the addition, the mixture was cooled to -5 °C in an ice bath, and then T3P (50% DMF) (0.5 mL, 1.024 mmol, 2.5 eq) was slowly added. The mixture was naturally warmed and stirred until the reaction was complete. The reaction mixture was directly purified by pre-HPLC (TFA (0.05%) water-acetonitrile) to give compound **4-C-2** (140 mg, 35.1% yield).

Ms (ESI): m/z 949.3 [M+Na]⁺.

### Step 2

To a 50 mL three-necked flask, compound **4-C-2** (0.34 g, 0.366 mmol, 1.0 eq) was added, and the starting material tetrazole (0.360 g, 5.13 mmol, 14.0 eq), di-tert-butyl N,N-diethylphosphoramidite (1.099 g, 4.40 mmol, 12.0 eq) and DMF (5 mL) were added. After the addition, the mixture was allowed to react at room temperature for 2 h, cooled to 0 °C in an ice bath, and then H₂O₂ (30%) (0.234 g, 2.02 mmol, 5.5 eq) was slowly added. After the addition, the mixture was stirred at room temperature until the reaction was complete. The reaction mixture was directly purified using a medium-pressure boston reversed-phase column to give compound **4-C-3** (304 mg, 64.1% yield).

Ms (ESI): m/z 1141.3 [M+Na]⁺.

### Step 3

To a 25 mL single-necked flask, compound **4-C-3** (0.304 g, 0.272 mmol, 1.0 eq) was added, and the starting material piperidine (0.255 g, 1.63 mmol, 9.0 eq) and acetonitrile (8 mL) were added. After the addition, the mixture was stirred at room temperature until the reaction was complete. The reaction mixture was concentrated under reduced pressure and triturated with 4 mL of petroleum ether. The triturate was stirred at 35 °C for 2 h and then filtered. The filter cake was washed with 2 mL of petroleum ether twice to give compound **4-C-4** (243 mg, 99% yield).

Ms (ESI): m/z 897.6 [M+H]⁺.

### Step 4

To a 25 mL single-necked flask, the starting material 2-bromoacetic acid (0.112 g, 0.813 mmol, 3.0 eq) was added, and the starting material EEDQ (0.201 g, 0.813 mmol, 3.0 eq) and DMF (2 mL) were added. After the addition, the mixture was stirred at room temperature for 0.6 h, and a solution of compound **4-C-4** (0.243 g, 0.271 mmol, 1.0 eq) in DMF (1 mL) was added. After the addition, the mixture was stirred at room temperature until the reaction was complete. The reaction mixture was first diluted with dichloromethane (50 mL), then washed with 1 M HBr (15 mL × 2), then washed with saturated sodium bicarbonate (15 mL × 2), and finally washed with saturated brine. The organic phase was dried over anhydrous sodium sulfate, filtered, concentrated to dryness by rotary evaporation under reduced pressure using an oil pump, and dried by nitrogen blowing for 10 min to give compound **4-C-5** (275 mg, crude).

Ms (ESI): m/z 1039.1 [M+Na]⁺ and 1041.1 [M+Na+2]⁺.

### Step 5

To a 25 mL single-necked flask, the starting material compound **4-C-5** (0.275 g, 0.271 mmol, 1.0 eq) and DCM (2.5 mL) were added. After the addition, the mixture was cooled to 0 °C in an ice bath, and trifluoroacetic acid (1 mL) was slowly added. After the addition, the mixture was stirred at room temperature until the reaction was complete. The reaction mixture was concentrated under reduced pressure in an ice bath, then purified by pre-HPLC, and lyophilized to give compound **4-C00** (120 mg, 48.3% yield).

Ms (ESI): m/z 905.2 [M+H]⁺.

### Example 11

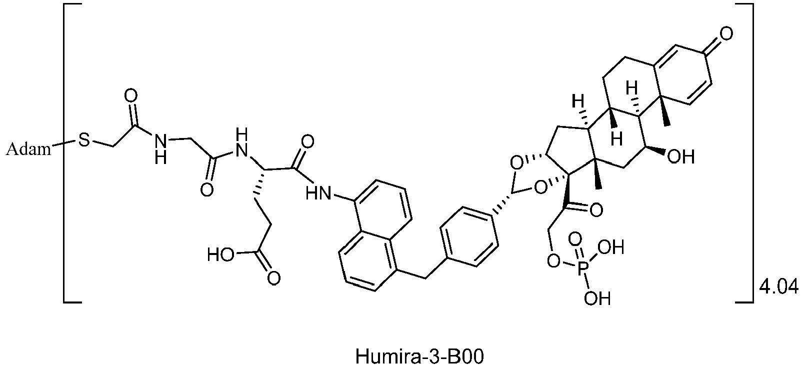

To adalimumab in buffer A (a 0.05 M aqueous buffer solution having a pH of 6.3; 10.0 mg/mL, 6.0 mL, 405.11 nmol), a prepared aqueous solution of tris(2-carboxyethyl)phosphine (TCEP) (2.5 mM, 356.8 µL, 891.24 nmol) was added at 37 °C. The mixture was allowed to react in a shaking water bath at 37 °C for 3 h, and the reaction was stopped. The reaction mixture was cooled to 25 °C in a water bath. 1.0 M Tris buffer (840 µL) was added to the above reaction mixture, and then a solution of compound **3-B00** (4.08 mg, 4051.10 nmol) in 300 µL of DMSO was added to the above reaction mixture. The mixture was allowed to react in a shaking water bath at 25 °C for 3 h, and the reaction was stopped. The reaction mixture was desalted and purified using a Sephadex G25 gel column (elution phase: buffer A) and concentrated using an ultrafiltration tube to give antibody-drug conjugate **Humira-3-B00** in buffer A (2.55 mg/mL, 23.5 mL). The product was stored frozen at 4 °C.

### Example 12

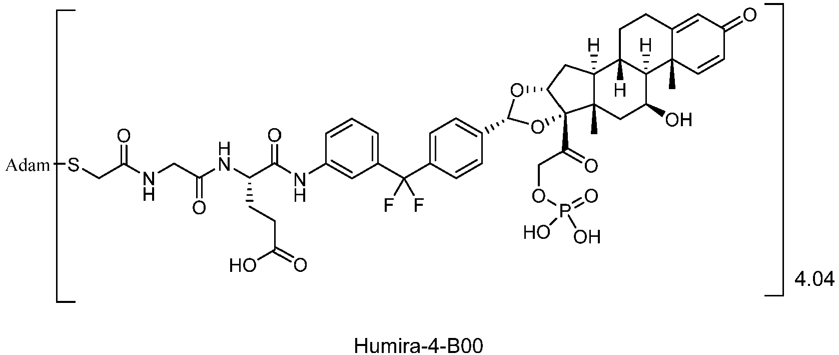

To adalimumab in buffer A (a 0.05 M aqueous buffer solution having a pH of 6.3; 10.0 mg/mL, 6.0 mL, 405.11 nmol), a prepared aqueous solution of tris(2-carboxyethyl)phosphine (TCEP) (2.5 mM, 405.4 µL, 1012.77 nmol) was added at 37 °C. The mixture was allowed to react in a shaking water bath at 37 °C for 3 h, and the reaction was stopped. The reaction mixture was cooled to 25 °C in a water bath. 1.0 M Tris buffer (840 µL) was added to the above reaction mixture, and then a solution of compound **4-B00** (4.02 mg, 4051.10 nmol) in 300 µL of DMSO was added to the above reaction mixture. The mixture was allowed to react in a shaking water bath at 25 °C for 3 h, and the reaction was stopped. The reaction mixture was desalted and purified using a Sephadex G25 gel column (elution phase: buffer A) and concentrated using an ultrafiltration tube to give the title product antibody-drug conjugate **Humira-4-B00** in buffer A (2.58 mg/mL, 23.25 mL). The product was stored frozen at 4 °C.

### Biological Evaluation

The present disclosure is further described and explained below with reference to test examples, which are not intended to limit the scope of the present disclosure.

### Test Example 1: In Vitro Activity of Small-Molecule Steroids

### 1. Test samples

Compounds 1-A to 5-A, and compound A-A (prepared according to Example 2 of WO2017210471).

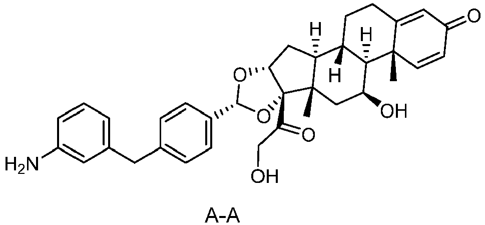

### 2. Glucocorticoid receptor binding assay

The binding activity of small-molecule steroids for glucocorticoid receptor (GR) was tested using a human glucocorticoid NHR (radiolabeled agonist) binding assay (#232020, eurofins). The assays were based on the following principle: Different concentrations of a small-molecule steroid and 5 nM [3H]dexamethasone are incubated with human recombinant GR at 4 °C for 24 h. The small-molecule steroid competes with [3H]dexamethasone for binding to human GR. The binding activity of the small-molecule steroid for GR can be calculated by counting the number of [3H]dexamethasone molecules that bind specifically to the receptor. The results are detailed in Table 1.

### 3. Mineralocorticoid receptor agonist activity assay

The mineralocorticoid receptor (MR) agonist activity of small-molecule steroids was tested using a PathHunter^{®} NHR nuclear translocation assay. PathHunter NHR CHO-K1 cells were plated onto a 384-well white-wall microplate and co-incubated with test small-molecule steroids at 37 °C in 5% CO₂ to induce reactions. Assay signals were generated using the PathHunter assay reagent mix. After one hour of incubation at room temperature, chemiluminescent signals were detected. The data were analyzed using four-parameter curve fitting to generate EC₅₀ values. The results are detailed in Table 1.

### 4. GRE reporter gene assay

A549 cells were plated (40,000 cells/96-well plate), transfected with pGL4.36 (MMTV-Luc, 100 ng/well) using lipo3000, cultured overnight and then incubated with different concentrations of small-molecule steroids. After 24 h, the expression of luciferase in the reporter gene system was detected using the Bright-Glo (promega) assay reagent.

**Table 1. In vitro activity of small-molecule steroids**

| **Compound No.** | **GR binding IC₅₀ (nM)** | **GRE reporter gene EC₅₀ (nM)** | **GRE reporter gene Eₘₐₓ (RLU)** | **MR agonist EC₅₀ (µM)** |
|---|---|---|---|---|
| **Compound A-A** | 2.52 | 0.60 | 1.45E6 | 0.06 |
| **Compound 1-A** | 4.37 | 22.65 | 7.67E5 | > 200 |
| **Compound 2-A** | 6.69 | / | / | 0.22 |
| **Compound 3-A** | 2.24 | 1.47 | 1.53E6 | 0.19 |
| **Compound 4-A** | 2.12 | 2.16 | 1.69E6 | 0.26 |
| **Compound 5-A** | 5.23 | 4.3 | 2.3E7 | / |

### Test Example 2: Anti-Inflammatory Activity of Small-Molecule Steroids in Lipopolysaccharide (LPS)'s Stimulation of Human PBMCs' Cytokine Secretion

### 1. Test samples

Compounds 1-A to 5-A, and compound A-A.

### 2. Test method

Frozen primary human peripheral blood mononuclear cells (PBMCs) were resuspended in RPMI (2% FBS, 1% penicillin-streptomycin) and plated onto a 96-well plate. After PBMCs were co-incubated with different concentrations of small-molecule steroids at 37 °C in 5% CO₂ for 4 h, 0.01 ng/mL LPS was added for overnight stimulation. The following day, the medium supernatants were collected and assayed for IL-6 concentration using alpha LISA (Cisbio).

### 3. Test results

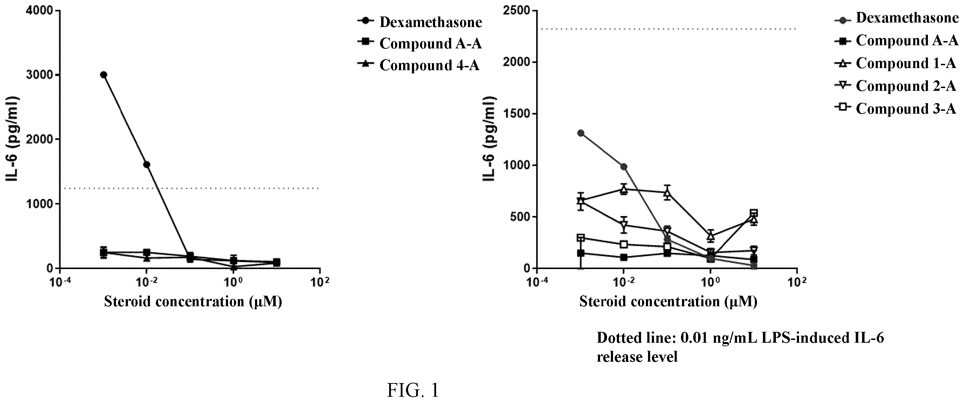

The test results are shown in FIG. 1: the small-molecule steroid compounds of the present disclosure can significantly inhibit LPS-induced IL-6 release.

### Test Example 3: Activity of Anti-TNF-ADCs in Membrane-Bound TNFα-Mediated GRE Reporter Gene System

### 1. Test samples

Humira-3-B00, Humira-4-B00, and Humira-A-B00 (prepared according to Example 7 of WO2019106609).

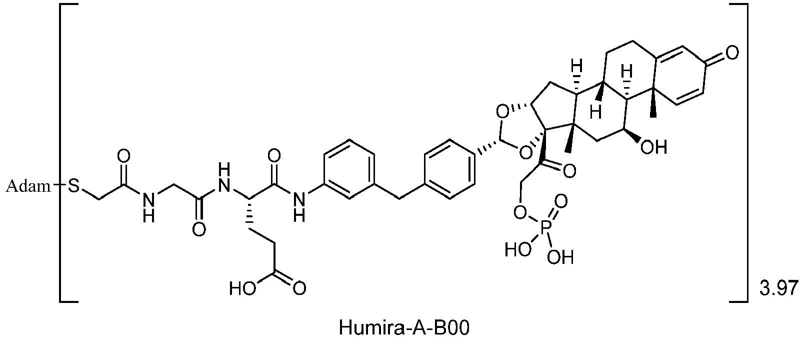

### 2. Test method

A reporter gene cell line stably transfected with MMLV-Luc was established using lentiviruses in Hela cells. The stably transfected Hela-MMTV-Luc cell line was plated onto a 96-well plate (30000 cells/well), and cells in each well were transfected with an empty plasmid or a human TNFα mutant plasmid (TNFαΔ12, with the restriction enzyme cutting site for TACE removed, 50 ng/well) using lipo3000. After being left overnight, the cells were incubated with different concentrations of anti-TNF-ADCs. After 24 h, the expression of luciferase in the reporter gene system was detected using the Bright-Glo (promega) assay reagent.

### 3. Test results

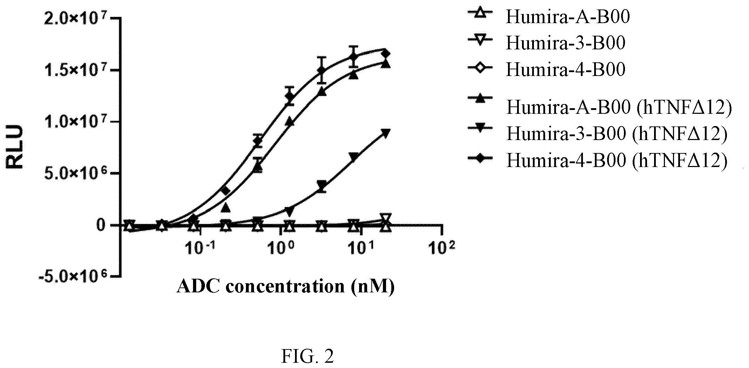

The test results are shown in Table 2 and FIG. 2.

**Table 2. The activity of anti-TNF-ADCs in a membrane-bound TNFα-mediated GRE reporter gene system**

| **hTNF△12** | **Humira-A-B00** | **Humira-3-B00** | **Humira-4-B00** |
|---|---|---|---|
| **EC₅₀ (nM)** | 0.84 | 7.4 | 0.55 |

### Test Example 4: Activity of Anti-TNF-ADCs in Lipopolysaccharide (LPS)'s Stimulation of Human Monocytes' Cytokine Secretion

Monocytes were selected and enriched from frozen primary human peripheral blood PBMCs by sorting using an EsaySep^{™} human CD14 sorting kit and plated onto a 96-well plate. After monocytes were co-incubated with different concentrations of anti-TNF-ADCs at 37 °C in 5% CO₂ for 4 h, 0.01 ng/mL LPS was used for overnight stimulation. The following day, the medium supernatants were collected and assayed for IL-6 concentration using alpha LISA (cisbio).

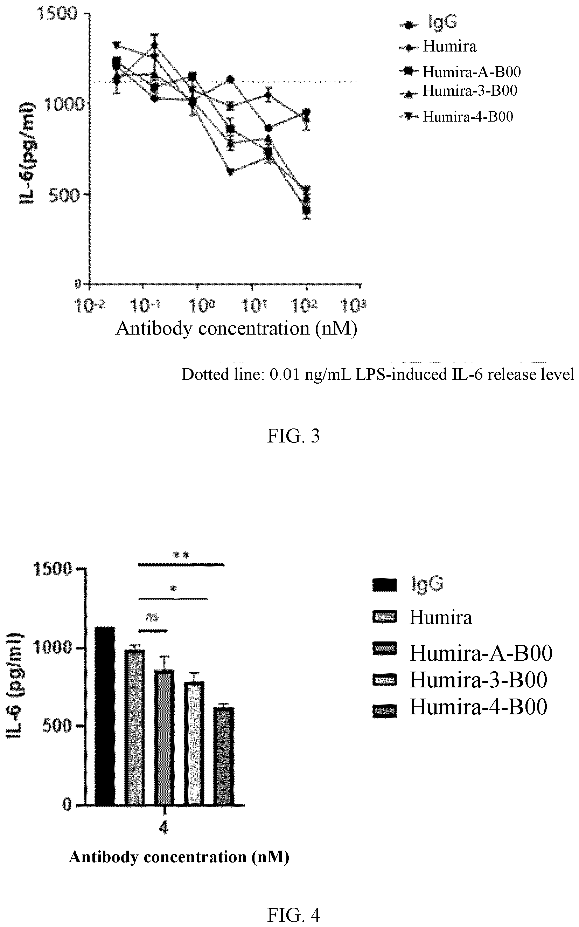

Compared to adalimumab (Humira), the ADCs of the present disclosure are effective in inhibiting LPS-induced IL-6 secretion in high concentrations (4-100 nM) (as shown in FIG. 3). In addition, 4 nM Humira-3-B00 and Humira-4-B00 have significantly higher anti-inflammatory activity than the ADC molecule Humira-A-B00 in that concentration (as shown in FIG. 4).

### Test Example 5: Mouse Collagen Antibody-Induced Arthritis (CAIA) Model

### Test animals:

Male balb/c mice, 6 w, purchased from the Laboratory Animal Management Department, Shanghai Institute of Planned Parenthood Research. Housing environment: SPF; production license SCXK (Shanghai) 2018-0006; Balb/c mouse certification number: 20180006023393.

### Test samples:

Humira-3-B00, Humira-4-B00, and Humira-A-B00.

### Test method:

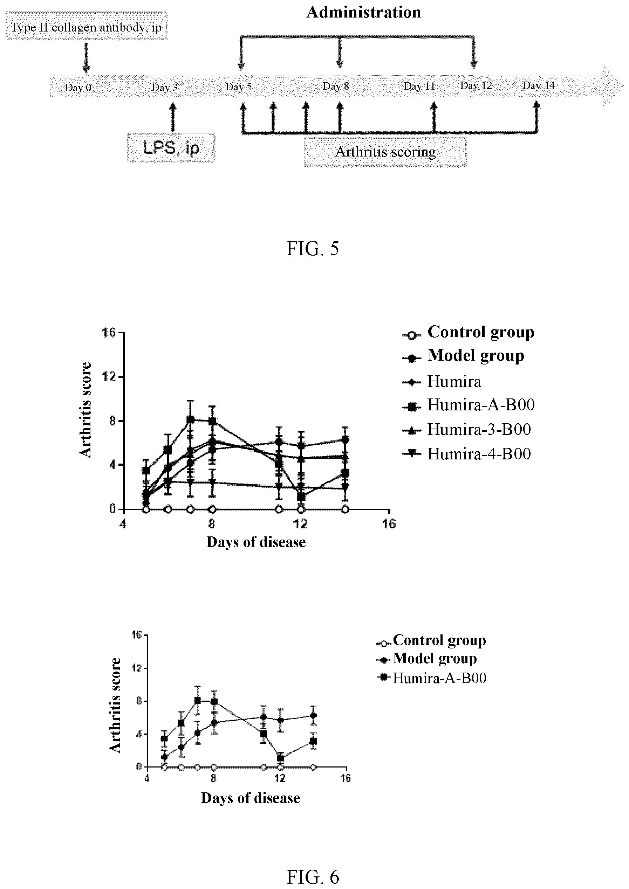

Upon arrival, the test animals were acclimatized for 7 days and randomly grouped. On day 0, each group of mice was intraperitoneally injected with 1.5 mg/mouse of type II collagen antibody cocktail (purchased from Chondrex Inc.) for modeling except for the control group. On day 3, each group of mice was intraperitoneally injected with 50 µg/mouse of LPS (100 µL) to boost the immune response except for the control group. Administration to each group of mice began on day 5, and limb arthritis was scored every 1-3 days. The specific experimental process is shown in FIG. 5. Mice were dosed according to the following administration regimens. The severity of arthritis in each group of mice was semi-quantitatively scored every 1-3 days and further, the anti-inflammatory activity of anti-TNF-ADCs was evaluated.

**Table 3: Administration regimens**

| Group No. | Group | Number | CII Ab (mg/mouse) | Dose (mg/kg) | Route of administration | Frequency of administration |
|---|---|---|---|---|---|---|
| 1 | Control group | 4 | - | NA | Intraperitoneally | Twice a week |
| 2 | Model group | 8 | 1.5 | 10 | Intraperitoneally | Twice a week |
| 3 | Humira | 8 | 1.5 | 10 | Intraperitoneally | Twice a week |
| 4 | Humira-A-B00 | 8 | 1.5 | 10 | Intraperitoneally | Twice a week |
| 5 | Humira-3-B00 | 8 | 1.5 | 10 | Intraperitoneally | Twice a week |
| 6 | Humira-4-B00 | 8 | 1.5 | 10 | Intraperitoneally | Twice a week |

### Test results:

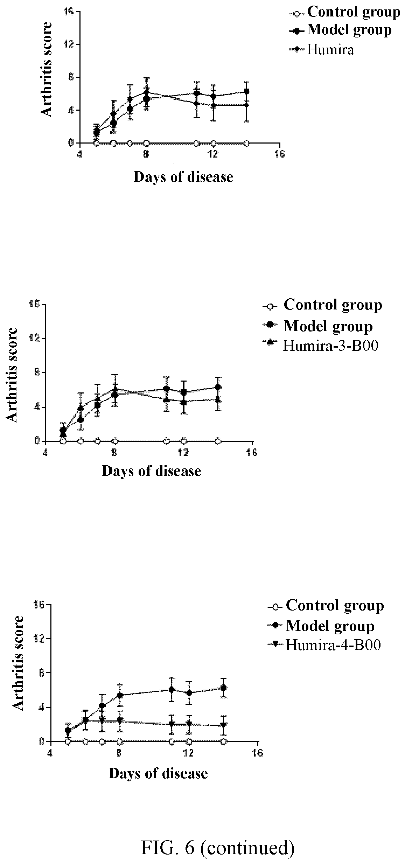

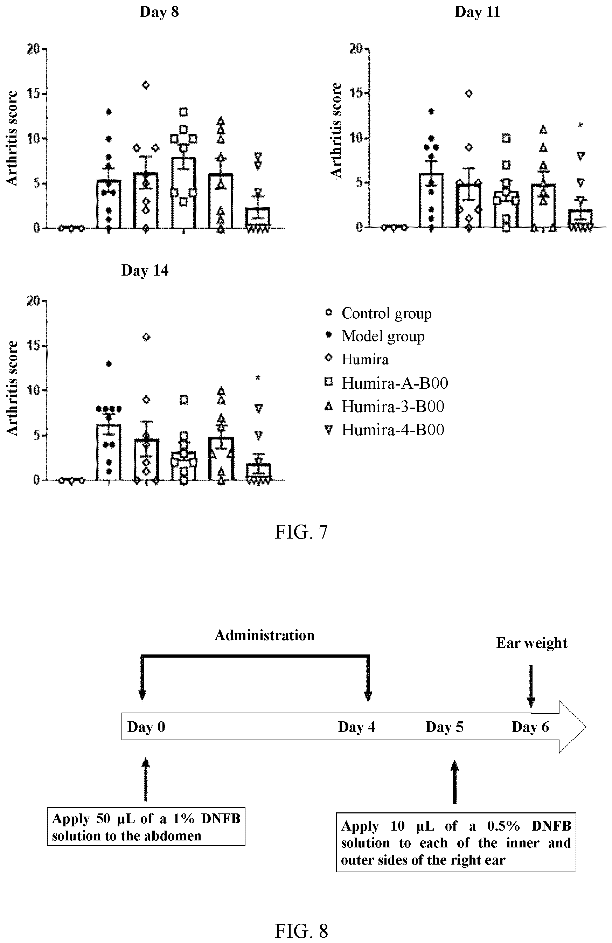

Humira has weak anti-inflammatory activity in the CAIA model. Humira-4-B00 has a rapid onset of action (began to take effect on day 5) and can cause a sustained reduction in arthritic inflammation. The control ADC molecule Humira-A-B00 showed an alleviating effect on arthritis only late in the course of arthritis (from day 11 onwards) (as shown in FIG. 6). From day 8 to day 14 after arthritis modeling, Humira-4-B00 significantly reduced the mouse arthritis score (*, p < 0.05) compared to the model group, whereas the control ADC molecule Humira-A-B00 showed no significant difference from the model group in anti-inflammatory activity (as shown in FIG. 7).

### Test Example 6: Delayed-Type Hypersensitivity (DTH) Model

### Test animals:

Male ICR mice, 6 w, purchased from the Laboratory Animal Management Department, Shanghai Institute of Planned Parenthood Research. Housing environment: SPF; production license SCXK (Shanghai) 2018-0006; certification number: 20180006023622.

### Test samples:

Humira-3-B00, Humira-4-B00, Humira-A-B00, and Humira-A-A00 (prepared according to Example 2 of WO2017210471 and Example 7 of WO2019106609).

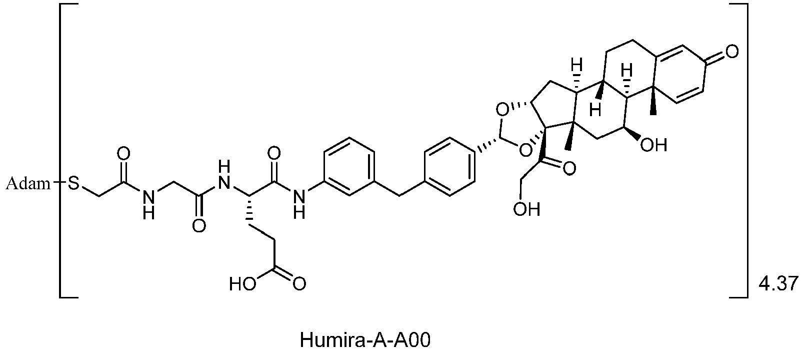

### Test method:

Upon arrival, the animals were acclimatized for 7 days and randomly grouped. On day 0, mice were immunized by applying 50 µL of a 1% solution of DNFB (2,4-dinitrofluorobenzene) to the abdomen where the hair had been removed. On day 5, 10 µL of a 0.5% solution of DNFB was applied to each of the inner and outer sides of the right ears of the mice as a booster (20 µL in total). On day 6 (after 24 h), the mice were sacrificed, and ear samples 8 mm in diameter were taken from both sides using a punch and weighed. Each group of mice was dosed on day 0 and day 4. The specific experimental process is shown in FIG. 8. Mice were dosed according to the following administration regimens. The ear samples from the control sides and the model sides of the mice were weighed, and the weights (indicating degrees of swelling) were used to evaluate the anti-inflammatory activity of the anti-TNF-ADCs.

**Table 4: Administration regimens**

| Group No. | Group | Number | Dose (mg/kg) | Route of administration | Frequency of administration |
|---|---|---|---|---|---|
| 1 | Model group | 8 | 10 | Intraperitoneally | Twice a week |
| 2 | Humira | 8 | 10 | Intraperitoneally | Twice a week |
| 3 | Humira-A-A00 | 8 | 10 | Intraperitoneally | Twice a week |
| 4 | Humira-A-B00 | 8 | 10 | Intraperitoneally | Twice a week |
| 5 | Humira-3-B00 | 8 | 10 | Intraperitoneally | Twice a week |
| 6 | Humira-4-B00 | 8 | 10 | Intraperitoneally | Twice a week |

### Test results:

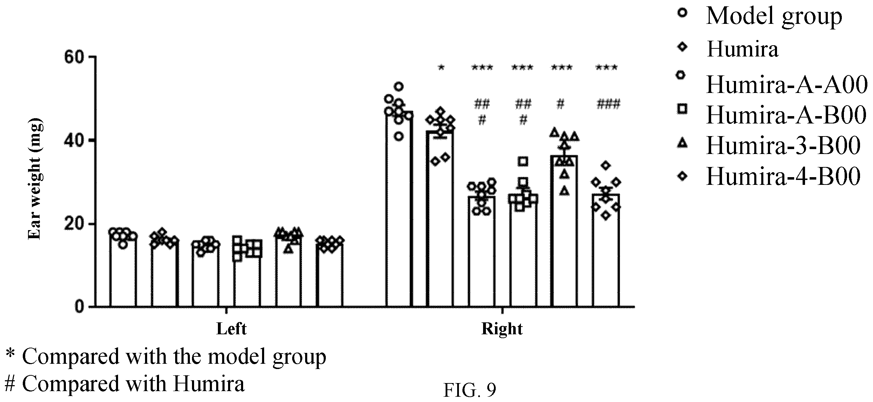

There was no difference in weight between the untreated left ears of these groups of mice. Humira-4-B00 showed comparable anti-inflammatory activity to the positive control Humira-A-B00 in the model. Humira-3-B00 showed relatively low anti-inflammatory activity but is still significantly better than Humira mAb (as shown in FIG. 9).

### Test Example 7: Stability of ADC Samples in Plasma

### Test samples:

Humira-3-B00, Humira-4-B00, Humira-A-B00, and Humira-A-A00.

### Test plasma:

Human, cynomolgus monkey, rat, mouse and 1% BSA

### Test method:

1 mg/mL solutions of test molecules were prepared in PBS and sterilized by filtration through a 0.22 µm filter membrane. 15 µL of a 1 mg/mL sample was added to 135 µL of a reaction medium to make a final concentration of 100 µg/mL. The mixture was incubated at 37 °C in a dark place for 0 days, 7 days, 14 days and 21 days, and samples were tested for free toxins.

The data are shown in Table 5.

**Table 5: Plasma stability data**

| **100 µg/mL, plasma, 37 °C** | | **Free toxins (%)** | | | | | | |
|---|---|---|---|---|---|---|---|---|
| | | **Humira-A-A00** | **Humira-A-B00** | | **Humira-4-B00** | | **Humira-3-B00** | |
| | | **Toxin compound A-A** | **Toxin compound A-A** | **Toxin compound A-B** | **Toxin compound 4-A** | **Toxin compound 4-B** | **Toxin compound 3-A** | **Toxin compound 3-B** |
| Human | D0 | 0.000 | 0.000 | 0.000 | 0.000 | 0.000 | 0.000 | 0.000 |
| | D7 | 0.000 | 0.000 | 0.000 | 0.000 | 0.000 | 0.000 | 0.000 |
| | D14 | 0.000 | 0.000 | 0.000 | 0.000 | 0.000 | 0.000 | 0.000 |
| | D21 | 0.000 | 0.000 | 0.000 | 0.000 | 0.000 | 0.000 | 0.000 |
| Cynomolgus monkey | D0 | 0.000 | 0.000 | 0.000 | 0.000 | 0.000 | 0.000 | 0.000 |
| | D7 | 0.000 | 0.000 | 0.000 | 0.000 | 0.000 | 0.000 | 0.000 |
| | D14 | 0.000 | 0.000 | 0.000 | 0.000 | 0.000 | 0.000 | 0.000 |
| | D21 | 0.000 | 0.000 | 0.000 | 0.000 | 0.000 | 0.000 | 0.000 |
| BSA (1%) | D0 | 0.000 | 0.000 | 0.000 | 0.000 | 0.000 | 0.000 | 0.000 |
| | D7 | 0.000 | 0.000 | 0.000 | 0.000 | 0.000 | 0.000 | 0.000 |
| | D14 | 0.000 | 0.000 | 0.000 | 0.000 | 0.000 | 0.000 | 0.000 |
| | D21 | 0.000 | 0.000 | 0.000 | 0.000 | 0.000 | 0.000 | 0.000 |
| Rat | D0 | 0.000 | 0.000 | 0.000 | 0.000 | 0.000 | 0.000 | 0.000 |
| | D7 | 0.000 | 0.000 | 0.000 | 0.000 | 0.000 | 0.000 | 0.000 |
| | D14 | 0.232 | 0.232 | 0.168 | 0.000 | 0.000 | 0.000 | 0.000 |
| | D21 | 0.000 | 0.000 | 0.000 | 0.000 | 0.000 | 0.000 | 0.000 |
| Mouse | D0 | 0.000 | 0.000 | 0.145 | 0.000 | 0.000 | 0.000 | 0.000 |
| | D7 | 0.000 | 0.000 | 0.540 | 0.000 | 0.000 | 0.000 | 0.000 |
| | D14 | 0.000 | 0.000 | 0.818 | 0.000 | 0.000 | 0.000 | 0.000 |
| | D21 | 0.000 | 0.000 | 0.975 | 0.000 | 0.000 | 0.000 | 0.000 |

In the concentration of 100 µg/mL, Humira-3-B00 and Humira-4-B00 showed excellent stability in human, cynomolgus monkey, mouse and rat plasma and 1% BSA, with free toxin levels below the lower limit of detection; Humira-A-A00 released a small amount of a free toxin in rat plasma; Humira-A-B00 released small amounts of free toxins in rat and mouse plasma.

## Claims

1. An antibody-drug conjugate of formula (I),
Ab-(L-D)ₖ (I)
wherein Ab is an antibody or an antigen-binding fragment thereof;
L is a linker covalently linking Ab to D, and k is 1 to 20;
D is represented by formula (II-A) or (II-B): 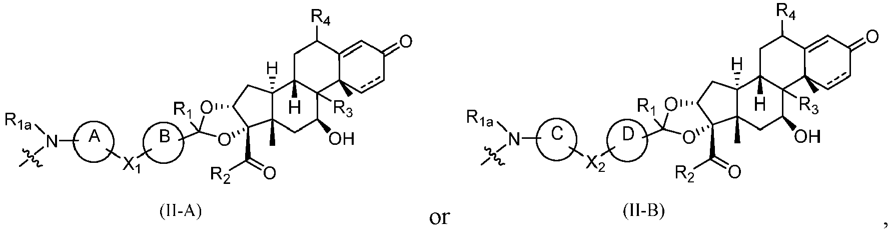 wherein,
-̅ -̅ -̅ represents a single bond or a double bond;
each R₁ₐ is independently selected from the group consisting of hydrogen, alkyl and alkoxy, and the alkyl and alkoxy are each independently optionally substituted with one or more substituents selected from the group consisting of alkyl, alkoxy, halogen, deuterium, amino, cyano, nitro, hydroxy and hydroxyalkyl; preferably, each R₁ₐ is independently selected from the group consisting of hydrogen, C₁-C₆ alkyl and C₁-C₆ alkoxy;
ring A is aryl or heteroaryl optionally substituted with one or more Qi substituents;
ring B is aryl or heteroaryl optionally substituted with one or more Qi substituents;
X₁ is -(CR₅ₐR_{5b})m- or is aryl or heteroaryl optionally substituted with one or more Qi substituents;
R₅ₐ and R_{5b} are each independently selected from the group consisting of hydrogen, halogen, hydroxy, sulfhydryl, deuterium, nitro, cyano, and the following groups optionally substituted with one or more Qi substituents: alkyl, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, alkoxy, alkylthio, alkenyl and alkynyl, or together R₅ₐ and R_{5b} form oxo or thio;
ring C and ring D are each independently selected from the group consisting of aryl and heteroaryl optionally substituted with one or more Qi substituents, and at least one of ring C and ring D is fused cycloaryl or fused heteroaryl optionally substituted with one or more Qi substituents;
X₂ is selected from the group consisting of -(CR₆ₐR_{6b})n-, aryl or heteroaryl optionally substituted with one or more Qi substituents, -O-, -S-, -S(O)-, -S(O)(O)-, -NR_{6c}-, -CH₂S-, -CHzO-, -NHCR_{6d}R₆ₑ-, -CR_{6f}=CR_{6g}- and -C≡C-, or X₂ is absent;
R₆ₐ and R_{6b} are each independently selected from the group consisting of hydrogen, halogen, hydroxy, sulfhydryl, deuterium, nitro, cyano, and the following groups optionally substituted with one or more Qi substituents: alkyl, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, alkoxy, alkylthio, alkenyl and alkynyl, or R₆ₐ and R_{6b}, together with the carbon atom to which they are attached, form 3- to 10-membered cycloalkyl, or together R₆ₐ and R_{6b} form oxo or thio;
R_{6c}, R_{6d}, R₆ₑ, R_{6f} and R_{6g} are each independently selected from the group consisting of hydrogen, C₁-C₆ alkyl, C₁-C₆ haloalkyl and C₁-C₆ alkoxy;
each R₁ is independently selected from the group consisting of hydrogen, alkyl and alkoxy, wherein the alkyl and alkoxy are each independently optionally substituted with one or more substituents selected from the group consisting of alkyl, alkoxy, halogen, deuterium, amino, cyano, nitro, hydroxy and hydroxyalkyl; preferably, each R₁ is independently selected from the group consisting of hydrogen, C₁-C₆ alkyl and C₁-C₆ alkoxy; more preferably, each R₁ is independently hydrogen;
each R₂ is independently selected from the group consisting of -CH₂OH, -CH₂SH, -CH₂Cl, -SCH₂Cl, -SCH₂F, -SCH₂CF₃, -OH, -OCH₂CN, -OCH₂Cl, -OCH₂F, -OCH₃, -OCH₂CH₃, -SCH₂CN, 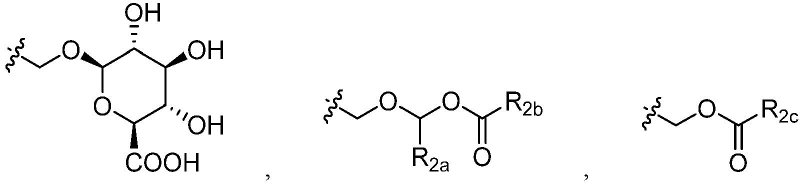 and 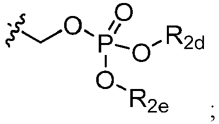
each R₂ₐ is independently hydrogen or C₁-C₆ alkyl;
each R_{2b} is independently C₁-C₆ alkyl or C₁-C₆ alkoxy;
each R_{2c} is independently selected from the group consisting of hydrogen, C₁-C₆ alkyl, -CH₂OH and C₁-C₆ alkoxy;
R_{2d} and R₂ₑ are each independently hydrogen or C₁-C₆ alkyl;
each R₃ is independently hydrogen or a halogen;
each R₄ is independently selected from the group consisting of hydrogen, halogen and hydroxy; preferably, each R₄ is independently hydrogen;
m and n are each independently an integer of 1 to 6;
the Qi substituents are each independently selected from the group consisting of C₁-C₆ alkyl, halogen, deuterium, hydroxy, sulfhydryl, -NRᵢRⱼ, oxo, thio, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthio, C₂-C₆ alkenyl, C₂-C₆ alkynyl, 3- to 10-membered cycloalkyl, 3- to 10-membered heterocyclyl, 6- to 10-membered aryl, 5- to 10-membered heteroaryl, 8-to 12-membered fused cycloaryl, and 5- to 12-membered fused heteroaryl; preferably, each Qi is independently selected from the group consisting of halogen, hydroxy, sulfhydryl, deuterium, oxo, thio, cyano, amino, carboxyl, C₁-C₆ alkyl and C₁-C₆ alkoxy;
Rᵢ and Rⱼ are each independently selected from the group consisting of hydrogen, hydroxy, C₁-C₆ alkyl and C₁-C₆ alkoxy;
each Rₖ is independently selected from the group consisting of hydrogen, C₁-C₆ alkyl, C₁-C₆ haloalkyl, C₁-C₆ alkoxy, hydroxy and -NRᵢRⱼ, wherein the alkyl, alkoxy and haloalkyl are each independently optionally substituted with one or more substituents selected from the group consisting of C₁-C₆ alkyl, halogen, hydroxy, sulfhydryl, -NRᵢRⱼ, oxo, thio, carboxyl, nitro, cyano, C₁-C₆ alkoxy, C₁-C₆ alkylthio, C₂-C₆ alkenyl, C₂-C₆ alkynyl, 3- to 10-membered cycloalkyl, 3- to 10-membered heterocyclyl, 6- to 10-membered aryl, and 5- to 10-membered heteroaryl; preferably, each Rₖ is independently selected from the group consisting of hydrogen, C₁-C₆ alkyl, C₁-C₆ haloalkyl, C₁-C₆ alkoxy, hydroxy and -NRᵢRⱼ;
provided that when R₅ₐ is hydrogen or alkyl, R_{5b} is not hydrogen or alkyl.

2. The antibody-drug conjugate according to claim 1, wherein ring A is 6- to 10-membered aryl or 5- to 10-membered heteroaryl optionally substituted with one or more Qi substituents, and the heteroaryl comprises at least one nitrogen atom; preferably, ring A is 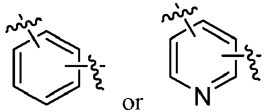 optionally substituted with one or more Qi substituents.

3. The antibody-drug conjugate according to claim 1 or 2, wherein ring B is 6- to 10-membered aryl or 5- to 10-membered heteroaryl optionally substituted with one or more Qi substituents, and the heteroaryl comprises at least one nitrogen atom; preferably, ring B is 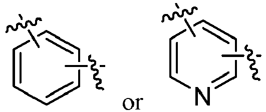 optionally substituted with one or more Qi substituents.

4. The antibody-drug conjugate according to any one of claims 1-3, wherein X₁ is -(CR₅ₐR_{5b})m- or is 6- to 10-membered aryl or 5- to 10-membered heteroaryl optionally substituted with one or more Qi substituents, and the heteroaryl comprises at least one nitrogen atom; preferably, R₅ₐ and R_{5b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ, -S(O)Rₖ, -S(O)ORₖ, -S(O)(O)Rₖ, -S(O)(O)ORₖ, C₁-C₆ alkoxy, C₁-C₆ alkylthio, C₂-C₆ alkenyl and C₂-C₆ alkynyl; more preferably, R₅ₐ and R_{5b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ, -S(O)Rₖ, -S(O)(O)Rₖ, C₁-C₆ alkoxy, C₂-C₆ alkenyl and C₂-C₆ alkynyl; most preferably, R₅ₐ and R_{5b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy, or together R₅ₐ and R_{5b} form oxo or thio.

5. The antibody-drug conjugate according to any one of claims 1-4, wherein ring C and ring D are each independently selected from the group consisting of 6- to 10-membered aryl, 5- to 10-membered heteroaryl, 8- to 12-membered fused cycloaryl and 5- to 12-membered fused heteroaryl optionally substituted with one or more Qi substituents, and the heteroaryl or fused heteroaryl comprises at least one nitrogen atom; preferably, ring C and ring D are each independently selected from the group consisting of the following groups optionally substituted with one or more Qi substituents: 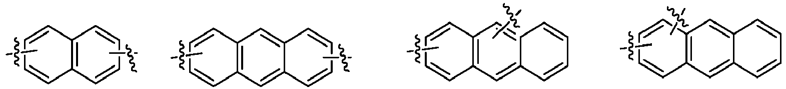 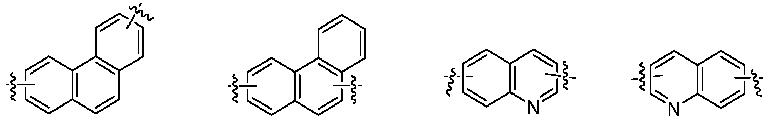 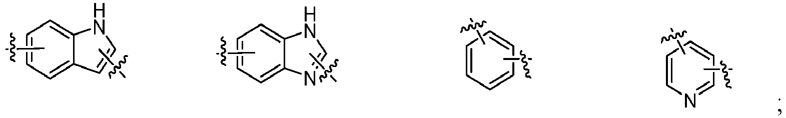 more preferably, ring C is selected from the group consisting of: 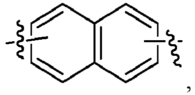 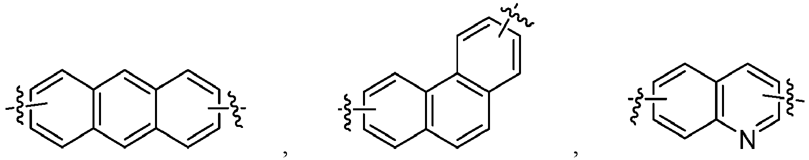 and 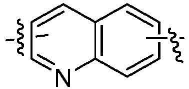 optionally substituted with one or more Qi substituents, and ring D is 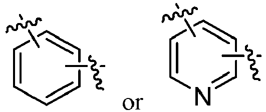 optionally substituted with one or more Qi substituents.

6. The antibody-drug conjugate according to any one of claims 1-5, wherein X₂ is selected from the group consisting of -(CR₆ₐR_{6b})n-, -O-, -S-, -NR_{6c}-,-CH₂S-, -CH₂O-, -NHCR_{6d}R₆ₑ-, and 6- to 10-membered aryl or 5- to 10-membered heteroaryl optionally substituted with one or more Qi substituents, and the heteroaryl comprises at least one nitrogen atom; preferably, R₆ₐ and R_{6b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ, -S(O)Rₖ, -S(O)(O)Rₖ, C₁-C₆ alkoxy, C₂-C₆ alkenyl and C₂-C₆ alkynyl, or R₆ₐ and R_{6b}, together with the carbon atom to which they are attached, form 3- to 10-membered cycloalkyl; more preferably, R₆ₐ and R_{6b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy.

7. The antibody-drug conjugate according to claim 1, wherein D is represented by formula (II-A') or (II-B'): 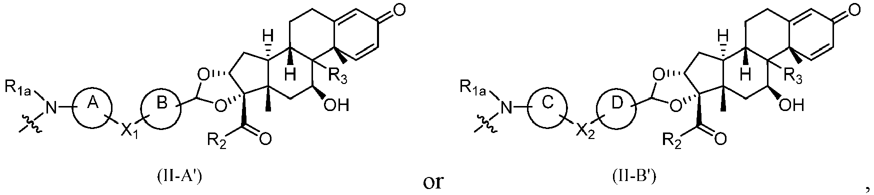 wherein,
each R₁ₐ is independently selected from the group consisting of hydrogen, C₁-C₆ alkyl and C₁-C₆ alkoxy, preferably hydrogen;
ring A is 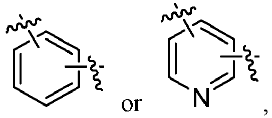 and the ring A is optionally substituted with one or more Qi substituents;
ring B is 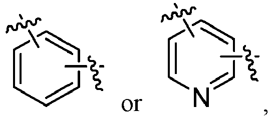 and the ring B is optionally substituted with one or more Qi substituents;
X₁ is -(CR₅ₐR_{5b})m- or is 6- to 10-membered aryl or 5- to 10-membered heteroaryl optionally substituted with one or more Qi substituents, and the heteroaryl comprises at least one nitrogen atom;
R₅ₐ and R_{5b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy, or together R₅ₐ and R_{5b} form oxo or thio;
ring C is selected from the group consisting of 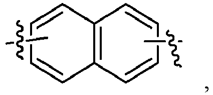 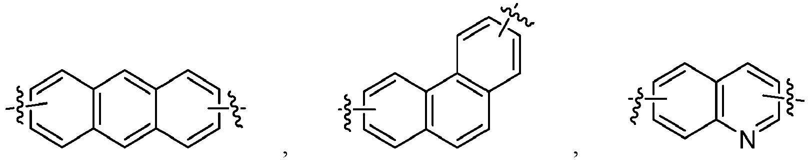 and 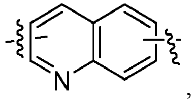 and the ring C is optionally substituted with one or more Qi substituents;
ring D is 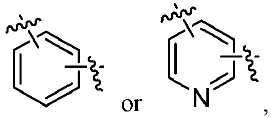 and the ring D is optionally substituted with one or more Qi substituents;
X₂ is selected from the group consisting of -(CR₆ₐR_{6b})n-, -O-, -S-, -NR_{6c}-,-CH₂S-, -CH₂O-, -NHCR_{6d}R₆ₑ-, and 6- to 10-membered aryl or 5- to 10-membered heteroaryl optionally substituted with one or more Qi substituents;
R₆ₐ and R_{6b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy;
R_{6c}, R_{6d} and R₆ₑ are each independently selected from the group consisting of hydrogen, C₁-C₆ alkyl, C₁-C₆ haloalkyl and C₁-C₆ alkoxy;
each R₂ is independently selected from the group consisting of -CH₂OH, -CH₂SH, -CH₂Cl, -SCH₂Cl, -SCH₂F, -SCH₂CF₃, -OH, -OCH₂CN, -OCH₂Cl, -OCH₂F, -OCH₃, -OCH₂CH₃, -SCH₂CN, 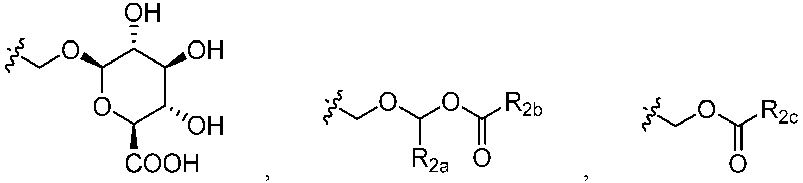 and 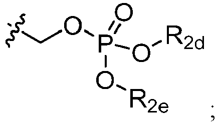
each R₂ₐ is independently hydrogen or C₁-C₆ alkyl;
each R_{2b} is independently C₁-C₆ alkyl or C₁-C₆ alkoxy;
each R_{2c} is independently selected from the group consisting of hydrogen, C₁-C₆ alkyl, -CH₂OH and C₁-C₆ alkoxy;
R_{2d} and R₂ₑ are each independently hydrogen or C₁-C₆ alkyl;
preferably, each R₂ is independently selected from the group consisting of -CH₂OH, -CH₂SH, -OH and 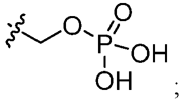
each R₃ is independently hydrogen or a halogen; preferably, each R₃ is independently hydrogen or fluorine;
m and n are each independently an integer of 1 to 6;
the Qi substituents are each independently selected from the group consisting of halogen, hydroxy, sulfhydryl, deuterium, oxo, thio, cyano, amino, carboxyl, C₁-C₆ alkyl and C₁-C₆ alkoxy;
Rᵢ and Rⱼ are each independently selected from the group consisting of hydrogen, hydroxy, C₁-C₆ alkyl and C₁-C₆ alkoxy;
Rₖ is independently selected from the group consisting of hydrogen, C₁-C₆ alkyl, C₁-C₆ haloalkyl, C₁-C₆ alkoxy, hydroxy and -NRᵢRⱼ;
provided that when R₅ₐ is hydrogen or alkyl, R_{5b} is not hydrogen or alkyl.

8. The antibody-drug conjugate according to any one of claims 1-7, wherein X₁ is -(CR₅ₐR_{5b})m-; R₅ₐ and R_{5b} are both fluorine; m is 1, 2 or 3, preferably 1; or
X₁ is -(CR₅ₐR_{5b})m-; together R₅ₐ and R_{5b} form oxo or thio, preferably oxo; m is 1, 2 or 3, preferably 1 or
X₁ is selected from the group consisting of 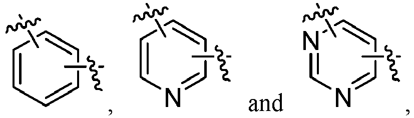 preferably 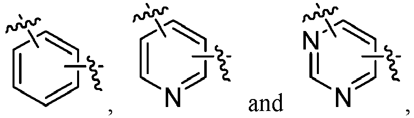 optionally substituted with one or more Qi substituents.

9. The antibody-drug conjugate according to any one of claims 1-8, wherein X₂ is selected from the group consisting of -(CR₆ₐR_{6b})n-, and 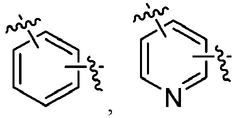 and 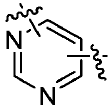 optionally substituted with one or more Qi substituents; preferably, R₆ₐ and R_{6b} are each independently selected from the group consisting of hydrogen, halogen, hydroxy, sulfhydryl, deuterium, cyano, C₁-C₆ alkyl, -NRᵢRⱼ, -C(O)ORₖ and C₁-C₆ alkoxy.

10. The antibody-drug conjugate according to any one of claims 1-9, wherein k is 1-10, preferably 2-5.

11. The antibody-drug conjugate according to any one of claims 1-10, wherein the linker comprises amino acid unit Li, and the amino acid unit Li preferably comprises a peptide residue consisting of 2 to 7 amino acids selected from the group consisting of phenylalanine, glycine, valine, lysine, citrulline, serine, glutamic acid, aspartic acid, homolysine, n-methyl-valine and 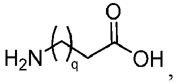 wherein q is an integer of 1-6; more preferably valine-citrulline (Val-Cit), alanine-phenylalanine (Ala-Phe),phenylalanine-lysine (Phe-Lys), phenylalanine-homolysine (Phe-Homolys), n-methyl-valine-citrulline (Me-Val-Cit), alanine-alanine (Ala-Ala), glycine-glutamic acid (Gly-Glu), glutamic acid-alanine-alanine (Glu-Ala-Ala) and glycine-lysine (Gly-Lys), glycine-valine-citrulline (Gly-Val-Cit), glycine-glycine-glycine (Gly-Gly-Gly) and 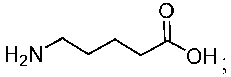 and most preferably glycine-glutamic acid (Gly-Glu) and

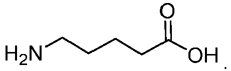

12. The antibody-drug conjugate according to any one of claims 1-11, wherein the linker comprises a stretcher unit; preferably, the stretcher unit is selected from the group consisting of 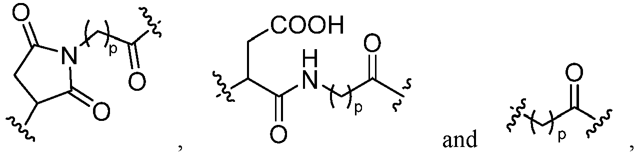 wherein each p is independently 1, 2, 3, 4, 5 or 6.

13. The antibody-drug conjugate according to any one of claims 1-12, wherein the linker is selected from the group consisting of 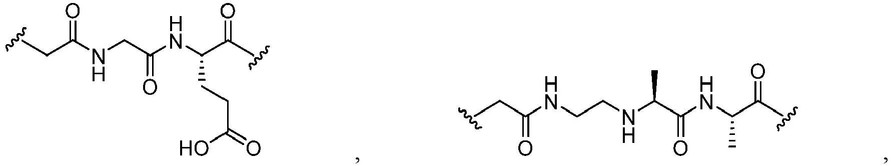 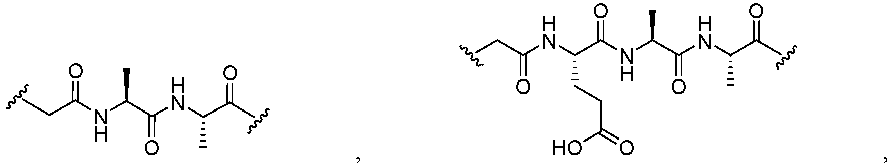 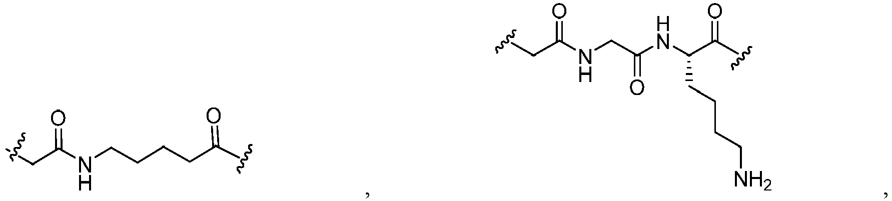 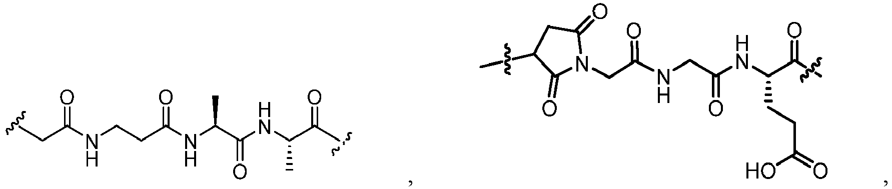 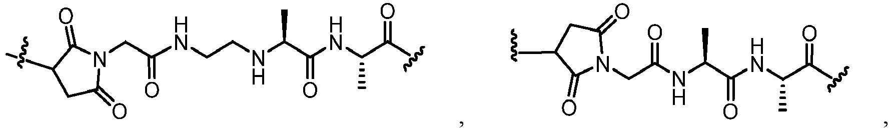 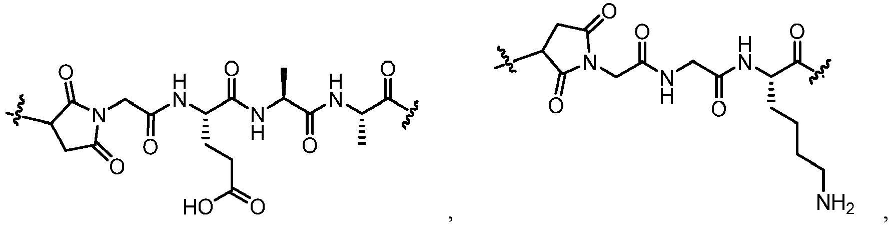 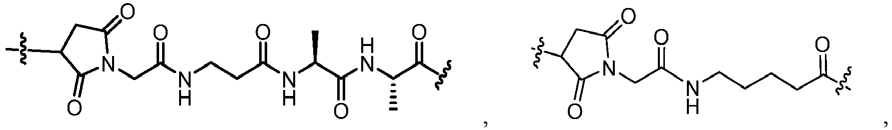 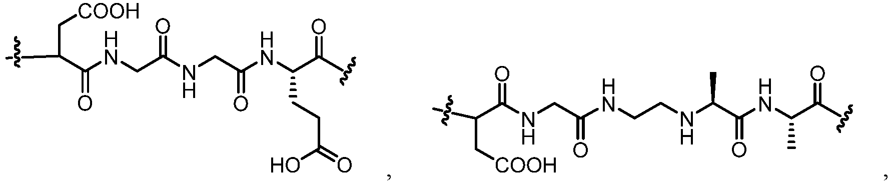 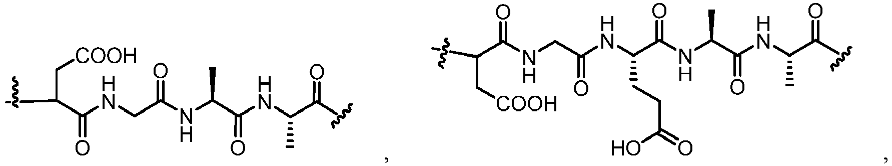 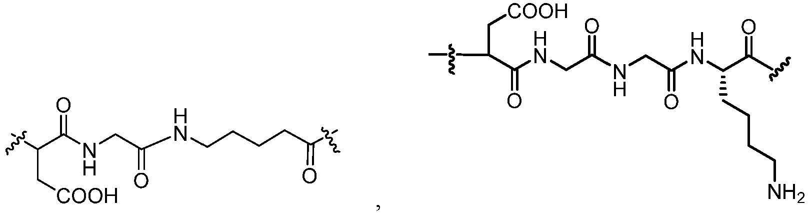 and

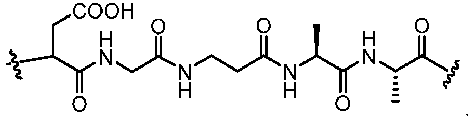

14. The antibody-drug conjugate according to any one of claims 1-13, being selected from the group consisting of: 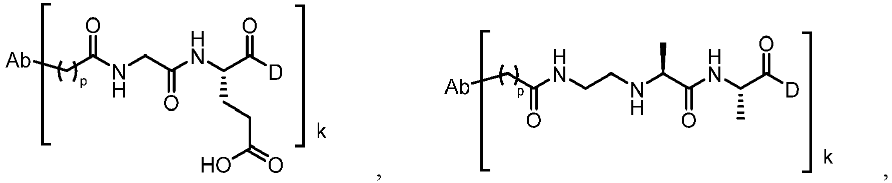 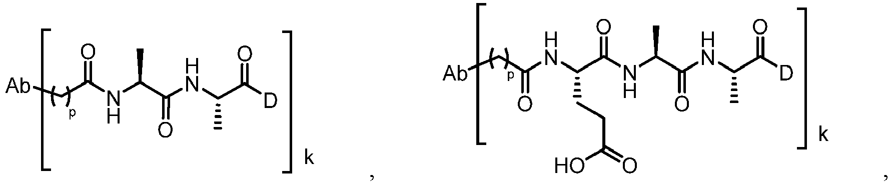 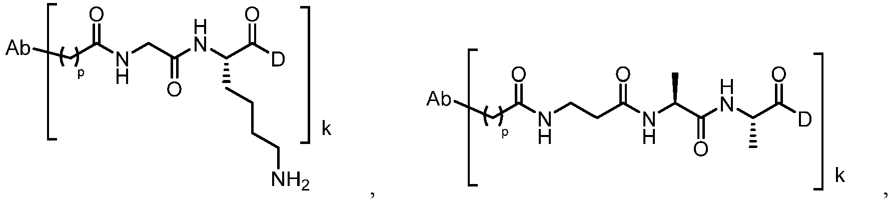 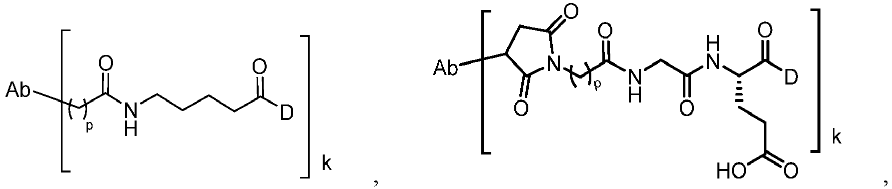 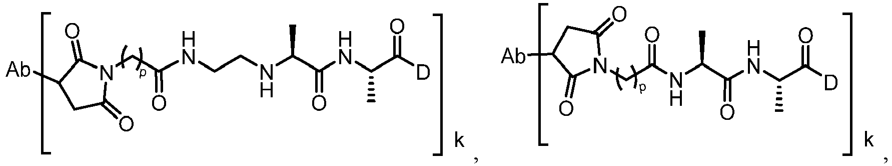 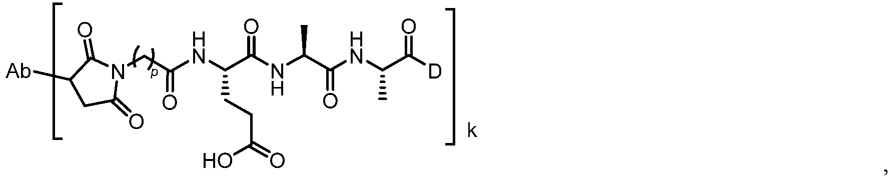 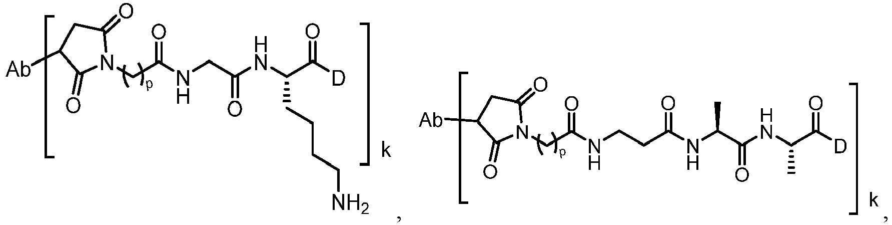 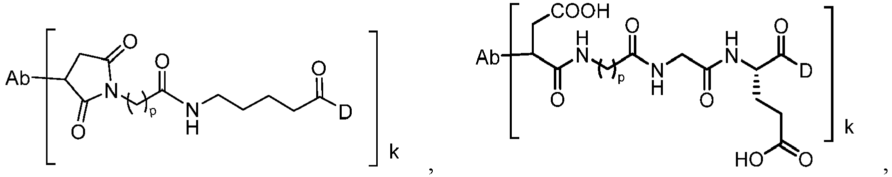 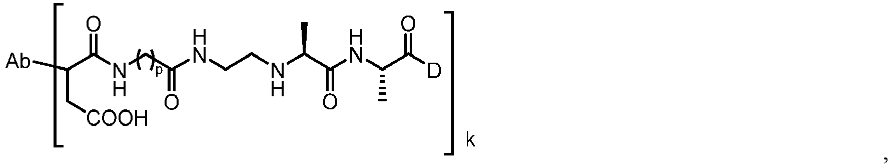 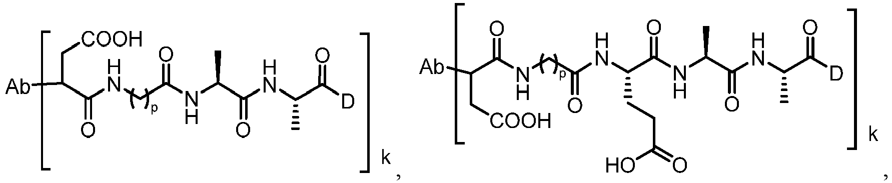 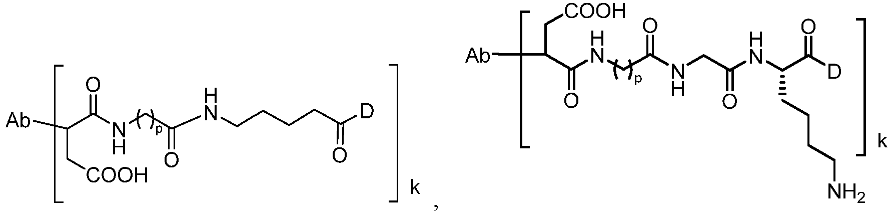 and 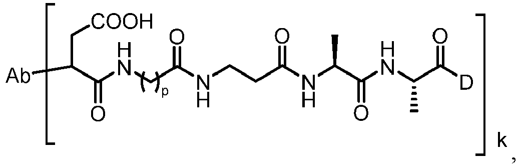 wherein each p is independently 1, 2, 3, 4, 5 or 6; Ab, D and k are as defined in claim 1.

15. The antibody-drug conjugate according to claim 1, being selected from the group consisting of 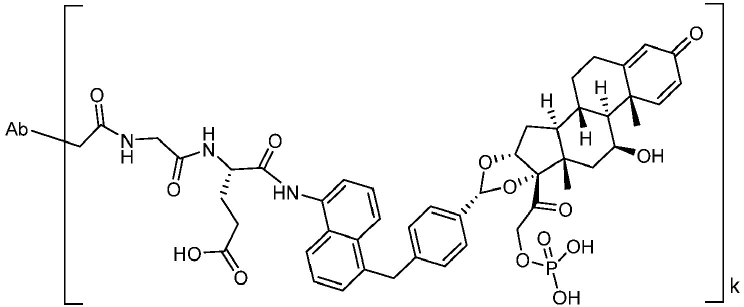 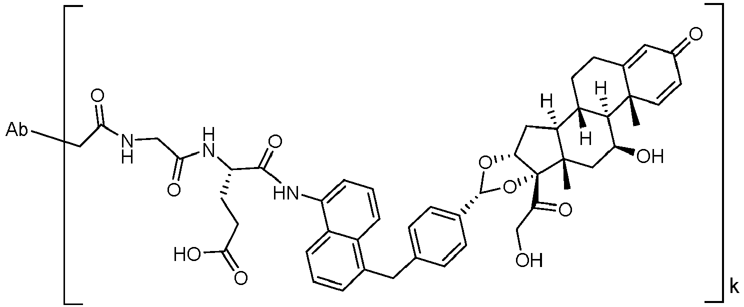 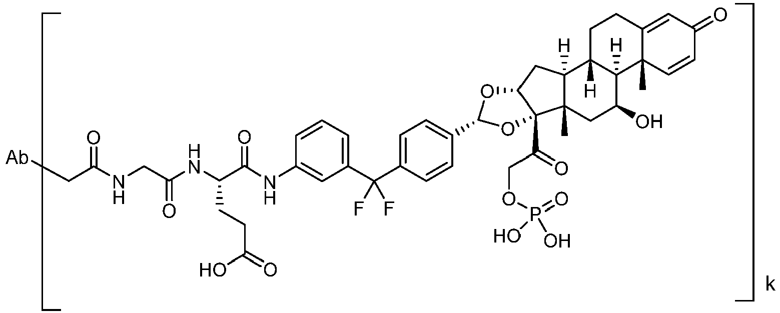 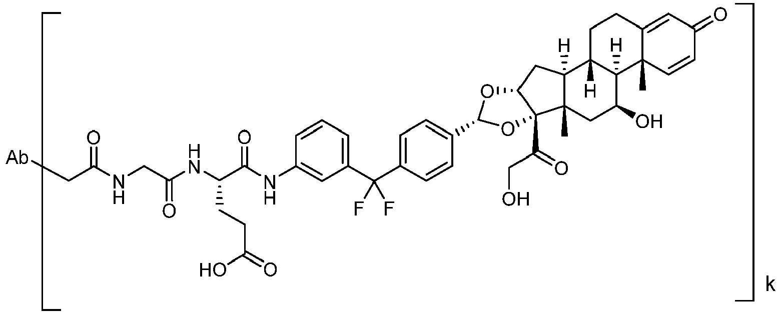 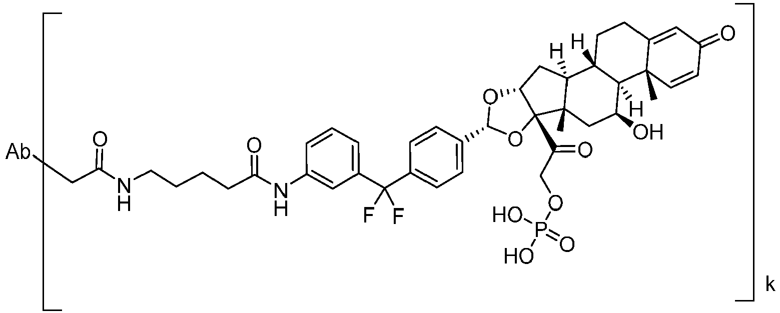 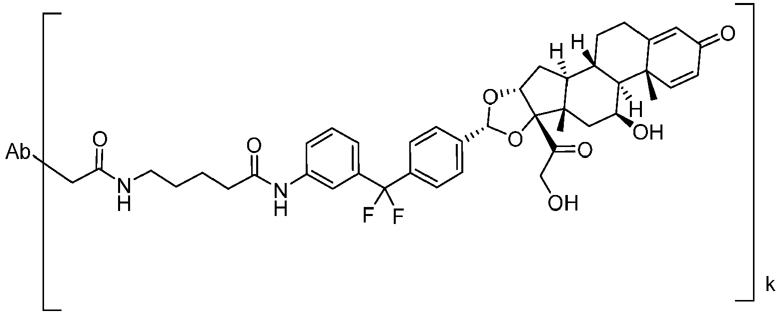 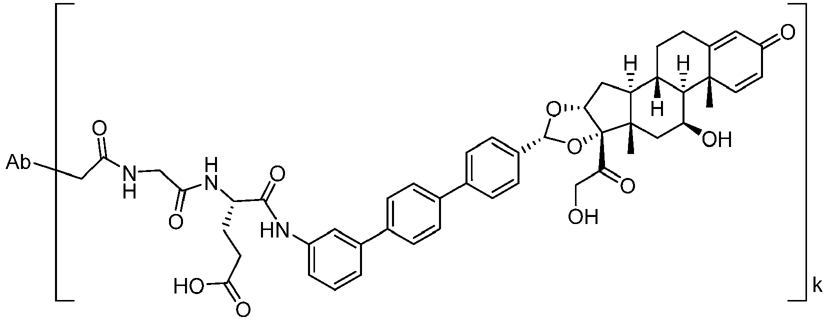 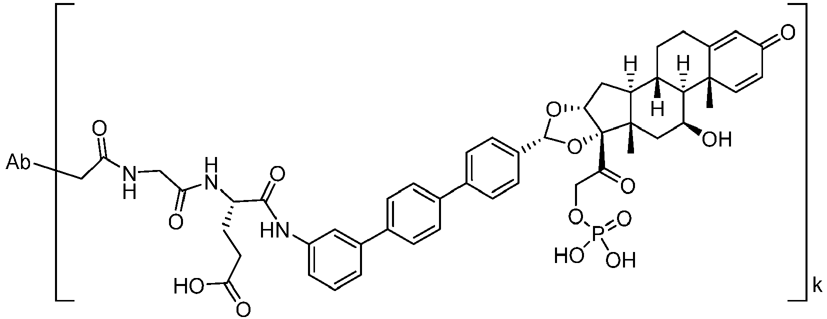 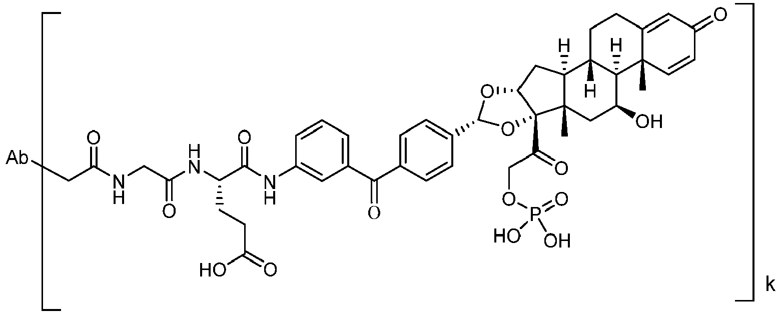 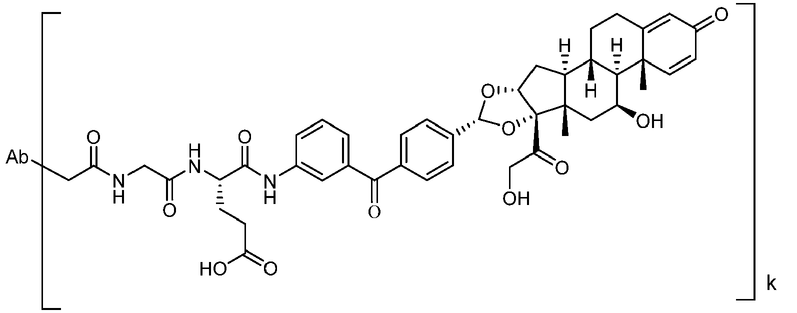 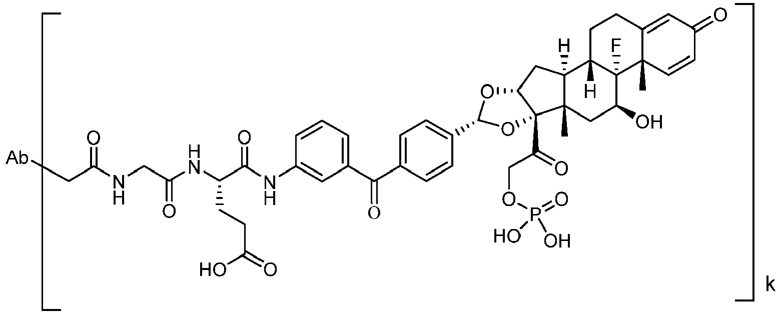 and 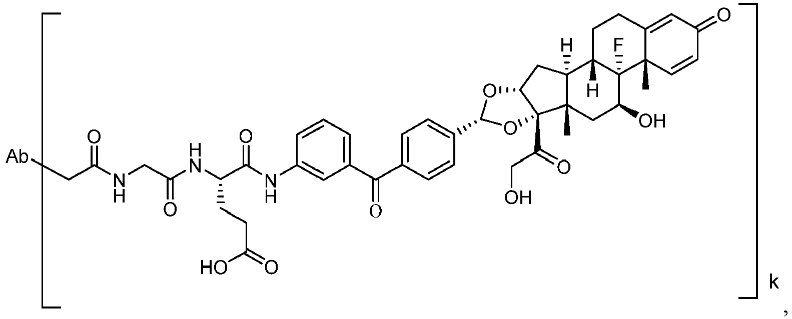 wherein k is 1 to 10; Ab is as defined in claim 1.

16. The antibody-drug conjugate according to any one of claims 1-15, wherein the antibody is selected from the group consisting of a murine antibody, a chimeric antibody, a humanized antibody and a fully human-derived antibody.

17. The antibody-drug conjugate according to any one of claims 1-16, wherein the antibody or the antigen-binding fragment thereof is selected from the group consisting of an anti-TNFα antibody, an anti-IL-4R antibody, an anti-IL-6/IL-6R antibody, an anti-IL-13R antibody, an anti-IL-17/IL-17R antibody, an anti-IL-23/IL23R antibody, an anti-IL-36R antibody, an anti-CD20 antibody, an anti-CD22 antibody, an anti-CD28 antibody, an anti-CD40 antibody and an anti-TSLP antibody or antigen-binding fragments thereof; preferably adalimumab, infliximab, certolizumab pegol, afelimomab, nerelimomab, ozoralizumab, placulumab and golimumab or antigen-binding fragments thereof; and more preferably adalimumab.

18. A compound of formula (III-A) or (III-B) or a pharmaceutically acceptable salt thereof, 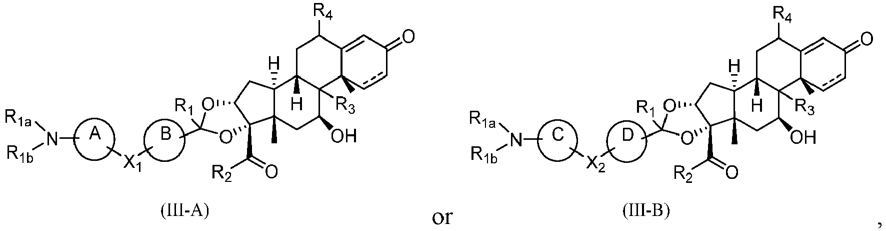 wherein,
-̅ -̅ -̅ represents a single bond or a double bond;
ring A is aryl or heteroaryl optionally substituted with one or more Qi substituents;
ring B is aryl or heteroaryl optionally substituted with one or more Qi substituents;
X₁ is -(CR₅ₐR_{5b})m- or is aryl or heteroaryl optionally substituted with one or more Qi substituents;
R₅ₐ and R_{5b} are each independently selected from the group consisting of hydrogen, halogen, hydroxy, sulfhydryl, deuterium, nitro, cyano, and the following groups optionally substituted with one or more Qi substituents: alkyl, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, alkoxy, alkylthio, alkenyl and alkynyl, or together R₅ₐ and R_{5b} form oxo or thio;
ring C and ring D are each independently selected from the group consisting of aryl and heteroaryl or fused heteroaryl optionally substituted with one or more Qi substituents, and at least one of ring C and ring D is fused cycloaryl or fused heteroaryl optionally substituted with one or more Qi substituents;
X₂ is selected from the group consisting of -(CR₆ₐR_{6b})n-, aryl or heteroaryl optionally substituted with one or more Qi substituents, -O-, -S-, -S(O)-, -S(O)(O)-, -NR_{6c}-, -CH₂S-, -CHzO-, -NHCR_{6d}R₆ₑ-, -CR_{6f}=CR_{6g}- and -C≡C-, or X₂ is absent;
R₆ₐ and R_{6b} are each independently selected from the group consisting of hydrogen, halogen, hydroxy, sulfhydryl, deuterium, nitro, cyano, and the following groups optionally substituted with one or more Qi substituents: alkyl, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, alkoxy, alkylthio, alkenyl and alkynyl, or R₆ₐ and R_{6b}, together with the carbon atom to which they are attached, form 3- to 10-membered cycloalkyl;
R_{6c}, R_{6d}, R₆ₑ, R_{6f} and R_{6g} are each independently selected from the group consisting of hydrogen, C₁-C₆ alkyl, C₁-C₆ haloalkyl and C₁-C₆ alkoxy;
each R₁ is independently selected from the group consisting of hydrogen, alkyl and alkoxy, wherein the alkyl and alkoxy are each independently optionally substituted with one or more substituents selected from the group consisting of alkyl, alkoxy, halogen, deuterium, amino, cyano, nitro, hydroxy and hydroxyalkyl;
each R₂ is independently selected from the group consisting of -CH₂OH, -CH₂SH, -CH₂Cl, -SCH₂Cl, -SCH₂F, -SCH₂CF₃, -OH, -OCH₂CN, -OCH₂Cl, -OCH₂F, -OCH₃, -OCH₂CH₃, -SCH₂CN, 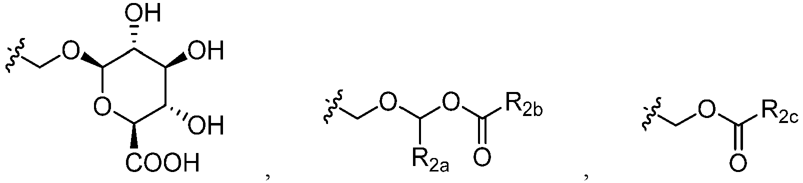 and 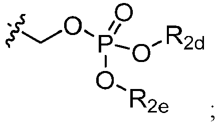
each R₂ₐ is independently hydrogen or C₁-C₆ alkyl;
each R_{2b} is independently C₁-C₆ alkyl or C₁-C₆ alkoxy;
each R_{2c} is independently selected from the group consisting of hydrogen, C₁-C₆ alkyl, -CH₂OH and C₁-C₆ alkoxy;
R_{2d} and R₂ₑ are each independently hydrogen or C₁-C₆ alkyl;
each R₃ is independently hydrogen or a halogen;
each R₄ is independently selected from the group consisting of hydrogen, halogen and hydroxy;
m and n are each independently selected from an integer of 1 to 6;
the Qi substituents are each independently selected from the group consisting of C₁-C₆ alkyl, halogen, deuterium, hydroxy, sulfhydryl, -NRᵢRⱼ, oxo, thio, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthio, C₂-C₆ alkenyl, C₂-C₆ alkynyl, 3- to 10-membered cycloalkyl, 3- to 10-membered heterocyclyl, 6- to 10-membered aryl, 5- to 10-membered heteroaryl, 8-to 12-membered fused cycloaryl, and 5- to 12-membered fused heteroaryl;
Rᵢ and Rⱼ are each independently selected from the group consisting of hydrogen, hydroxy, C₁-C₆ alkyl and C₁-C₆ alkoxy;
Rₖ is independently selected from the group consisting of hydrogen, C₁-C₆ alkyl, C₁-C₆ haloalkyl, C₁-C₆ alkoxy, hydroxy and -NRᵢRⱼ, wherein the alkyl, alkoxy and haloalkyl are each independently optionally substituted with one or more substituents selected from the group consisting of C₁-C₆ alkyl, halogen, hydroxy, sulfhydryl, -NRᵢRⱼ, oxo, thio, carboxyl, nitro, cyano, C₁-C₆ alkoxy, C₁-C₆ alkylthio, C₂-C₆ alkenyl, C₂-C₆ alkynyl, 3- to 10-membered cycloalkyl, 3- to 10-membered heterocyclyl, 6- to 10-membered aryl, and 5- to 10-membered heteroaryl;
each R₁ₐ is independently selected from the group consisting of hydrogen, alkyl and alkoxy, and the alkyl and alkoxy are each independently optionally substituted with one or more substituents selected from the group consisting of alkyl, alkoxy, halogen, deuterium, amino, cyano, nitro, hydroxy and hydroxyalkyl;
each R_{1b} is independently selected from the group consisting of hydrogen, PG-, H-Li-, PG-Li-, 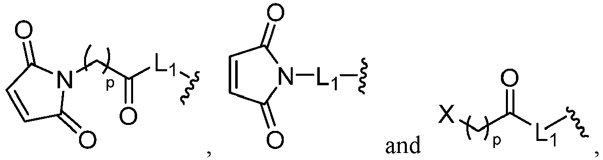 or
R₁ₐ and R_{1b}, together with the nitrogen atom to which they are attached, form: 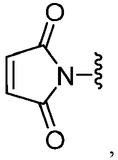 or R₁ₐ and R_{1b}, together with the nitrogen atom to which they are attached, form a nitro group;
each p is independently 1, 2, 3, 4, 5 or 6;
Li is an amino acid unit, preferably -glycine-glutamic acid- or 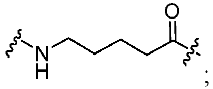
X is a halogen;
PG is an amino protecting group;
provided that when R₅ₐ is hydrogen or alkyl, R_{5b} is not hydrogen or alkyl.

19. The compound or the pharmaceutically acceptable salt thereof according to claim 18, being a compound of formula (III-A') or (III-B') or a pharmaceutically acceptable salt thereof: 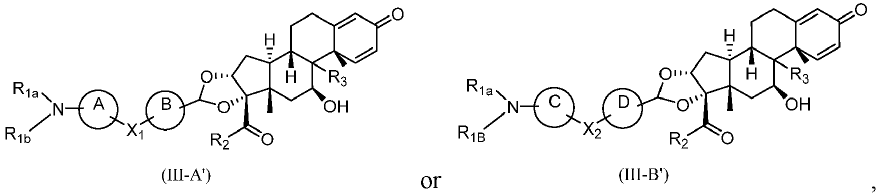 wherein,
ring A is 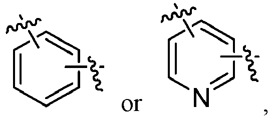 and the ring A is optionally substituted with one or more Qi substituents;
ring B is 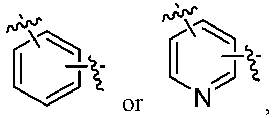 and the ring B is optionally substituted with one or more Qi substituents;
X₁ is -(CR₅ₐR_{5b})m- or is 6- to 10-membered aryl or 5- to 10-membered heteroaryl optionally substituted with one or more Qi substituents, and the heteroaryl comprises at least one nitrogen atom;
R₅ₐ and R_{5b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy, or together R₅ₐ and R_{5b} form oxo or thio;
ring C is selected from the group consisting of 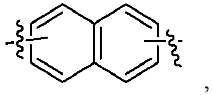 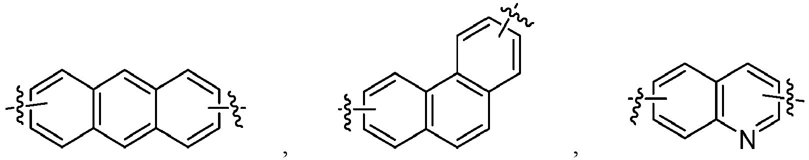 and 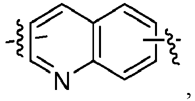 and the ring C is optionally substituted with one or more Qi substituents;
ring D is 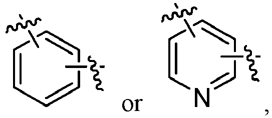 and the ring D is optionally substituted with one or more Qi substituents;
X₂ is selected from the group consisting of -(CR₆ₐR_{6b})n-, -O-, -S-, -NR_{6c}-,-CH₂S-, -CH₂O-, -NHCR_{6d}R₆ₑ-, and 6- to 10-membered aryl or 5- to 10-membered heteroaryl optionally substituted with one or more Qi substituents;
R₆ₐ and R_{6b} are each independently selected from the group consisting of hydrogen, halogen, hydroxy, sulfhydryl, deuterium, cyano, and the following groups optionally substituted with one or more Qi substituents: C₁-C₆ alkyl, -NRᵢRⱼ, -C(O)Rₖ, -C(O)ORₖ and C₁-C₆ alkoxy;
R_{6c}, R_{6d} and R₆ₑ are each independently selected from the group consisting of hydrogen, C₁-C₆ alkyl, C₁-C₆ haloalkyl and C₁-C₆ alkoxy;
each R₂ is independently selected from the group consisting of -CH₂OH, -CH₂SH, -CH₂Cl, -SCH₂Cl, -SCH₂F, -SCH₂CF₃, -OH, -OCH₂CN, -OCH₂Cl, -OCH₂F, -OCH₃, -OCH₂CH₃, -SCH₂CN, 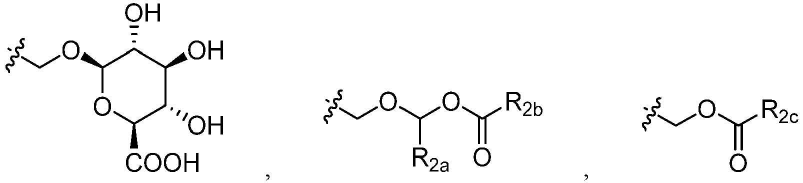 and 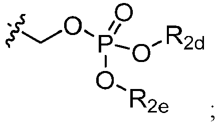
each R₂ₐ is independently hydrogen or C₁-C₆ alkyl;
each R_{2b} is independently C₁-C₆ alkyl or C₁-C₆ alkoxy;
each R_{2c} is independently selected from the group consisting of hydrogen, C₁-C₆ alkyl, -CH₂OH and C₁-C₆ alkoxy;
R_{2d} and R₂ₑ are each independently hydrogen or C₁-C₆ alkyl;
each R₃ is independently hydrogen or a halogen;
m and n are each independently an integer of 1 to 6;
the Qi substituents are each independently selected from the group consisting of halogen, hydroxy, sulfhydryl, deuterium, oxo, thio, cyano, amino, carboxyl, C₁-C₆ alkyl and C₁-C₆ alkoxy;
Rᵢ and Rⱼ are each independently selected from the group consisting of hydrogen, hydroxy, C₁-C₆ alkyl and C₁-C₆ alkoxy;
Rₖ is independently selected from the group consisting of hydrogen, C₁-C₆ alkyl, C₁-C₆ haloalkyl, C₁-C₆ alkoxy, hydroxy and -NRᵢRⱼ;
each R₁ₐ is independently selected from the group consisting of hydrogen, C₁-C₆ alkyl and C₁-C₆ alkoxy;
each R_{1b} is independently selected from the group consisting of hydrogen, PG-, H-Li-, PG-Li-, 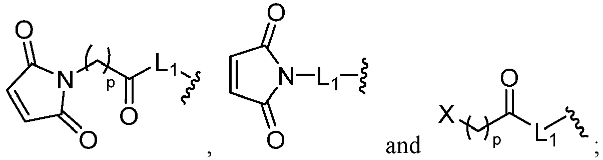
each p is independently 1, 2, 3, 4, 5 or 6;
Li is an amino acid unit, preferably -glycine-glutamic acid- or 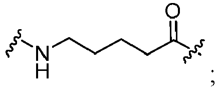
X is a halogen;
PG is an amino protecting group;
provided that when R₅ₐ is hydrogen or alkyl, R_{5b} is not hydrogen or alkyl.

20. The compound or the pharmaceutically acceptable salt thereof according to claim 18, being selected from the group consisting of 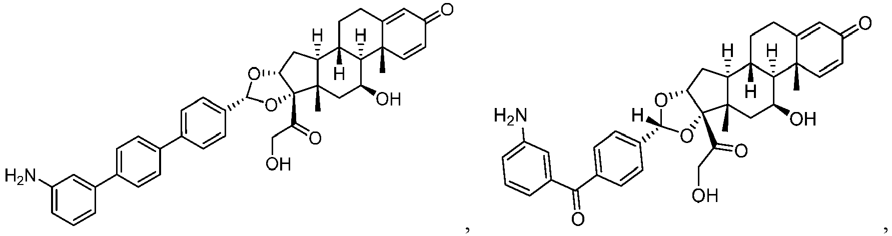 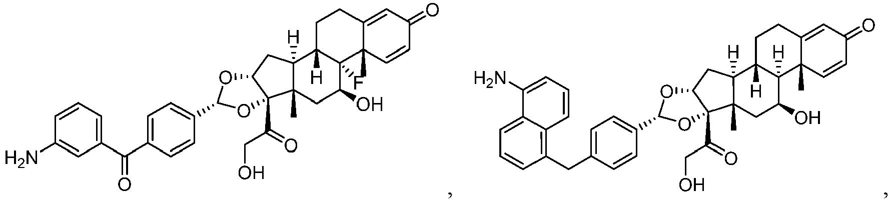 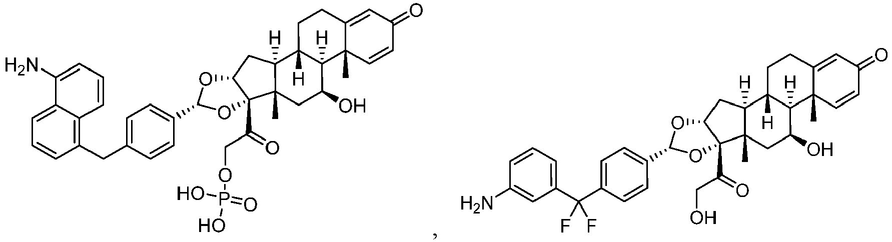 and 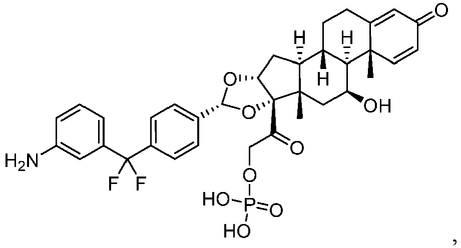 or pharmaceutically acceptable salts thereof.

21. The compound or the pharmaceutically acceptable salt thereof according to claim 18, being selected from the group consisting of 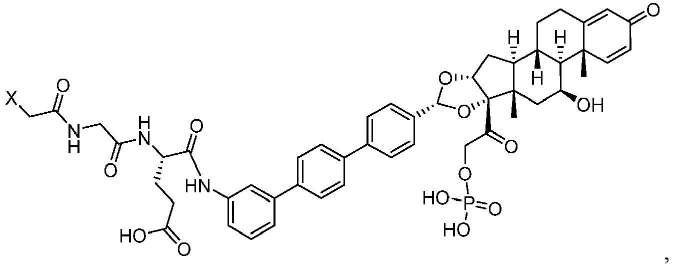 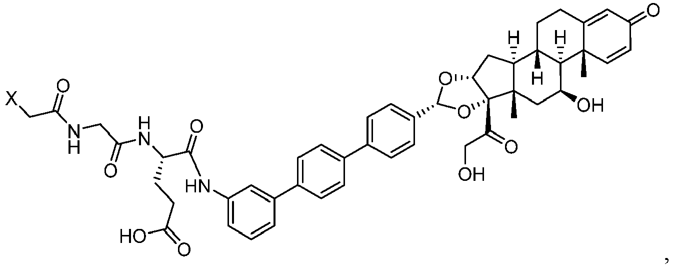 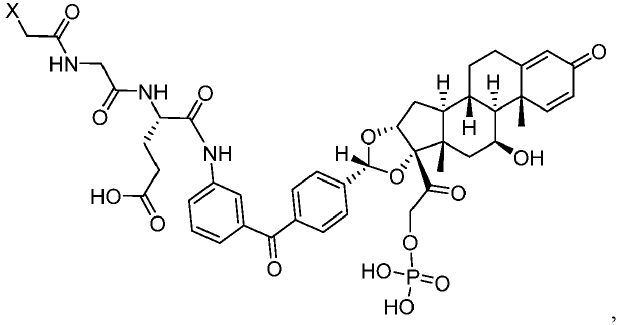 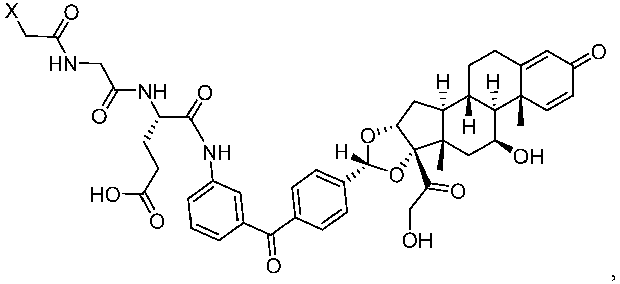 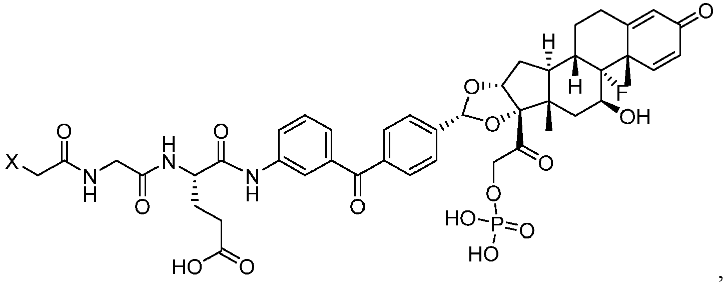 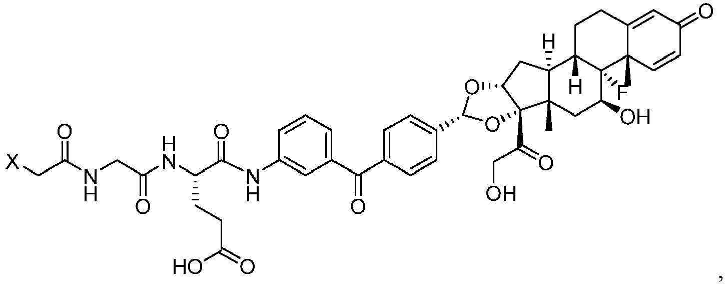 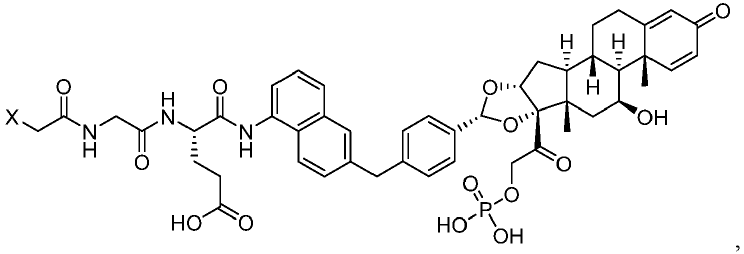 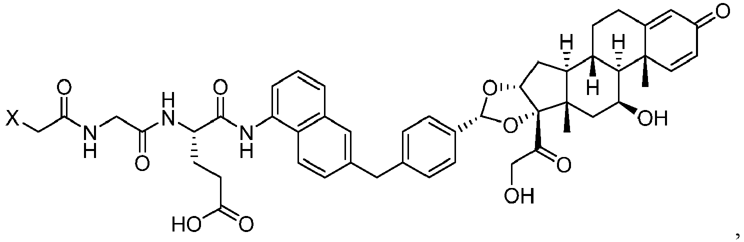 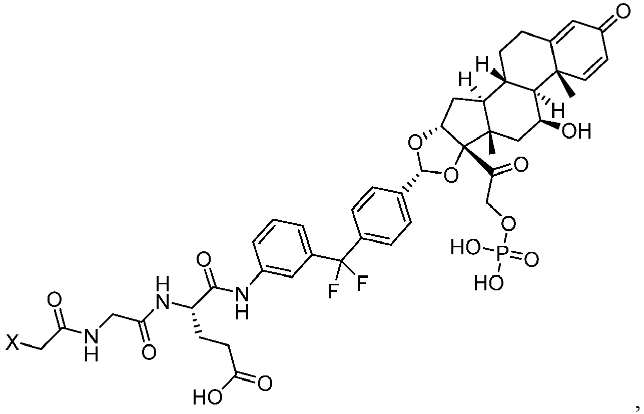 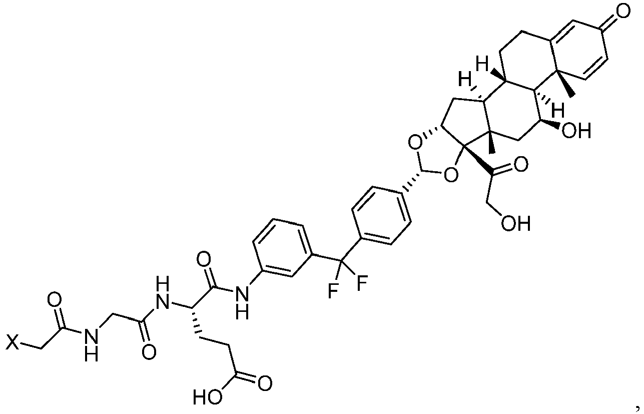 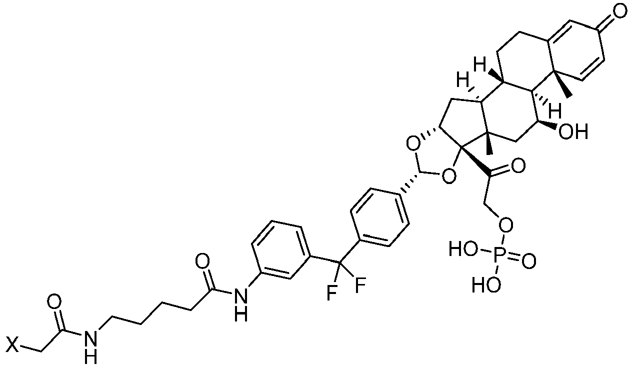 and 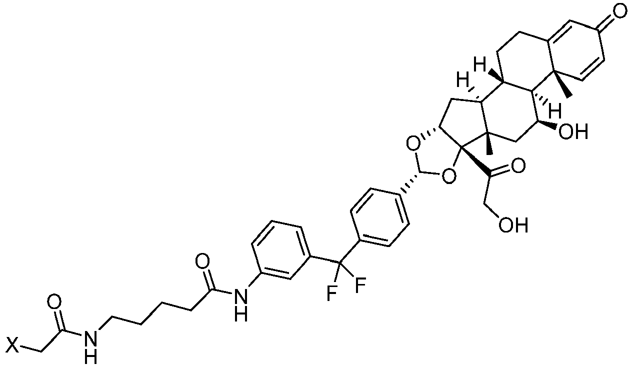 or pharmaceutically acceptable salts thereof, wherein X is a halogen, preferably chlorine or bromine, and more preferably bromine.

22. A pharmaceutical composition comprising the antibody-drug conjugate according to any one of claims 1-17 or the compound or the pharmaceutically acceptable salt thereof according to any one of claims 18-21, and a pharmaceutically acceptable excipient.

23. Use of the antibody-drug conjugate according to any one of claims 1-17 or the pharmaceutical composition according to claim 22 in the preparation of a medicament for treating an immune disease, wherein the immune disease is preferably selected from the group consisting of rheumatoid arthritis, juvenile idiopathic arthritis, psoriatic arthritis, ankylosing spondylitis, adult Crohn's disease, pediatric Crohn's disease, ulcerative colitis, hidradenitis suppurativa, uveitis, Behcet's disease, spondyloarthropathy and psoriasis.
